# EUROPEAN PATENT SPECIFICATION

(11) **EP 3 032 952 B1**
(45) Date of publication and mention of the grant of the patent: **27.11.2019**
(21) Application number: 14750736.2
(22) Date of filing: 13.08.2014
(51) Int. Cl.: A01N 33/24, A01N 37/44, A01N 41/08, C12N 15/82, A01N 25/00, A01C 1/06, A01H 5/10

(54) **METHODS FOR ENHANCING DROUGHT TOLERANCE IN PLANTS**
METHODEN ZUR VERBESSERUNG DER TROCKENHEITSTOLERANZ VON PFLANZEN
PROCÉDÉS D'AMÉLIORATION DE LA RÉSISTANCE DES PLANTES À LA SÉCHERESSE

(30) Priority: 13.08.2013 US 201361865549 P; 27.12.2013 US 201314142285; 10.03.2014 US 201414203261
(43) Date of publication of application: 22.06.2016
(73) Proprietor: PlantResponse Biotech, S.L., 28223 Pozuelo de Alarcon, Madrid (ES); Consejo Superior De Investigaciones Científicas, 28006 Madrid (ES)
(72) Inventor: BORJA Y TOME, Marisé, E-28224 Pozuelo de Alarcón (ES); BONET GIGANTE, Julio, E-28031 Madrid (ES); MOLINA FERNÁNDEZ, Antonio, E-28220 Majadahonda (ES); SALINAS MUÑOZ, Julio, E-28015 Madrid (ES); CATALA RODRÍGUEZ, Rafael, E-28925 Alcorcón (ES)
(74) Representative: ABG Intellectual Property Law, S.L.
(86) International application number: PCT/EP2014/067333
(87) International publication number: WO 2015/022365

(56) References cited:
- WO-A1-95/35022
- WO-A1-96/41532
- WO-A1-97/08951
- WO-A2-2008/023263
- WO-A2-2008/060555
- ES-A1- 2 347 399
- US-A1- 2013 130 902
- US-B1- 6 455 468
- AURÃ CR LIE CHARRIER ET AL: "The effect of carnitine ondevelopment and recovery in salt stress conditions", PLANTA ; AN INTERNATIONAL JOURNAL OF PLANT BIOLOGY, SPRINGER, BERLIN, DE, vol. 235, no. 1, 19 August 2011 (2011-08-19), pages 123-135, XP019995632, ISSN: 1432-2048, DOI: 10.1007/S00425-011-1499-4
- MINYOUNG LEE ET AL: "Activation of a flavin monooxygenase gene YUCCA7 enhances drought resistance in Arabidopsis", PLANTA, vol. 235, no. 5, 1 May 2012 (2012-05-01), pages 923-938, XP55158177, ISSN: 0032-0935, DOI: 10.1007/s00425-011-1552-3
- J. I. KIM ET AL: "Overexpression of Arabidopsis YUCCA6 in Potato Results in High-Auxin Developmental Phenotypes and Enhanced Resistance to Water Deficit", MOLECULAR PLANT, vol. 6, no. 2, 17 September 2012 (2012-09-17), pages 337-349, XP55158182, ISSN: 1674-2052, DOI: 10.1093/mp/sss100

## Description

### TECHNICAL FIELD

The invention relates to methods for improving traits in plants or photosynthetic organisms, including enhanced drought tolerance.

### BACKGROUND

When plants are exposed to conditions where reduced water content in the soil due to a shortage of rainfall or irrigation leads to impaired water absorption, what could be called drought stress conditions, physiological functions of cells may deteriorate and thus various disorders may arise in the plant. When subjected to such stress factor plants display a variety of mechanistic responses as protective measures, with a resultant adverse effect on growth, development, and productivity. Significant losses in quality and yield are commonly observed.

While it has been known that phytohormones and some chemical substances such as plant growth regulators have effects on plants in reducing drought stress such as drought stress or excessive moisture stress (see Journal of Plant Growth Regulation (2010) 29: 366-374), those effects are not necessarily satisfactory in practice. For example, organic osmolytes are small solutes used by cells of numerous water-stressed organisms and tissues to maintain cell volume. Similar compounds are accumulated by some organisms in anhydrobiotic, thermal and possibly pressure stresses. These solutes are amino acids and derivatives, polyols and sugars, methylamines, methylsulfonium compounds and urea. Except for urea, they are often called "compatible solutes", a term indicating lack of perturbing effects on cellular macromolecules and implying interchangeability. However, these features may not always exist, and the practical use cannot be taken for granted since high levels might cause overstabilization of proteins and some protective properties of osmolytes are harmful in the absence of a perturbant to offset (Yancey, P.H. (2005). J. Exp. Biol. 208 (Pt 15): 2819-30). For example the osmolite glycinebetaine (betaine) affords osmoprotection in bacteria, plants and animals, and protects cell components against harsh conditions *in vitro,* however, engineering of betaine production in three diverse species lacking it, *Arabidopsis, Brassica napus,* and tobacco (*Nicotiana tabacum*), by constitutive expression of a bacterial choline oxidase gene only conferred a moderate stress tolerance in some but not all betaine-producing transgenic lines and the responses to stresses such as salinity, drought, and freezing were variable among the three species. Furthermore, a fitness cost was observed in the three species (Jun H, Hariji et al. (2000) Plant Physiol. 122: 747-56). Betaine has also been used for producing a drought tolerant plant in WO 96/41532 A1, WO 95/35022 A1 and WO 97/08951 A1 and choline chloride has been used for the same purpose in US 6 455 468 B1. ES 2 347 399 A1 shows that TMAO is useful for increasing resistance to cold and US 2013/130902 A1 discloses that in order to increase the bioavailability of silicate, an alkali metal silicate may be formulated in the presence of a first and second osmolyte compound (N-methylated compound) such as TMAO, and a third osmolyte compound.

Therefore, alternative strategies for producing drought stress tolerant plants are needed.

### SUMMARY OF THE INVENTION

The following embodiments and aspects thereof are described and illustrated in conjunction with systems, tools and methods, which are meant to be exemplary and illustrative, not limiting in scope.

In a first aspect, the invention relates to a method for producing a drought tolerant plant or photosynthetic organism comprising:
applying at least one treatment of an effective amount of Trimethylamine N-oxide (TMAO); Trimethylamine N-oxide di-hydrate or N,N-Dimethyldecylamine N-oxide (DDAO) to a plant, plant part, photosynthetic organism or seed; wherein the effective amount of the TMAO, TMAO di-hydrate or DDAO is from 0.1 to 10 g per liter for spray or irrigation, or wherein the at least one treatment is a seed treatment and the effective amount of the TMAO, TMAO di-hydrate or DDAO is from 0.1 g to 1000 g per 100 kg of seed; and growing said plant, plant part, photosynthetic organism or seed, wherein a drought tolerant plant or photosynthetic organism is produced.

Various embodiments are set forth in the Detailed Description as provided herein and as embodied by the claims. It should be understood, however, that this Summary does not contain all of the aspects and embodiments of the present invention, is not meant to be limiting or restrictive in any manner, and that the invention(s) as disclosed herein is/are understood by those of ordinary skill in the art to encompass obvious improvements and modifications thereto.

Additional advantages of the present inventions will become readily apparent from the following discussion, particularly when taken together with the accompanying drawings and sequence listings.

### BRIEF DESCRIPTION OF THE SEQUENCE LISTINGS

SEQ ID NO: 1 discloses the *At FMO GS-OX5* nucleic acid sequence (NM_101086.4|) (At1g12140).
SEQ ID NO: 2: discloses the *At FMO GS-OX5* amino acid sequence (NM_101086.4|) (At1g12140).
SEQ ID NO: 3 discloses the *Br FMO GS-OX1* nucleic acid sequence (FJ376070.1).
SEQ ID NO: 4 discloses the *Br FMO GS-OX1* amino acid sequence (FJ376070.1).
SEQ ID NO: 5 discloses the *Cs FMO GS-OX3* nucleic acid sequence (XM_004150596.1) (LOC101212991).
SEQ ID NO: 6 discloses the *Cs FMO GS-OX3* amino acid sequence (XM_004150596.1) (LOC101212991).
SEQ ID NO: 7 discloses the *Cs FMO GS-OX3* nucleic acid sequence (XM_004150602.1) (LOC101220318).
SEQ ID NO: 8 discloses the *Cs FMO GS-OX3* amino acid sequence (XM_004150602.1) (LOC101220318).
SEQ ID NO: 9 discloses the *Cs FMO GS-OX3* nucleic acid sequence (XM_004170413.1) (LOC101220079).
SEQ ID NO: 10 discloses the *Cs FMO GS-OX3* amino acid sequence (XM_004170413.1) (LOC101220079).
SEQ ID NO: 11 discloses the *Cs FMO GS-OX3* nucleic acid sequence (XM_004164404.1) (LOC101227975).
SEQ ID NO: 12 discloses the *Cs FMO GS-OX3* amino acid sequence (XM_004164404.1) (LOC101227975).
SEQ ID NO: 13 discloses the *Mt FMO GS-OX5* nucleic acid sequence (XM_003611223.1) (MTR_5g012130).
SEQ ID NO: 14 discloses the *Mt FMO GS-OX5* amino acid sequence (XM_003611223.1) (MTR_5g012130).
SEQ ID NO: 15 discloses the *Os FMO* nucleic acid sequence (NC_008403.2).
SEQ ID NO: 16 discloses the *Os FMO* amino acid sequence (NP_001065338.1).
SEQ ID NO: 17 discloses the *Vv FMO GS-OX3-3* nucleic acid sequence (XM_003631392.1) (LOC100255688).
SEQ ID NO: 18 discloses the *Vv FMO GS-OX3-3* amino acid sequence (XM_003631392.1) (LOC100255688).
SEQ ID NO: 19 discloses the *Vv FMO GS-OX3-2* nucleic acid sequence (XM_003631391.1) (LOC100255688).
SEQ ID NO: 20 discloses the *Vv FMO GS-OX3-2* amino acid sequence (XM_003631391.1) (LOC100255688).
SEQ ID NO: 21 discloses the *Vv FMO GS-OX3-2* nucleic acid sequence (XM_003635084.1) (LOC100242032).
SEQ ID NO: 22 discloses the *Vv FMO GS-OX3-2* amino acid sequence (XM_003635084.1) (LOC100242032).
SEQ ID NO: 23 discloses the *Gh FMO-1* nucleic acid sequence (DQ122185.1).
SEQ ID NO: 24 discloses the *Gh FMO-1* amino acid sequence (DQ122185.1).
SEQ ID NO: 25 discloses the *Zm FMO* nucleic acid sequence (NM_001157345.1).
SEQ ID NO: 26 discloses the *Zm FMO* amino acid sequence (NP_001150817.1).
SEQ ID NO: 27 discloses the *Pt FMO GS-OX* nucleic acid sequence (XM_002329873.1).
SEQ ID NO: 28 discloses the *Pt FMO GS-OX* amino acid sequence (XM_002329873.1).
SEQ ID NO: 29 discloses the *Pt FMO GS-OX* nucleic acid sequence (XM_002318967.1).
SEQ ID NO: 30 discloses the *Pt FMO GS-OX* amino acid sequence (XM_002318967.1).
SEQ ID NO: 31 discloses the *Pt FMO GS-OX* nucleic acid sequence (XM_002329874.1).
SEQ ID NO: 32 discloses the *Pt FMO GS-OX* amino acid sequence (XM_002329874.1).
SEQ ID NO: 33 discloses the *Gm FMO* nucleic acid sequence (NM_003538657.1).
SEQ ID NO: 34 discloses the *Gm FMO* amino acid sequence (XP_003538705.1).
SEQ ID NO: 35 discloses the *Sl FMO GS-OX* nucleic acid sequence (XM_004241959.1) (LEFL1075CA11).
SEQ ID NO: 36 discloses the *Sl FMO GS-OX* amino acid sequence (XP_004242007.1) (LEFL1075CA11).
SEQ ID NO: 37 discloses the *Sl FMO GS-OX* nucleic acid sequence (SGN-U584070) (Solyc06g060610).
SEQ ID NO: 38 discloses the *Sl FMO GS-OX* amino acid sequence (SGN-U584070) (Solyc06g060610).
SEQ ID NO: 39 discloses the *Hs FMO-3* nucleic acid sequence (NC_000001.10 (171,060,018..171,086,961)).
SEQ ID NO: 40 discloses the *Hs FMO-3* amino acid sequence (NP_001002294.1).
SEQ ID NO: 41 discloses the *Oc FMO-3* nucleic acid sequence (NC _013681.1).
SEQ ID NO: 42 discloses the *Oc FMO-3* amino acid sequence (NP_001075714.1).
SEQ ID NO: 43 discloses the consensus sequence of the polypeptide SEQ ID No. from 2 to 38.
SEQ ID NO: 44 discloses the 5'UTR in combination with the DNA sequence of *AtFMO GS.*

### BRIEF DESCRIPTION OF THE FIGURES

The accompanying figures, illustrate some, but not the only or exclusive, example embodiments and/or features. It is intended that the embodiments and figures disclosed herein are to be considered illustrative rather than limiting.

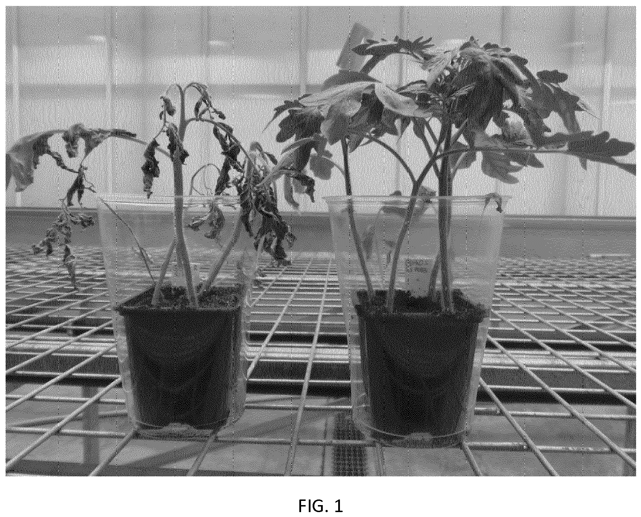

Figure 1 shows from the left, tomato plants irrigated with water and on the right, plants irrigated with 5.5g/L TMAO di-hydrate after drought recovery.

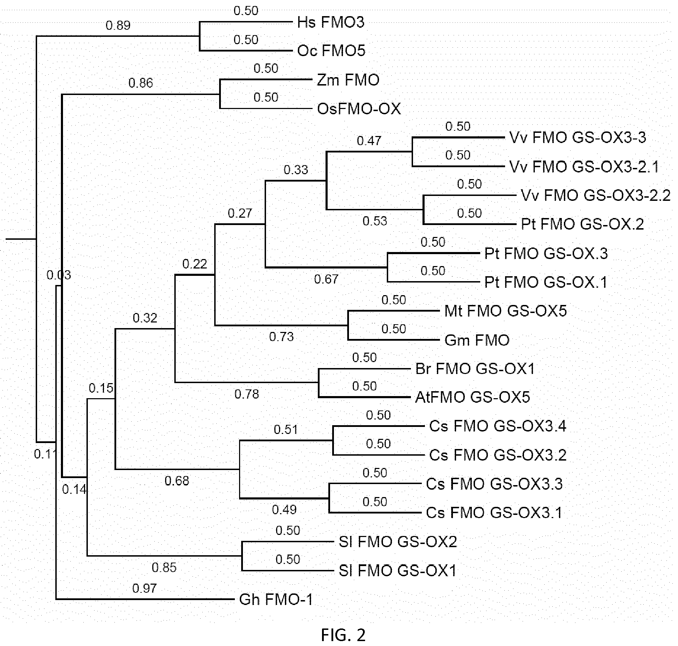

Figure 2 shows a phylogenetic tree based on protein similarities using the alignment-free algorithm, named CLUSS, for clustering protein families of the polypeptide sequences of FMO from *Arabidopsis thaliana,* grapevine, *Populus trichocarpa,* rice, soybean, melon, tomato, sorghum, corn, wheat, barley, human and rabbit.

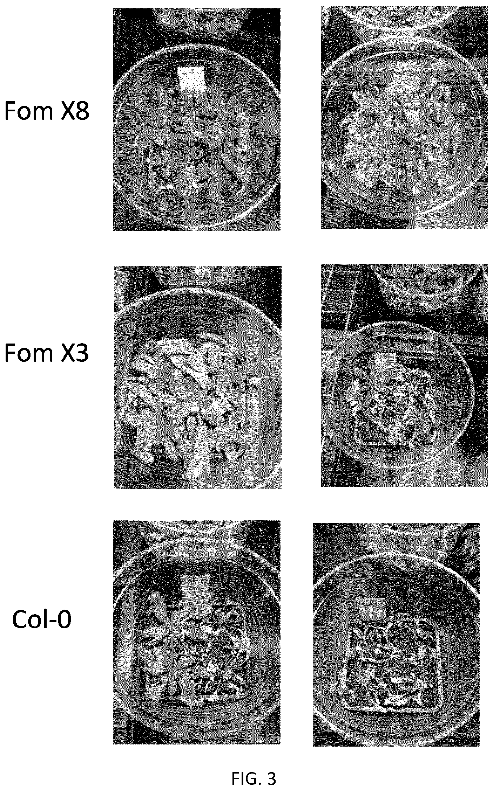

Figure 3 shows, from the bottom, wild type Col-0 (labeled Col-0) *Arabidopsis thaliana* plants, in the middle (labels FOM X3), transgenic *Arabidopsis thaliana* plants overexpressing three copies of the At FMO GS-OX5 sequence and in the upper panel (labeled FOM X8) transgenic *Arabidopsis thaliana* plants over expressing eight copies of the At FMO GS-OX5 sequence after drought recovery.

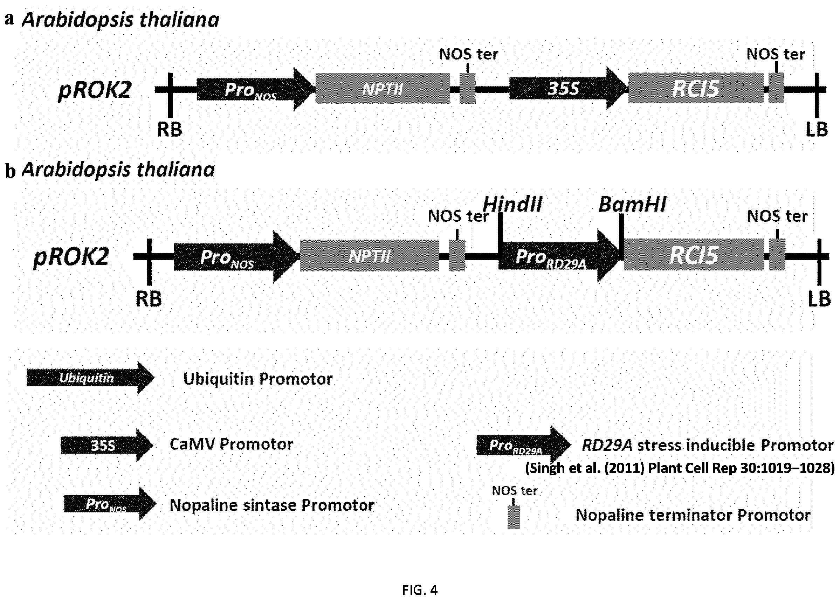

Figure 4a is a map of a DNA construct that may be used to obtain the *Arabidopsis thaliana* plants for constitutive overexpression of the At FMO GS-OX5 sequence, which includes (from 5' to 3'), a promoter (PRONOS), a selectable marker (NPTII), a constitutive promoter (35S) and a FMO protein coding sequence (RCI5) stably integrated into a pROK2 vector.
Figure 4b is a second map of a DNA construct that may be used to obtain the *Arabidopsis thaliana* plants for constitutive overexpression the At FMO GS-OX5 sequence, that includes (from 5' to 3'), a promoter (PRONOS), a selectable marker (NPTII), a stress inducible promoter (PRORD29A) and a FMO protein coding sequence (RCI5) stably integrated into a pROK2 vector.

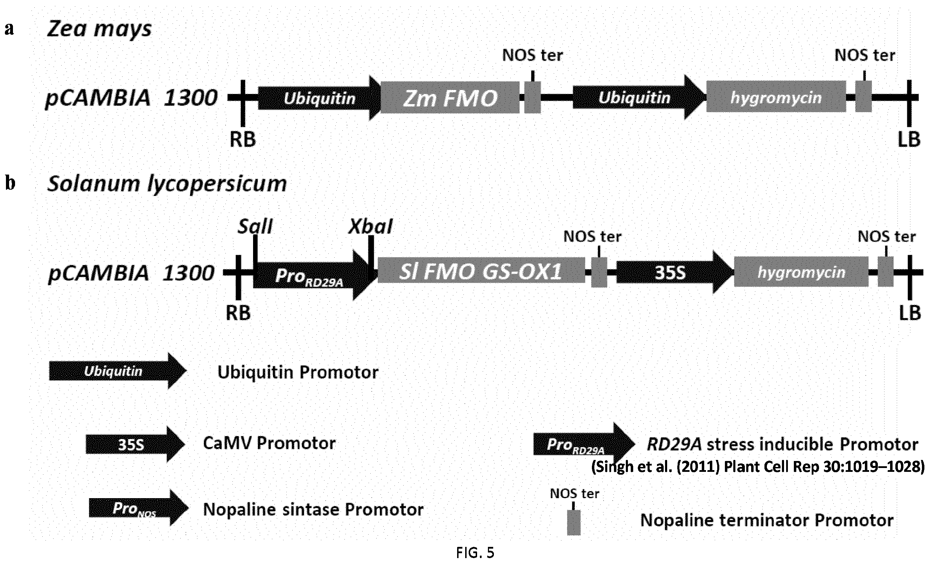

Figure 5a is a map of a DNA construct that may be used to obtain the *Zea mays* plants for constitutive overexpression of the Zm FMO protein coding sequence that includes (from 5' to 3'), constitutive promoter (Ubiquitin), an FMO protein coding sequence (Zm FMO), a second promoter (35S) and a selectable marker (hygromycin) stably integrated into a pCAMBIA 1300 vector.
Figure 5b is a map of a DNA construct that may be used to obtain the *Solanum lycopersicum* plants for overexpression of the Sl FMO GS-OX1 protein coding sequence, that includes (from 5' to 3'), a stress inducible promoter (PRORD29A), an FMO protein coding sequence (SI FMO GS-OX1), a second promoter (35S) and a selectable marker (hygromycin) stably integrated into a pCAMBIA 1300 vector.

### DETAILED DESCRIPTION OF THE INVENTION

The present disclosure includes methods for producing plants or photosynthetic organisms tolerant to drought stress, including tolerances to but not limited to drought, excessive moisture as well as efficient water usage where normal yields are produced with less water input. These methods include the application of organic compounds, such as trimethylamine *N*-oxide ("TMAO") or a TMAO analog, a TMAO derivative, or TMAO di-hydrate to plants or seeds to produce a plant tolerant to drought stress. The present disclosure also includes plants or photosynthetic organisms tolerant to drought stress, including tolerances to but not limited to drought and excessive moisture. These drought stress tolerant plants and photosynthetic organisms may be produced through the application of organic compounds, such as trimethylamine *N*-oxide ("TMAO") or a TMAO analog, a TMAO derivative, or TMAO di-hydrate to induce drought stress tolerance, allowing for the production of plants and photosynthetic organisms with more a biomass, fruit or seed when compared to plants and photosynthetic organisms that have not been treated with organic compounds to produce drought stress tolerance.

Thus, in a first aspect, the invention relates to a method for producing a drought tolerant plant or photosynthetic organism (first method) comprising:
applying at least one treatment of an effective amount of Trimethylamine N-(TMAO), Trimethylamine N-oxide di-hydrate, or N,N-Dimethyldecylamine N-oxide (DDAO)to a plant, plant part, photosynthetic organism or seed, wherein the effective amount of the TMAO, TMAO di-hydrate or DDAO is from 0.1 to 10 g per liter for spray or irrigation, or wherein the at least one treatment is a seed treatment and the effective amount of the TMAO, TMAO di-hydrate or DDAO is from 0.1 g to 1000 g per 100 kg of seed; and
growing said plant, plant part, photosynthetic organism or seed, wherein a drought stress tolerant plant or photosynthetic organism is produced.

As used herein the term "drought stress" is used interchangeably with water stress. The term "drought stress" as used herein can be induced in plants under conditions where reduced water content in the soil, due to a shortage of rainfall or irrigation, leads to impaired or reduced water absorption by the plant or photosynthetic organism. Water stress may trigger in plants a deterioration of physiological functions of cells, thereby leading to various disorders. While the conditions which induce drought stress may vary depending on the kind of the soil where plants are cultivated, examples of the conditions include but are not limited to: a reduction in the water content in the soil of 15% by weight or less, more severely 10% by weight or less, and still more severely 7.5% by weight or less; or the pF value of the soil of 2.3 or more, more severely 2.7 or more, and still more severely 3.0 or more.

As introduced above, it is disclosed one or more methods for producing plants or photosynthetics organisms tolerant to water stress, including but not limited to drought tolerance or excessive moisture, in plants wherein an application of trimethylamine N-oxide or "TMAO", wherein TMAO includes but is not limited to, TMAO di-hydrate , TMAO chemical derivative, or a TMAO chemical analogue, to a plant or seed to reduce water stress in the plant when the plant is exposed to water stress conditions. Further methylamines (e.g. trimethylamine N-oxide (TMAO)) can enhance protein folding and ligand binding and counteract perturbations by urea (e.g. in elasmobranchs and mammalian kidney), inorganic ions, and hydrostatic pressure in deep-sea animals (Yancey, 2005, cited *supra*).

According to the invention, the TMAO chemical derivative is N,N-Dimethyldecylamine N-oxide (DDAO). It is disclosed that the TMAO chemical analogue is a N-methylated compound. In a disclosure, the N-methylated compound is selected from the group consisting of carnitine, sarcosine, N-methyl aspartic acid, N-methyl taurine.

The one or more methods of producing a plant or photosynthetic organism tolerant to water stress is applicable to a variety of plants including monocotyledonous or dicotyledonous plants, including but not limited to transgenic plants. As used herein, transgenic plants include plants, or photosynthetic organism, which have been genetically modified to contain DNA constructs as will be discussed further herein. The methods for producing a plant or organism tolerant to water stress may be applicable to the whole plant or organism or a part of a plant, for example in an organ, tissue, a cell or a part of a plant cell, for example in an organelle, which comprises introducing into, and expressing in, the plant or plant cell a nucleic acid which codes for a monooxygenase or FMO protein, and which mediates an increased production of endogenous TMAO and therefore a water stress tolerance, such as an increased tolerance to drought or an increased tolerance to excessive moisture.

One or more embodiments described herein may further provide methods for producing a water stress tolerant plant or photosynthetic organism which comprises applying an effective enough amount of TMAO, TMAO di-hydrate or DDAO to a plant or organism that has been exposed to or to be exposed to water stress conditions.

Thus, in a preferred embodiment, the first method for producing a drought tolerant plant or photosynthetic organism further comprises applying at least one second treatment of an effective amount of TMAO, TMAO di-hydrate or DDAO to said drought tolerant plant or photosynthetic organism, previously treated with TMAO, TMAO di-hydrate or DDAO.

This method may further include a seed treatment application, a spray treatment or an irrigation treatment of the TMAO, TMAO di-hydrate or DDAO. In a preferred embodiment, the at least one treatment of an effective amount of TMAO, TMAO di-hydrate or DDAO is a seed treatment. It is disclosed that an effective amount of TMAO seed treatment may include a seed treatment of TMAO in an amount from 0.1 to 1000 g per 100 kg seeds, from 0.1 to 1000 g per 100 kg seeds, or 0.1 to 100 g per 100 kg seeds, 0.1 to 10 g per 100 kg seeds or 0.1 g to 5 g per 100 kg seeds. In the method of the invention wherein the at least one treatment is a seed treatment, the effective amount of the TMAO, TMAO di-hydrate or DDAO is from 0.1 g to 1000 g per 100 kg of seed.

In a more preferred disclosure, said effective amount of TMAO in a seed treatment is between 0.1g to 1000 g per kg of seed. In another preferred disclosure, said effective amount of TMAO in a seed treatment is between 1 g to 100 g per kg of seed. In another preferred disclosure, said effective amount of TMAO in a seed treatment is between 1 g to 100 g per kg of seed, between 0.1 to 10 g per kg seeds or 0.1 g to 5 g per kg seeds

In another preferred disclosure, the at least one treatment of said effective amount of TMAO is an irrigation treatment or a spray treatment. In a preferred disclosure, effective amount of said TMAO is between 0.01 to 10000 g per litre for said irrigation treatment or spray treatment. According to the invention, the effective amount of said TMAO, TMAO di-hydrate or DDAO is from 0.1 to 10 g per litre for said irrigation treatment or spray treatment. In another embodiment, the effective amount is 0.1 to to 5 g per litre for said irrigation treatment.

In another disclosure, the at least one treatment of said effective amount of TMAO in the methods disclosed herein comprises two or more different compounds selected from the group consisting of TMAO, TMAO di-hydrate, a TMAO chemical derivative, such as N,N-Dimethyldecylamine N-oxide (DDAO), or a TMAO chemical analogue, such as an N-methylated compound such as carnitine, sarcosine, N-methyl aspartic acid, N-methyl taurine thereof. According to the invention, the at least one treatment of said effective amount of TMAO, TMAO di-hydrate or DDAO comprises two or more different compounds selected from the group consisting of TMAO, TMAO di-hydrate and DDAO.

The use of TMAO, TMAO di-hydrate, a TMAO derivative or chemical analogue and agriculturally acceptable salts, wherein agriculturally acceptable salts may include but is not limited to, a mixture of ammonium phosphate, ammonium nitrate, potassium nitrate, and calcium nitrate in a 2-2-1-1 proportion for reducing water stress in a plant.

In another embodiment, the said water stress tolerant plant or photosynthetic organism has a biomass, seed or fruit production that is 6%, 10%, 15%, 20%, 25%, 30%, 35%, 40%, 45%, 50%, 55%, 60%, 65%, 70%, 75%, 80%, 85%, 90%, 95%, 100%, 110%, 120%, 130%, 140%, 150%, including or more than the biomass, seed or fruit production of water stressed plants or photosynthetic organisms where an effective amount of TMAO has not been applied to the non-tolerant water stressed plant or photosynthetic organism. In preferred embodiments, the biomass, seed or fruit production is between 6% and 30% or more, between 31% and 50% or more, between 51% and 70% or more, between 71% and 100% or more than the a biomass, fruit or seed production of water stressed plants or photosynthetic organisms where an effective amount of TMAO has not been applied to the non-tolerant plant or photosynthetic organism. The description of a range should be considered to have specifically disclosed all the possible sub-ranges as well as individual numerical values within that range.

The biomass, fruit or seed production can be measured by any method known in the art.

Water stress in plants produced using the methods of the invention may be recognized or identified by comparing a change in plant phenotypes described in more detail below between plants which have been exposed to water stress conditions and plants which have not been exposed to the same water stress conditions. Water stress in a plant or photosynthetic organism may be indicated by a change in one or more of the following plant phenotypes, which can serve as indicators of the water stress in plants: (1) germination percentage, (2) seedling establishment rate, (3) number of healthy leaves, (4) plant length, (5) plant weight, (6) leaf area, (7) leaf colour, (8) number or weight of seeds or fruits, (9) quality of harvests, (10) flower setting rate or fruit setting rate, (11) chlorophyll fluorescence yield, (12) water content, (13) leaf surface temperature, and (14) transpiration capacity.

Water stress may be quantified as the "intensity of stress" where intensity of stress is represented as following: "Intensity of stress" = 100 x "any one of plant phenotypes in plants which have not been exposed to water stress" / "the plant phenotype in plants which have been exposed to water".

The methods described herein are applied to plants that have been exposed to or to be exposed to water stress conditions whose intensity of stress represented by the above equation is from 105 to 450, preferably from 110 to 200, and more preferably from 115 to 160. The description of a range should be considered to have specifically disclosed all the possible subranges as well as individual numerical values within that range. In a plant exposed to water stress conditions, an influence may be recognized on at least one of the above phenotypes. That is, observed as: (1) decrease in germination percentage, (2) decrease in seedling establishment rate, (3) decrease in number of healthy leaves, (4) decrease in plant length, (5) decrease in plant weight, (6) decrease in leaf area increasing rate, (7) leaf color fading, (8) decrease in number or weight of seeds or fruits, (9) deterioration in quality of harvests, (10) decrease in flower setting rate or fruit setting rate, (11) decrease in chlorophyll fluorescence yield, (12) decrease in water content, (13) increase in leaf surface temperature, or (14) decrease in transpiration capacity, among others, and the magnitude of the water stress in the plant can be measured using that as an indicator.

The methods described herein are directed to methods for reducing water stress in a plant or organism by producing a plant or organism tolerant to water stress by applying the TMAO to the plant that has been exposed to or will be exposed to water stress conditions. The effect of reducing the water stress of a plant can be evaluated by comparing the above phenotypic indicators between a plant treated with TMAO and a plant which has not been treated with TMAO after the plants or organisms are exposed to water stress conditions. Stages in which plants or organisms treated with TMAO can be exposed to the water stress conditions include all growth stages of plants, including a germination period, a vegetative growing period, a reproductive growing period and a harvesting period. The application period of the TMAO as used herein may be any growth stage of plants or organisms, and examples thereof include the germination period such as before seeding, at the time of seeding, and after seeding and before or after emergence; the vegetative growing period such as at the time of seedling raising, at the time of seedling transplantation, at the time of cutting or sticking, or at the time of growing after settled planting; the reproductive growing period such as before blooming, during blooming, after blooming, immediately before earing or during the ear formation period; and the harvesting period such as before harvesting plan, before ripening plan, or a coloration initiation period of fruits. Plants to which TMAO is to be applied may be plants which have been exposed to or will be exposed to the water stress conditions. That is, the compound can also be preventively applied to plants before being exposed to the water stress conditions in addition to plants which have been exposed to the water stress conditions.

In another preferred embodiment, the method of the invention further comprises applying salts or any other additive to said plant, plant part, photosynthetic organism or seed and growing said plant, plant part, photosynthetic organism or seed wherein a drought tolerant plant or photosynthetic organism is produced.

The TMAO used in the methods described herein can be used alone, or in combination with various inert ingredients such as solid carriers, liquid carriers, and surfactants as described hereinafter.

Examples of a solid carrier used in formulation with TMAO may include powders, fine powders or granules such as minerals such as kaolin clay, attapulgite clay, bentonite, montmorillonite, acid white clay, pyrophyllite, tale, diatomaceous earth and calcite; natural organic materials such as corn rachis powder and walnut husk powder; synthetic organic materials such as urea; salts such as calcium carbonate and ammonium sulfate; and synthetic inorganic materials such as synthetic hydrated silicon oxide.

Examples of a liquid carrier may include aromatic hydrocarbons such as xylene, alkylbenzene and methylnaphthalene; alcohols such as 2-5 propanol, ethyleneglycol, propylene glycol, and ethylene glycol monoethyl ether; ketones such as acetone, cyclohexanone and isophorone; vegetable oil such as soybean oil and cotton seed oil; and petroleum aliphatic hydrocarbons, esters, dimethylsulfoxide, acetonitrile and water.

Examples of a surfactant include anionic surfactants such as alkyl sulfonate ester salts, alkylaryl sulfonate salts, dialkyl sulfosuccinate salts, polyoxyethylene alkyl aryl ether phosphate ester salts, lignosulfonate salts and naphthalene sulfonate formaldehyde polycondensates; and nonionic surfactants such as polyoxyethylene alkyl aryl ethers, polyoxyethylene alkylpolyoxypropylene block copolymers and sorbitan fatty acid esters; and cationic surfactants such as alkyltrimethylammonium salts.

Examples of the other formulation auxiliary agents include water-soluble polymers such as polyvinyl alcohol and polyvinylpyrrolidone, polysaccharides such as Arabic gum, alginic acid and the salt thereof, CMC (carboxymethyl-cellulose), Xanthan gum, inorganic materials such as aluminium magnesium silicate and alumina sol, preservatives, colouring agents and stabilization agents such as PAP (acid phosphate isopropyl) and butylated hydroxytoluene (BHT).

The methods for production of a plant or photosynthetic organism tolerant to water stress as described herein may be carried out by applying an effective amount of the TMAO to plants or growing sites of plants. As used herein an effective amount of TMAO may include a range from 0.1 to 1.000 g per liter per 1-10 kg seeds. When incorporated into the entire soil, an effective amount of TMAO may range from 0.1 to 1.000 g or 1 to 500 g, per 1.000 m² of soil. When treatment with TMAO is used as an emulsion, a wettable powder, a flowable agent, or a microcapsule may be used for the treatment by spraying the plant after dilution with water. In this case, the concentration of the TMAO may range from 0.01 to 10,000 ppm, or from 1 to 5,000 ppm. A dust formulation and a granule of TMAO may be used for the treatment of water stress without dilution of the TMAO. In the treatment of seeds or the treatment of bulbs, an example of the weight of the TMAO per 100 kg of seeds may range from 0.1 to 1000 g, as well as from 1 to 30 g. Examples of the seeds or bulbs used in the methods described herein include those having a weight of 100 g or less, including 20 g or less, 0.5 g or less, as well as 50 mg or less. In the treatment of seedlings, an example of the weight of the TMAO per seedling may range from 0.01 to 20 mg, including 0.5 to 8 mg. In the treatment of the soil before or after sowing seedlings, the weight of the TMAO per 1.000 m² may range from 0.1 to 1000 g, including from 10 to 100 g.

TMAO may be applied to a variety of plants in various forms or sites, such as foliage, buds, flowers, fruits, ears or spikes, seeds, bulbs, stem tubers, roots and seedlings. As used herein, bulbs mean discoid stem, rhizomes, root tubers, and rhizophores. In the present specification, TMAO may also be applied to cuttings and sugar cane stem cuttings.

The following are examples of the growing sites of plants include soil before or after sowing plants. When the TMAO is applied to plants or growing sites of plants, the TMAO is applied to the target plants once or more. TMAO may be applied as a treatment to foliage, floral organs or ears or spikes of plants, such as foliage spraying; treatment of seeds, such as seed sterilization, seed immersion or seed coating; treatment of seedlings; treatment of bulbs; and treatment of cultivation lands of plants, such as soil treatment. The TMAO may be applied only to specific sites of plants, such as floral organ in the blooming season including before blooming, during blooming and after blooming, and the ear or spike in the earing season, or may be applied to entire plants.

TMAO may be applied as a soil treatment in the form a spray onto soil, soil incorporation, and perfusion of a chemical liquid into the soil (irrigation of chemical liquid, soil injection, and dripping of chemical liquid). The placement of TMAO during soil treatment includes but is not limited to planting hole, furrow, around a planting hole, around a furrow, entire surface of cultivation lands, the parts between the soil and the plant, area between roots, area beneath the trunk, main furrow, growing box, seedling raising tray and seedbed, seedling raising. TMAO soil treatment may be before seeding, at the time of seeding, immediately after seeding, raising period, before settled planting, at the time of settled planting, and growing period after settled planting.

When applying TMAO as a soil treatment, two or more kinds of TMAOs may be simultaneously applied to the plant, for example, TMAO and TMAO di-hydrate, or TMAO di-hydrate and an aryl amine oxide, or a solid fertilizer such as a paste fertilizer containing the TMAO may be applied to the soil. TMAO may be mixed in an irrigation liquid, and, examples thereof include injecting to irrigation facilities (irrigation tube, irrigation pipe, sprinkler, etc.), mixing into the flooding liquid between furrows, mixing into a hydroponic medium and the like.

Alternatively, an irrigation liquid may be mixed with the TMAO in advance and, for example, used for treatment by an appropriate irrigating method including the irrigation method mentioned above and the other methods such as sprinkling and flooding. TMAO may also be applied by winding a crop with a resin formulation processed into a sheet or a string, putting a string of the resin formulation around a crop so that the crop is surrounded by the string, and/or laying a sheet of the resin formulation on the soil surface near the root of a crop.

In another embodiment, TMAO may be used for treating seeds or bulbs as well as a TMAO spraying treatment for seeds in which a suspension of TMAO is atomized and sprayed on a seed surface or bulb surface. A smearing treatment may also be used in where a wettable powder, an emulsion or a flowable agent of the TMAO is applied to seeds or bulbs with a small amount of water added or applied as is without dilution. In addition, an immersing treatment may be used in which seeds are immersed in a solution of the TMAO for a certain period of time, film coating treatment, and pellet coating treatment.

TMAO may be used for the treatment of seedlings, including spraying treatment comprised of spraying the entire seedlings with a dilution having a proper concentration of active ingredients prepared by diluting the TMAO with water. As with seed treatment, an immersing treatment may also be used comprised of immersing seedlings in the dilution, and coating treatment of adhering the TMAO formulated into a dust formulation to the entire seedlings.

TMAO may be treated to soil before or after sowing seedlings including spraying a dilution having a proper concentration of active ingredients prepared by diluting TMAO with water and applying the mixture to seedlings or the soil around seedlings after sowing seedlings. A spray treatment of TMAO formulated into a solid formulation such as a granule to soil around seedlings at sowing seedlings may also be used.

TMAO may be used for treatment of hydroponics. Examples may include dissolving or suspending TMAO in a conventionally used culture medium for hydroponics, at a concentration within a range from 0.0001 to 10g/litre.

TMAO may be used at the time of tissue culture or cell culture of a plant to promote tolerance to water stress. TMAO may be dissolved or suspended in a conventionally used culture medium for plant tissue culture or other organisms, such as a Murashige and Skoog ("MS") culture medium. Examples may include a concentration within a range from 0.0001 to 10g/liter. In this case, in accordance with a usual method, saccharides as a carbon source, various phytohormones and the like can be appropriately added.

It is disclosed that the method for producing a drought tolerant plant comprises a first step wherein the plant is sprayed with a solution containing trimethylamine N-oxide (TMAO), Trimethylamine N-oxide di-hydrate, a TMAO chemical derivative or a TMAO chemical analogue followed by irrigation with a solution containing trimethylamine N-oxide (TMAO), Trimethylamine N-oxide di-hydrate, a TMAO chemical derivative or a TMAO chemical analogue. In a preferred embodiment, the initial spray treatment is carried out with a solution containing an effective amount of TMAO which is between 0.01 to 10000 g per litre, more preferably between 0.1 to 1000 g per litre, between 0.1 to 100 g per litre, between 0.1 and 10 g per litre, between 0.1 g to 5 g per litre for said spray treatment. In another disclosure, the irrigation treatment is carried out with a solution containing an effective amount of TMAO which is between 0.01 to 10000 g per litre, more preferably between 0.1 to 1000 g per litre, between 0.1 to 100 g per litre, between 0.1 and 10 g per litre, between 0.1 g to 5 g per litre for said irrigation treatment.

In a preferred disclosure, the method for producing a drought tolerant plant comprises a first step wherein the seed is treated with a solution containing trimethylamine N-oxide (TMAO), Trimethylamine N-oxide di-hydrate, a TMAO chemical derivative or a TMAO chemical analogue followed by irrigation or spraying of the plant with a solution containing trimethylamine N-oxide (TMAO), Trimethylamine N-oxide di-hydrate, a TMAO chemical derivative or a TMAO chemical analogue. In a preferred disclosure, the initial seed treatment is carried out with an effective amount of TMAO which is between 0.01 to 10000 g per kg of seed, more preferably between 0.1 to 1000 g per kg of seed, between 0.1 to 100 g per kg of seed, between 0.1 and 10 g per kg seed or between 0.1 g to 5 g per kg seed. In another disclosure, the irrigation treatment is carried out with a solution containing an effective amount of TMAO which is between 0.01 to 10000 g per litre, more preferably between 0.1 to 1000 g per litre, between 0.1 to 100 g per litre, between 0.1 and 10 g per litre, between 0.1 g to 5 g per litre for said irrigation treatment.

A variety of seeds or bulbs may be used in the methods described herein including but are not limited to plants in the families' Solanaceae and Cucurbitaceae, as well as plants selected from the plant genera *Calibrachoa, Capsicum, Nicotiana, Nierembergia, Petunia, Solanum, Cucurbita, Cucumis, Citrullus, Glycine,* such as *Glycine max* (Soy), *Calibrachoa* × *hybrida, Capsicum annuum* (pepper), *Nicotiana tabacum* (tobacco), *Nierenbergia scoparia* (cupflower), *Petunia* ×*hybrida, Solanum lycopersicum* (tomato), *Solanum tuberosum* (potato), *Solanum melongena* (eggplant), *Cucurbita maxima* (squash), *Cucurbita pepo* (pumpkin, zucchini), *Cucumis metuliferus* (Horned melon) *Cucumis melo* (Musk melon), *Cucumis sativus* (cucumber) and *Citrullus lanatus* (watermelon). Various monocotyledonous plants, in particular those which belong to the family Poaceae, may be used with the methods described herein, including but not limited to, plants selected from the plant genera *Hordeum, Avena, Secale, Triticum, Sorghum, Zea, Saccharum, Oryza, Hordeum vulgare* (barley), *Triticum aestivum* (wheat), *Triticum aestivum* subsp. *spelta* (spelt), *Triticale, Avena sativa* (oats), *Secale cereale* (rye), *Sorghum bicolor* (sorghum), *Zea mays* (maize), *Saccharum officinarum* (sugarcane) and *Oryza sativa* (rice). Additional examples of plants in which water stress may be produced using the methods described herein include the followings. crops: buckwheat, beet, canola, rapeseed, sunflower, sugar cane, tobacco, and pea, etc.; vegetables: solanaceous vegetables such as paprika and potato; cucurbitaceous vegetables; cruciferous vegetables such as Japanese radish, white turnip, horseradish, kohlrabi, Chinese cabbage, cabbage, leaf mustard, broccoli, and cauliflower, asteraceous vegetables such as burdock, crown daisy, artichoke, and lettuce; liliaceous vegetables such as green onion, onion, garlic, and asparagus; ammiaceous vegetables such as carrot, parsley, celery, and parsnip; chenopodiaceous vegetables such as spinach, Swiss chard; lamiaceous vegetables such as *Perilla frutescens,* mint, basil; strawberry, sweet potato, *Dioscorea japonica,* colocasia; flowers; foliage plants; grasses; fruits: pomaceous fruits (apple, pear, Japanese pear, Chinese quince, quince, etc.), stone fleshy fruits (peach, plum, nectarine, *Prunus mume,* cherry fruit, apricot, prune, etc.), citrus fruits (*Citrus unshiu,* orange, tangerine, lemon, lime, grapefruit, etc.), nuts (chestnuts, walnuts, hazelnuts, almond, pistachio, cashew nuts, macadamia nuts, etc.), berries (blueberry, cranberry, blackberry, raspberry, etc.), grape, kaki fruit, olive, Japanese plum, banana, coffee, date palm, coconuts, etc.; and trees other than fruit trees; tea, mulberry, flowering plant, roadside trees (ash, birch, dogwood, Eucalyptus, *Ginkgo biloba,* lilac, maple, *Quercus,* poplar, Judas tree, *Liquidambar formosana,* plane tree, zelkova, Japanese arborvitae, fir wood, hemlock, juniper, *Pinus, Picea,* and *Taxus cuspidata*). Examples of plants in which water stress tolerance may be produced may include rice, corn, canola, soybean and wheat.

The aforementioned "plants" include transgenic plants, expressing other gene traits.

As used herein, "plants" means all dicotyledonous or monocotyledonous plants, including but is not limited to annual and perennial dicotyledonous or monocotyledonous plants and includes by way of example, but not by limitation, those of the genera *Glycine, Vitis, Asparagus, Populus, Pennisetum, Lolium, Oryza, Zea, Avena, Hordeum, Secale, Triticum, Sorghum, Saccharum* and *Lycopersicum.* The term plant may also include but not limited to the class of the Liliatae (Monocotyledoneae or monocotyledonous plants). The term includes the mature plants, seeds, shoots and seedlings, and parts, propagation material, plant organs, tissue, protoplasts, callus and other cultures, for example cell cultures derived from the above, and all other types of associations of plant cells which give functional or structural units. "Mature plants" means plants at any developmental stage beyond the seedling stage. Seedling means a young, immature plant in an early developmental stage.

Dicotyledonous plants includes but is not limited to the mature plants, seeds, shoots and seedlings, and parts, propagation material, plant organs, tissue, protoplasts, callus and other cultures, for example cell cultures derived from the above, and all other types of associations of plant cells which give functional or structural units. Mature plants means plants at any developmental stage beyond the seedling stage. Seedling means a young, immature plant in an early developmental stage.

As used herein "photosynthetic organisms" may include but is not limited to organisms such as *Arthrospira* spp., *Spirulina* spp., *Synechococcus elongatus, Synechococcus* spp., *Synechosystis* spp., *Synechosystis* spp., and *Spirulina plantensis, Calothrix* spp., *Anabaena flos-aquae, Aphanizomenon* spp., *Anabaena* spp., *Gleotrichia* spp., *Oscillatoria* spp. and *Nostoc* spp.; eukaryotic unicellular algae such as but not limited to *Chaetoceros* spp., *Chlamydomonas reinhardtii, Chlamydomonas* spp., *Chlorella vulgaris, Chlorella* spp., *Cyclotella* spp., *Didymosphenia* spp., *Dunaliella tertiolecta, Dunaliella* spp., *Botryococcus braunii, Botryococcus* spp., *Gelidium* spp., *Gracilaria* spp., *Hantzschia* spp., *Hematococcus* spp., *Isochrysis* spp., *Laminaria* spp., *Nannochloropsis spp., Navicula* spp., *Nereocystis luetkeana, Pleurochrysis* spp., *Postelsia palmaeformis,* and *Sargassum* spp.

In another disclosure, one or more methods are provided for the production of a product described herein may comprise: a) growing the plants described herein or obtainable by the methods of described herein and b) producing the product from or by the plants of the disclosure and/or parts, e.g. seeds, of these plants.

It is also disclosed a plant seed treated with an effective amount of trimethylamine N-oxide (TMAO), trimethylamine N-oxide di-hydrate, a TMAO chemical derivative, a TMAO chemical derivative, such as N,N-Dimethyldecylamine N-oxide (DDAO), or a TMAO chemical analogue, such as an N-methylated compound such as carnitine, sarcosine, N-methyl aspartic acid, N-methyl taurine thereof.

In a preferred disclosure the plant seed is treated with an effective amount of said TMAO between 0.1 to 1.000 g per kg of seeds, preferably between 0.1 to 100 g per kg of seeds, more preferably from 0.1 to 10 g per kg of seeds and even more preferably from 0.1 to 5 g per kg of seeds.

The description of a range should be considered to have specifically disclosed all the possible subranges as well as individual numerical values within that range.

In a further embodiment the method may comprise the steps a) growing the plants of the invention, b) removing the harvestable parts as defined above from the plants and c) producing the product from or by the parts of the plant or organism.

It is also disclosed a water stress tolerant plant produced by growing the plant seed disclosed herein.

The water stress tolerant plants or organisms may be produced at the site where the plant has been grown, the plants and/or parts thereof may be removed from the site where the plants have been grown to produce the product. Typically, the plant is grown, the desired harvestable parts are removed from the plant, if feasible in repeated cycles, and the product made from the harvestable parts of the plant. The step of growing the plant may be performed only once each time the methods of the invention is performed, while allowing repeated times the steps of product production e.g. by repeated removal of harvestable parts of the plants of the disclosure and if necessary further processing of these parts to arrive at the product. It is also possible that the step of growing the plants of the disclosure is repeated and plants or harvestable parts are stored until the production of the product is then performed once for the accumulated plants or plant parts. Also, the steps of growing the plants and producing the product may be performed with an overlap in time, even simultaneously to a large extend or sequentially. Generally the plants are grown for some time before the product is produced.

It is also disclosed a water stress tolerant plant produced through the application of at least one treatment of an effective amount of TMAO to a plant, plant part, photosynthetic organism or seed.

In a preferred embodiment, the water stress plant of the invention has a plant seed production that is 6% or more than the biomass, fruit or seed production of non-tolerant water stressed plants or photosynthetic organisms where an effective amount of TMAO has not been applied. In another preferred embodiment the water stress plant has a plant biomass, fruit or seed production that is 10%, 15%, 20%, 25%, 30%, 35%, 40%, 45%, 50%, 55%, 60%, 65%, 70%, 75%, 80%, 85%, 90%, 95%, 100%, 110%, 120%, 130%, 140%, 150%, including or more than the biomass, fruit or seed production of non-tolerant water stressed plants or photosynthetic organisms where an effective amount of TMAO has not been applied.

In a preferred embodiment, the seed production is between 6% and 30% or more, between 31% and 50% or more, between 51% and 70% or more, between 71% and 100% or more than the seed production of non-tolerant water stressed plants or photosynthetic organisms where an effective amount of TMAO has not been applied.

In another disclosurethe water stress tolerant plant produced through the application of at least one treatment of an effective amount of TMAO to a plant, plant part, photosynthetic organism and at least one second treatment of an effective amount of TMAO is applied to said water stress tolerant plant or photosynthetic organism.

In another preferred disclosureof the water stress tolerant plant, said at least one treatment of said effective amount of TMAO comprises two or more different compounds selected from the group consisting of TMAO di-hydrate, TMAO chemical derivative, or a TMAO chemical analogue.

In another preferred embodiment, the water stress tolerant plant of the invention is produced by a further treatment of salts or any other additive to a plant, plant part, photosynthetic organism or seed and growing said plant, plant part, photosynthetic organism or seed wherein a water stress tolerant plant or photosynthetic organism is produced.

In another disclosurethe products produced by the methods described herein are plant products such as, but not limited to, a foodstuff, feedstuff, a food supplement, feed supplement, fiber, cosmetic and/or pharmaceutical. Foodstuffs are regarded as compositions used for nutrition and/or for supplementing nutrition. Animal feedstuffs and animal feed supplements, in particular, are regarded as foodstuffs.

In another disclosurethe methods for the production are used to make agricultural products such as, but not limited to, plant extracts, proteins, amino acids, carbohydrates, fats, oils, polymers, vitamins, and the like. Please note that it is possible that a plant product consists of one or more agricultural products to a large extent.

Methods for producing transgenic plants or photosynthetic organisms tolerant to water stress which include but is not limited to stably introducing a construct into the plant or photosynthetic organism where the construct includes a gene or genes such as SEQ ID NO:1 or SEQ ID NO: 2 encoding a monooxygenase protein or FMO protein such as the FMO GS-OX5 protein are also disclosed. The overexpression, either constitutive or stress induced, of the monooxygenase protein mediates an increased TMAO expression in a plant or photosynthetic organism through the catalyzation of the oxidation of endogenous metabolites containing nucleophilic nitrogen. Additional disclosures may comprise a transgenic plant or organism overexpressing a water stress tolerant gene, such as SEQ ID NO:1 or SEQ ID NO: 2 encoding an FMO protein, where the gene is operably linked to a constitutive promoter or a stress inducible promoter and has been stably integrated into the plant or organism's genome under conditions suitable for the overexpression of a water stress tolerance protein.

It is also disclosed a method for producing a plant or photosynthetic organism with a tolerance to water stress, such as a monocotyledonous or dicotyledonous plant, which comprises introducing into and overexpressing in the plant or photosynthetic organism a nucleic acid or amino acid such as SEQ ID NO:1 or SEQ ID NO: 2 which codes for a monooxygenase protein, such as the FMO *GS-OX5* protein.

It is also disclosed a water stress tolerant plant or photosynthetic organism, wherein the water stress tolerant plant or photosynthetic organism overexpresses FMO proteins in the plant's or photosynthetic organism's nuclear genome or the plant's chloroplast genome.

Thus, it is disclosed a method of producing a water stress tolerant plant, wherein the method comprises transforming a plant with a sequence encoding a FMO protein operably linked to a promoter under conditions suitable for overexpression of the FMO protein in the plant of at least three times with respect to the expression level of the endogenous FMO protein, wherein the overexpression of the FMO protein coding sequence in said water stress tolerant plant catalyzes the oxidation of endogenous metabolites containing nucleophilic nitrogen.

In a preferred disclosure the FMO protein coding sequence encodes an amino acid sequence selected from SEQ ID NO: 2, SEQ ID NO: 4, SEQ ID NO:6, SEQ ID NO:8, SEQ ID NO:10, SEQ ID NO:12, SEQ ID NO:14, SEQ ID NO:16, SEQ ID NO:18, SEQ ID NO:20, SEQ ID NO:22, SEQ ID NO:24, SEQ ID NO: 26, SEQ ID NO:28, SEQ ID NO:30, SEQ ID NO:32, SEQ ID NO:34, SEQ ID NO:36, SEQ ID NO:38 SEQ ID NO:40, SEQ ID NO:42 and SEQ ID NO:43.

In another preferred disclosure the promoter is a constitutive promoter. In another preferred disclosure, the overexpression of the FMO protein coding sequence is constitutive overexpression. In a preferred disclosure, the FMO protein is overexpressed at least 8 times with respect to the expression levels of the endogenous FMO protein.

In another preferred disclosure, the promoter is a stress inducible promoter, more preferably, the stress is water stress.

In another disclosure, the overexpression of the FMO protein coding sequence is induced by a stress, preferably water stress.

The constitutive overexpression or stress induced overexpression of the monooxygenase or FMO protein mediates an increased TMAO expression in a plant or photosynthetic organism, increasing the plant or organism's tolerance to various forms of water stress when compared to wild type plants, wild type plant parts, wild type photosynthetic organisms or wild type plant cells. The monooxygenase or FMO protein may be overexpressed in the plant or photosynthetic organism as a whole or a part, is provided, for example in an organ, tissue, a cell, or a part of a plant cell, for example, in an organelle. The monooxygenase protein comprises an amino acid coding sequence having at least 80% identity with SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42 or SEQ ID NO: 43, and/or the 5'-untranslated region (5'UTR). The overexpression of the protein mediates an increased TMAO expression in a plant or photosynthetic organism, increasing the plant or organism's tolerance to various forms of water stress when compared to wild type plants. By way of example, human FMO1 and FMO3 proteins have an identity of 53% and 84% with the FMO3 proteins from rabbit (see Lawton et al, 1994, Archives of Biochemistry and Biophysics, Vol. 308, 254-257).

Further disclosures provide for a DNA construct comprising one or more FMO protein coding sequences operably linked to a constitutive promoter or a stress inducible promoter wherein the FMO proteins are stably integrated into a plant or photosynthetic organism DNA genome under conditions suitable for overexpression of the DNA construct in the plant or photosynthetic organism. The constitutive promoter or stress inducible promoter in the DNA construct induces overexpression of the FMO proteins in the plant or photosynthetic organism thereby mediating an increased TMAO expression in a plant or photosynthetic organism, increasing the plant or photosynthetic organism's tolerance to various forms of water stress when compared to wild type plants or wild type photosynthetic organisms.

It is disclosed DNA constructs for the overexpression of FMO proteins in the transgenic plants or photosynthetic organisms. Such DNA constructs may be represented as shown in Figures 4a, 4b, 5a, and 5b.

As shown in Figure 4a, a construct for overexpression of an FMO protein in an *Arabidopsis thaliana* plant is provided, where staring at the 5' end of the construct, a constitutive promoter coding sequence, such as *PRO_{NOS},* is provided with a transcription start site. A selectable marker, such as NPTII is provided with a transcription termination region, NOS ter on the 3'end of the selectable marker. A constitutive promoter, such as the *CaMv35S* promoter, (35S) is provided with a transcription start site. The FMO protein coding sequence *RCI5* (SEQ ID NO: 1 or SEQ ID NO:2) is provided with a transcription termination region, NOS ter on the 3'end of the FMO protein coding sequence. Each of these components is operably linked to the next, i.e., the constitutive promoter coding sequence, *PRO_{NOS},* is operably linked to the 5' end of the selectable marker, NPTII, protein sequence and the selectable marker protein sequence is operably linked to the 5' end of the *CaMv35S* constitutive promoter coding sequence which is operably linked to the 5' end of the FMO protein coding sequence RCI5. For overexpression, the expression vector pROK2 may be used. The DNA construct is then integrated into a plant or photosynthetic organism such as an *Arabidopsis thaliana* plant and photosynthetic organisms overexpressing the At FMO *GS-OX5* protein are produced, where the constitutive promoter induces the overexpression of the FMO protein. The overexpression of the FMO protein coding sequence in plant or photosynthetic organism catalyzes the oxidation of endogenous metabolites containing nucleophilic nitrogen.

As shown in Figure 4b, a construct for overexpression of an FMO protein in an *Arabidopsis thaliana* plant is provided, where staring at the 5' of the construct a promoter coding sequence, such as *PRO_{NOS},* is provided with a transcription start site. A selectable marker, such as NPTII is provided with a transcription termination region, NOS site on the 3'end of the selectable marker. A stress inducible promoter, such as the *PRO_{RD29A}* promoter, with a HindII site on the 5' end and BamHI site on the 3' end, is provided with a transcription start site. FMO protein coding sequence RCI5 (SEQ ID NO: 1 or SEQ ID NO:2) is provided with a transcription termination region, NOS ter site on the 3'end of the FMO protein coding sequence. Each of these components is operably linked to the next, i.e., the promoter coding sequence, *PRO_{NOS},* is operably linked to the 5' end of the selectable marker, NPTII, protein coding sequence and the selectable marker protein coding sequence is operably linked to the 5' end of the *PRO_{RD29A}* constitutive promoter coding sequence which is operably linked to the 5' end of the FMO protein coding sequence RCI5. For overexpression, the expression vector pROK2 may be used. The DNA construct is then integrated into a plant or photosynthetic organism such as an *Arabidopsis thaliana* plant and organisms overexpressing the At FMO *GS-OX5* protein are produced, where the stress inducible promoter induces the overexpression of the FMO protein. The overexpression of the FMO protein coding sequence in photosynthetic organism catalyzes the oxidation of endogenous metabolites containing nucleophilic nitrogen.

As shown in Figure 5a, a construct for overexpression of an FMO protein in a *Zea mays* plant is provided, where staring at the 5' a constitutive promoter coding sequence, such as the *Ubiquitin* promoter, is provided with a transcription start site. FMO protein coding sequence *SI FMO GX-OX1* (SEQ ID NO: 25 or SEQ ID NO:26) is provided with a transcription termination region, NOS ter site on the 3'end of the FMO protein coding sequence. A constitutive promoter, such as the *CaMv35S* promoter, (35S) is provided with a transcription start site. A selectable marker, such as hygromycin is provided with a transcription termination region, NOS ter on the 3'end of the selectable marker. Each of these components is operably linked to the next, i.e., the constitutive promoter coding sequence, Ubiquitin, is operably linked to the 5' end of the FMO protein coding sequence *ZM FMO,* the FMO protein coding sequence is operably linked to the 5' end of the constitutive promoter, such as the *CaMv35S* promoter, (35S) coding sequence which is operably linked to the 5' end of the selectable marker protein coding sequence. For overexpression, the expression vector pCAMBIA 1300 may be used. The DNA construct is then integrated into a plant or photosynthetic organism such as a *Zea mays* plant and organisms overexpressing the ZM FMO protein are produced, where the constitutive promoter induces the overexpression of the FMO protein and the overexpression of the FMO protein coding sequence in photosynthetic organism catalyzes the oxidation of endogenous metabolites containing nucleophilic nitrogen.

As shown in Figure 5b, a construct for overexpression of an FMO protein in a *Solanum lycopersicum* plant is provided, where staring at the 5' a stress inducible promoter, such as the PRO_{RD29A} promoter, is provided with a transcription start site. FMO protein coding sequence *SI FMO GS-OX1* (SEQ ID NO: 35, SEQ ID NO: 36, SEQ ID NO: 37 or SEQ ID NO: 38) is provided with a transcription termination region, NOS ter site on the 3'end of the FMO protein coding sequence. A constitutive promoter, such as the *CaMv35S* promoter, (35S) is provided with a transcription start site. A selectable marker, such as hygromycin is provided with a transcription termination region, NOS ter on the 3'end of the selectable marker. Each of these components is operably linked to the next, i.e., the stress inducible promoter coding sequence, *PRO_{RD29A}* promoter, is operably linked to the 5' end of the FMO protein coding sequence *SI FMO GS-OX1,* the FMO protein coding sequence is operably linked to the 5' end of the constitutive promoter, such as the *CaMv35S* promoter, (35S) coding sequence which is operably linked to the 5' end of the selectable marker protein coding sequence. For overexpression, the expression vector pCAMBIA 1300 may be used. The DNA construct is then integrated into a photosynthetic organism such as a *Solanum lycopersicum* plant and organisms overexpressing the *SI FMO GS-OX1* protein are produced, where the stress inducible promoter induces the overexpression of the FMO protein and the overexpression of the FMO protein coding sequence in photosynthetic organism catalyzes the oxidation of endogenous metabolites containing nucleophilic nitrogen.

It is also disclosed a DNA construct for the overexpression of an FMO protein coding sequences in photosynthetic organisms, wherein DNA construct comprises a promoter and the FMO protein coding sequence, wherein said promoter is operably linked to said FMO protein coding sequence, wherein the FMO protein coding sequence is selected from SEQ ID NO: 2, SEQ ID NO: 4, SEQ ID NO:6, SEQ ID NO:8, SEQ ID NO:10, SEQ ID NO:12, SEQ ID NO:14, SEQ ID NO:16, SEQ ID NO:18, SEQ ID NO:20, SEQ ID NO:22, SEQ ID NO:24, SEQ ID NO: 26, SEQ ID NO:28, SEQ ID NO:30, SEQ ID NO:32, SEQ ID NO:34, SEQ ID NO:36, SEQ ID NO:38, SEQ ID NO:40, SEQ ID NO:42 and SEQ ID NO:43.

As used herein, "nucleic acids" means biopolymers of nucleotides which are linked with one another via phosphodiester bonds (polynucleotides, polynucleic acids). Depending on the type of sugar in the nucleotides (ribose or deoxyribose), one distinguishes the two classes of the ribonucleic acids (RNA) and the deoxyribonucleic acids (DNA).

As introduced above, it is disclosed a method for producing plants tolerant to water stress, including but not limited to drought tolerance or excessive moisture, in plants wherein an application of trimethylamine N-oxide or "TMAO", wherein TMAO includes but is not limited to, TMAO dihydrate, TMAO chemical derivative, or a TMAO chemical analogue, to a plant or seed to reduce water stress in the plant when the plant is exposed to water stress conditions. This method of producing a plant tolerant to water stress is applicable to a variety of plants including monocotyledonous or dicotyledonous plants, including but not limited to transgenic plants. As used herein, transgenic plants include plants, or photosynthetic organism, which have been genetically modified to contain foreign DNA constructs as will be discussed further herein. The methods for producing a plant or organism tolerant to water stress may be applicable to the whole plant or organism or a part of a plant, for example in an organ, tissue, a cell or a part of a plant cell, for example in an organelle, which comprises introducing into, and expressing in, the plant or plant cell a nucleic acid which codes for a monooxygenase or FMO protein, and which mediates an increased production of endogenous TMAO and therefore a water stress tolerance, such as an increased tolerance to drought or an increased tolerance to excessive moisture.

Methylamines (e.g. trimethylamine N-oxide (TMAO)) can enhance protein folding and ligand binding and counteract perturbations by urea (e.g. in elasmobranchs and mammalian kidney), inorganic ions, and hydrostatic pressure in deep-sea animals (Yancey, 2005, cited supra).

It is also disclosed a method for water stress tolerance in a plant, a plant part, or a plant cell, where the method comprises the step of increasing the expression and/or activity of a monooxygenase protein in the plant, plant part, or plant cell in comparison to a wild type plant, wild type plant part or wild type plant cell.

As discussed above, the methods described herein are directed to methods for reducing water stress in a plant or organism by producing a plant or photosynthetic organism tolerant to water stress by overexpressing FMO to the plant that has been exposed to or will be exposed to water stress conditions. The effect of reducing the water stress of a plant or photosynthetic organism can be evaluated by comparing the above phenotypic indicators between a overexpressing FMO and a plant which does not overexpress FMO after the plants or photosynthetic organism are exposed to water stress conditions. Stages in which plants or photosynthetic organism overexpressing FMO can be exposed to the water stress conditions include, for example, all growth stages of plants, including a germination period, a vegetative growing period, a reproductive growing period and a harvesting period.

A variety of seeds or bulbs may be used in the methods described herein including but are not limited to plants in the families Solanaceae and Cucurbitaceae, as well as plants selected from the plant genera Calibrachoa, Capsicum, Nicotiana, Nierembergia, Petunia, Solanum, *Cucurbita, Cucumis, Citrullus, Glycine,* such as *Glycine max* (Soy), *Calibrachoa* x *hybrida, Capsicum annuum* (pepper), *Nicotiana tabacum* (tobacco), *Nierenbergia scoparia* (cupflower), *Petunia* x *hybrida, Solanum lycopersicum* (tomato), *Solanum tuberosum* (potato), *Solanum melongena* (eggplant), *Cucurbita maxima* (squash), *Cucurbita pepo* (pumpkin, zucchini), *Cucumis metuliferus* (Horned melon) *Cucumis melo* (Musk melon), *Cucumis sativus* (cucumber) and *Citrullus lanatus* (watermelon). Various monocotyledonous plants, in particular those which belong to the family Poaceae, may be used with the methods described herein, including but not limited to, plants selected from the plant genera Hordeum, Avena, Secale, Triticum, Sorghum, Zea, Saccharum, Oryza, *Hordeum vulgare* (barley), *Triticum aestivum* (wheat), *Triticum aestivum* subsp. *spelta* (spelt), x *Triticosecale* (Triticale), *Avena sativa* (oats), *Secale cereale* (rye), *Sorghum bicolor* (sorghum), *Zea mays* (maize), *Saccharum officinarum* (sugarcane) and *Oryza sativa* (rice). Additional examples of plants in which water stress may be produced using the methods described herein include the followings. crops: buckwheat, beet, canola, rapeseed, sunflower, sugar cane, tobacco, and pea, etc.; vegetables: solanaceous vegetables such as paprika and potato; cucurbitaceous vegetables; cruciferous vegetables such as Japanese radish, white turnip, horseradish, kohlrabi, Chinese cabbage, cabbage, leaf mustard, broccoli, and cauliflower, asteraceous vegetables such as burdock, crown daisy, artichoke, and lettuce; liliaceous vegetables such as green onion, onion, garlic, and asparagus; ammiaceous vegetables such as carrot, parsley, celery, and parsnip; chenopodiaceous vegetables such as spinach, Swiss chard; lamiaceous vegetables such as *Perilla frutescens,* mint, basil; strawberry, sweet potato, *Dioscorea japonica,* colocasia; flowers; foliage plants; grasses; fruits: pomaceous fruits (apple, pear, Japanese pear, Chinese quince, quince, etc.), stone fleshy fruits (peach, plum, nectarine, *Prunus mume,* cherry fruit, apricot, prune, etc.), citrus fruits (*Citrus unshiu,* orange, tangerine, lemon, lime, grapefruit, etc.), nuts (chestnuts, walnuts, hazelnuts, almond, pistachio, cashew nuts, macadamia nuts, etc.), berries (blueberry, cranberry, blackberry, raspberry, etc.), grape, kaki fruit, olive, Japanese plum, banana, coffee, date palm, coconuts, etc.; and trees other than fruit trees; tea, mulberry, flowering plant, roadside trees (ash, birch, dogwood, Eucalyptus, *Ginkgo biloba,* lilac, maple, *Quercus,* poplar, Judas tree, *Liquidambar formosana,* plane tree, zelkova, Japanese arborvitae, fir wood, hemlock, juniper, *Pinus, Picea,* and *Taxus cuspidate*). Examples of plants in which water stress tolerance may be produced may include rice, corn, canola, soybean and wheat. The aforementioned "plants" include transgenic plants, expressing other gene traits.

As used herein, "plants" means all dicotyledonous or monocotyledonous plants, including but not limited to the class of the Liliatae (Monocotyledoneae or monocotyledonous plants). The term includes the mature plants, seeds, shoots and seedlings, and parts, propagation material, plant organs, tissue, protoplasts, callus and other cultures, for example cell cultures derived from the above, and all other types of associations of plant cells which give functional or structural units. "Mature plants" means plants at any developmental stage beyond the seedling stage. Seedling means a young, immature plant in an early developmental stage.

Dicotyledonous plants includes the mature plants, seeds, shoots and seedlings, and parts, propagation material, plant organs, tissue, protoplasts, callus and other cultures, for example cell cultures derived from the above, and all other types of associations of plant cells which give functional or structural units. Mature plants means plants at any developmental stage beyond the seedling stage. Seedling means a young, immature plant in an early developmental stage.

"Plant" also comprises annual and perennial dicotyledonous or monocotyledonous plants and includes by way of example, but not by limitation, those of the genera *Glycine, Vitis, Asparagus, Populus, Pennisetum, Lolium, Oryza, Zea, Avena, Hordeum, Secale, Triticum, Sorghum, Saccharum* and *Lycopersicum.*

As discussed above, another disclosure provides a method for producing a plant or photosynthetic organism, such as a monocotyledonous or dicotyledonous plant, with a tolerance to water stress, which comprises introducing into and expressing in the plant or photosynthetic organism a nucleic acid or amino acid such as SEQ ID NO:1 or SEQ ID NO: 2, which codes for a monooxygenase protein, such as the FMO GS-OX5 protein. An example of the monooxygenase protein may include but is not limited to an amino acid sequence of SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42 or SEQ ID NO: 43, wherein the nucleotide sequence comprises at least one nucleic acid molecule. The amino acid sequence may have a percent identity of 80% or more of the sequences listed above and/or the 5'-untranslated region (5'UTR) in comparison with the original sequence.

The methods described herein also include a) introducing into a plant or photosynthetic organism cell a recombinant expression cassette comprising the nucleic acid molecule in an operable linkage with a promoter which is active in a plant or photosynthetic organisms; b) regenerating a plant or photosynthetic organism from the plant or photosynthetic organism cell, and c) expressing the nucleic acid molecule to generate or to increase a water tolerance in the plant or photosynthetic organism.

The disclosure also relates to a water stress tolerant plant produced by the methods disclosed herein.

The methods described herein further provide a transgenic photosynthetic organisms or a plant, comprising a nucleic acid sequence coding for an FMO protein (SEQ ID NOs: 1-43), a DNA expression cassette, or a vector comprising the DNA expression cassette, or comprising a cell comprising the nucleic acid molecule such as the FMO protein (SEQ ID NOs: 1-43), the expression cassette, or the vector. Examples may include generating a transgenic plant which is tolerant to water stress, which may comprise the nucleic acid molecule, such as an FMO protein coding sequence (SEQ ID NOs: 1-43), a DNA expression cassette, a vector comprising the expression cassette, or a cell comprising the nucleic acid molecule, the expression cassette, or the vector. A plant propagation material or composition may be generated comprising a nucleic acid molecule such as the FMO protein coding sequence (such as SEQ ID NOs: 1-43), a DNA expression cassette comprising the FMO protein coding sequence, or a vector comprising the expression cassette, or a cell comprising the nucleic acid molecule, the expression cassette, or the vector to provide a drought tolerant plant, plant part, or plant cell.

As discussed above and shown in Figure 2, the FMO proteins described herein may include an exogenous nucleotide sequence which codes for an amino acid sequence having at least 50%, 60%, 70%, 75%, 80% 85%, 90%, 95% identity with SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42 or SEQ ID NO: 43, into a plant or photosynthetic organism, a part of a plant, or a plant cell, and expressing the nucleotide sequence in the plant or photosynthetic organism, the part of the plant, or the plant cell. Further the nucleotide sequence may be increased in the plant or photosynthetic organism, the part of the plant, or the plant cell when compared with the original, or wild-type plant, part of the plant, or plant cell.

The methods of overexpression and increase of a FMO protein as described herein, including the one or more DNA constructs for use in the overexpression of FMO protein, stable integration of the FMO protein into a plant or photosynthetic organism DNA genome and overexpression of the DNA construct in the plant or photosynthetic organism, may be used in a variety of plants, including but not limited to: soybean, potato, cotton, rape, oilseed rape, canola, sunflower, alfalfa, clover, banana, blackberry, blueberry, strawberry, raspberry, cantaloupe, carrot, cauliflower, coffee, cucumber, eggplant, grape, honeydew, lettuce, mango, melon, onion, papaya, pepper, pineapple, pumpkin, spinach, squash, tobacco, tomato, tomatillo, watermelon, apple, peach, pear, cherry, plum, broccoli, cabbage, cauliflower, Brussels sprouts, kohlrabi, currant, avocado, orange, lemon, grapefruit, tangerine, artichoke, cherry, walnut, peanut, endive, leek, arrowroot, beet, cassava, tumip, radish, yam, sweet potato; pea, bean, sugarcane, turfgrass, Miscanthus, switchgrass, wheat, maize, sweet com, rice, millet, sorghum, barley, and rye as well as various types of photosynthetic organisms including but not limited to diatoms, eukaryotic algae and cyanobacteria

As shown in Figure 2, genes with high identity to *FMO GS-OX5* mediate similar functions. As shown in Figure 2 the genes, used nucleic acids or expressed proteins may have 40% or more identity, including but not limited to at least 50%, at least 60%, at least 70%, at least 80%, at least 85%, at least 90%, at least 95%, at least 97%, at least 98%, at least 99% or more identity, in comparison with the respective *FMO GS-OX5* sequence of *Arabidopsis* (At1g12140) (SEQ ID NO: 1) [cDNA sequence with UTR] or the protein sequence SEQ ID NO.: 2). The genes with the highest homologies to At1g12140 from *Solanum lycopersicum SIFMO GS-OX1* (Solyc06g060610) (SEQ ID NO: 37 and SEQ ID NO: 38), *SlFMO GS-OX2* (AK324297.1), *Vitis vinifera VvFMO GS-OX3-1* (SEQ ID NO: 21 and SEQ ID NO: 22) (LOC100242032), *VvFMO GS-OX3-2* (LOC100255688) (SEQ ID NO: 19 SEQ ID NO: 20), *VvFMO GS-OX3-3* (LOC100255688) (SEQ ID NO: 17 and SEQ ID NO: 18), *Populus trichocarpa PtFMO-GS-OX3* (XM_002329873.1) (SEQ ID NO: 27 and SEQ ID NO: 28), *PtFMO GS-OX2* (XM_002318967.1) (SEQ ID NO: 29 and SEQ ID NO: 30), *PtFMO GS-OX1* (XP002318210.1), *Oryza sativa OsFMO-OX* (Os10g40570.1) (SEQ ID NO: 15 and SEQ ID NO: 16), *Glycine max GmFMO* (Glyma11g03390.1) (SEQ ID NO: 33 and SEQ ID NO: 34), *Cucumus sativus CsFMO* GS-OX3-1 (LOC101227975) (SEQ ID NO: 11 and SEQ ID NO: 12), *CsFMO GS-OX3-2* (LOC101220079) (SEQ ID NO: 9 and SEQ ID NO: 10), *CsFMO GS-OX3-3* (LOC101220318) (SEQ ID NO: 7 and SEQ ID NO: 8), *CsFMO GS-OX3-4* (LOC101212991) (SEQ ID NO: 5 and SEQ ID NO: 6), *Brassica rapa subsp. pekinensis BrFMO GS-OX1* (FJ376070.1), *Medicago truncatula MtFMO GS-OX5* (MTR_5g012130) (SEQ ID NO: 13 and SEQ ID NO: 14), *Zea mays ZmFMO* (GRMZM2G089121_P01) (SEQ ID NO: 25 and SEQ ID NO: 26), *Gossypium hirsutum GhFMO-1* (DQ122185.1) SEQ ID NO: 23 and SEQ ID NO: 24) *Homo sapiens HsFMO-3* (NP_001002294.1) (SEQ ID NO: 39 and SEQ ID NO: 40) and *Oryctolagus cuniculus OcFMO-5* (NP_001075714.1) SEQ ID NO: 41 and SEQ ID NO: 42) probably exert similar functions in the plant or photosynthetic organism as FMO GS-OX5 polypeptide from *Arabidopsis* (*AtFMO GS-OX5*)*.* As discussed above, Figure 2 provides a phylogenetic tree of the polypeptide sequences listed above of FMO from *Arabidopsis thaliana,* grapevine, *Populus trichocarpa,* rice, soybean, melon, tomato, sorghum, corn, wheat, barley, human and rabbit.

As shown in Figure 2, the equivalent expression of FMO proteins may be expected for sequences having 40% or more identity, including but not limited to at least 50%, at least 60%, at least 70%, at least 80%, at least 85%, at least 90%, at least 95%, at least 97%, at least 98%, at least 99% or more identity, in comparison with other FMO sequences such as the respective FMO *GS-OX5* sequence of *Arabidopsis.*

As used herein, "FMO protein" or "FMO polypeptide" means a protein with 100% of the full or parts of the sequence, which mediates an increased TMAO expression in a plant or photosynthetic organism through the catalyzation of the oxidation of endogenous metabolites containing nucleophilic nitrogen and conferring enhanced water stress tolerance when expressed in plants or photosynthetic organisms. "FMO protein" is understood as meaning a sequence which comprises an N-terminal domain, a flavin-monooxygenase domain and a C-terminal domain (Li et al., Plant Physiol. 148(3):1721-33 (2008). For example, the polypeptide which is employed in the method, has an activity which is involved in the water stress defense responses and increases endogenous TMAO. A polypeptide can be identified as a FMO if it is capable of catalyzing the conversion of trimethylamine (TMA) to TMAO in the presence of FAD and NADPH. The activity can be determined in an vitro assay as shown, for instance, in example 2.2 of PCT application WO20100348261.

The term "over-expression", as used herein, means that a given cell produces an increased number of a certain protein relative to a normal cell. For the purposes of this disclosure, the original wild-type expression level might also be zero, i.e. absence of expression or immeasurable expression. It will be understood that the FMO protein that is overexpressed in the cells according to the methods of the disclosure can be of the same species as the plant cell wherein the overexpression is being carried out or it may derive from a different species. In the case wherein the endogenous FMO protein is overexpressed, the levels of the FMO protein are at least three times with respect to the same polypeptide which is endogenously produced by the plant cell. In the case wherein an heterologous FMO protein is overexpressed, the levels of the heterologous FMO protein are of at least three times the levels of the endogenous FMO protein. In a preferred disclosure, the FMO protein is overexpressed at least 4, at least 5, at least 6, at least 7, at least 8, at least 9, at least 10 or more times with respect to the endogenous FMO protein.

In one disclosure, the plant disclosed herein has been transformed by introduction of an homologous or heterologous FMO protein so that the FMO activity in a cell lysate from said plant is at least 2-fold. 3-fold, 4-fold, 5-fold, 6-fold, 7-fold, 8-fold, 9-fold, 10-fold, 20-fold, 30-fold, 40-fold, 50-fold, 60-fold, 70-fold, 80-fold, 90-fold, 100-fold or more with respect to the FMO activity in the non-transformed plant. The FMO activity in the cell lysate can be determined as described in example 2.2 of PCT application WO20100348261, wherein extract is contacted with TMA in the presence of FAD and NADPH and the production of TMAO is determined.

In another disclosure, the overexpression of the homologous or heterologous FMO protein should be enough to increase the endogenous TMAO levels at least at least 2-fold. 3-fold, 4-fold, 5-fold, 6-fold, 7-fold, 8-fold, 9-fold, 10-fold, 20-fold, 30-fold, 40-fold, 50-fold, 60-fold, 70-fold, 80-fold, 90-fold, 100-fold or more with respect to the endogenous TMAO levels measured in the absence of stress, wherein the stress is abiotic stress, including drought stress, saline stress, osmotic stress, clod stress, UV stress and heat stress. The levels of TMAO can be determined by any method known in the art, including, for instance, the method described on the PCT application WO20100348261 based on the reduction of TMAO to TMA in the presence of TiC13 and detecting the amount of TMA formed in the reaction.

The FMO protein is encoded for example, by a nucleic acid molecule comprising a nucleic acid molecule selected from the group consisting of: (a) nucleic acid molecule which codes for at least one polypeptide comprising the sequence shown in the nucleic acid sequence which codes for the FMO protein, such as FMO *GS-OX5* protein (SEQ ID NO: 1) or the functional parts of the protein, expresses and mediates an increased water stress tolerance, including an increased tolerance to drought. As discussed in the methods above, the FMO protein is introduced into and expressed in the plant or photosynthetic organism or plant cell or a part thereof, or the FMO protein may be expressed endogenously according to the methods described herein.

By way of example the nucleic acid sequence which codes for the FMO protein may be selected from the group consisting of: (a) a nucleic acid molecule which codes for at least one polypeptide comprising the sequence shown in SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42 or SEQ ID NO: 43; (b) a nucleic acid molecule which comprises at least one polynucleotide of the sequence shown in SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41 or SEQ ID NO: 44; (c) a nucleic acid molecule which codes for a polypeptide whose sequences has at least 50%, at least 60%, at least 70%, at least 80%, at least 85%, at least 90%, at least 95%, at least 97%, at least 98% or at least 99%, identity with any one of the sequences shown in paragraph (a) or (b) listed above where the nucleic acid molecule listed in paragraphs (a) and (b) above have the same or a similar biological function as a nucleic acid molecule encoding a polypeptide; (e) nucleic acid molecule according to (a) to (d) which codes for a fragment or an epitope of the sequences as shown in paragraphs (a) and (b), wherein the fragment is a functional fragment which confers drought stress tolerance; (f) a nucleic acid molecule which codes for a polypeptide which is recognized by a monoclonal antibody directed against a polypeptide which is encoded by the nucleic acid molecules as shown in (a) to (d); (g) nucleic acid molecule which hybridizes under stringent conditions with the complement of a nucleic acid molecule as shown in (a) to (d); and (h) nucleic acid molecule which can be isolated from a DNA library using a nucleic acid molecule as shown in (a) to (d) or their part-fragments of at least 15 nt, 20 nt, 30 nt, 50 nt, 100 nt, 200 nt or 500 nt, as probe under stringent hybridization conditions; (i) a nucleic acid encoding the same FMO protein as the nucleic acids sequences listed in paragraphs (a) to (d) above, but differing from the sequences of (a) to (d) above due to the degeneracy of the genetic code; or a complementary sequence thereof.

Other heterologous proteins encoded by the chimeric gene include polypeptides that form immunologically active epitopes, and enzymes that catalyze conversion of intracellular metabolites, with the consequent build-up of selected metabolites in the cells.

As used herein, the term "sequence(s)" is used for simplification reasons, and refers, depending on the context, to the nucleic acid and/or amino acid sequences disclosed herein. The skilled worker will know from the context what they refer to. The term "DNA fragment" as used in herein is understood as meaning portions of the DNA which code for a protein when this biological activity consists in mediating an increase in the drought stress tolerance. The term "fragments of the protein" as used herein refers to portions of the protein whose biological activity comprises mediating an increase in the drought stress tolerance in plants.

"Polypeptide quantity" as used herein means for example, the number of molecules, or moles, of FMO polypeptide molecules in an organism, a tissue, a cell or a such as substrate or agrochemical product, phytosanitary produccell compartment. Increasing the polypeptide quantity means the molar increase in the number of the respective polypeptides in an organism, a tissue, a cell or a cell compartment. For example, by one of the methods described herein below, in comparison with a suitable control, for example, the wild type (control plant) of the same genus and species to which this method has not been applied, under otherwise identical conditions (such as, for example, culture conditions, age of the plants and the like). The increase in this context amounts to at least 5%, at least 10% or at least 20%, as well as at least 40% or 60%, at least 70% or 80%, and at least 90%, 95%, 99%, 100%, more than 100%, including 150%, 200% or 300%.

Identity between two nucleic acid sequences is understood as meaning the identity of the nucleic acid sequence over in each case the entire sequence length, which is calculated by comparison with the aid of the program algorithm GAP (Wisconsin Package Version 10.0, University of Wisconsin, Genetics Computer Group (GCG), Madison, USA; Altschul et al., Nucleic Acids Res. 25, 3389 (1997)), setting the following parameters:\

| | |
|---|---|
| Gap weight: 50 | Length weight: 3 |
| Average match: 10 | Average mismatch: 0 |

For example, a sequence which has at least 80% identity with the sequence SEQ ID NO: 1 at the nucleic acid level is understood as meaning a sequence which, upon comparison with the sequence SEQ ID NO: 1 by the above program algorithm with the above parameter set, has at least 80% identity.

Identity between two polypeptides is understood as meaning the identity of the amino acid sequence over in each case the entire sequence length which is calculated by comparison with the aid of the program algorithm GAP (Wisconsin Package Version 10.0, University of Wisconsin, Genetics Computer Group (GCG), Madison, USA), setting the following parameters:

| | |
|---|---|
| Gap weight: 8 | Length weight: 2 |
| Average match: | 2.912 Average mismatch: -2.003 |

For example, a sequence which has at least 80% identity at the polypeptide level with the sequence SEQ ID NO: 2 is understood as meaning a sequence which, upon comparison with the sequence SEQ ID NO: 2 by the above program algorithm with the above parameter set, has at least 80% identity.

The drought stress tolerance of a plant or organism as described herein is obtained by introducing and overexpressing a nucleic acid sequence such as but not limited to SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42 or SEQ ID NO: 43. Additionally, it is also possible to increase the endogenous overexpression or activity of these sequences in a plant or organism by methods known to one skilled in the art. For example an increase in endogenous overexpression may be obtained by mutating a UTR region, such as the 5'-UTR, a promoter region, a genomic coding region for the active center, for binding sites, for localization signals, for domains, clusters and the like, such as, for example, of coding regions for the N-terminal, the FMO protein or the C-terminal domains. The endogenous expression or activity may be increased in accordance with the disclosure by mutations which affect the secondary, tertiary or quaternary structure of the protein.

Mutations can be inserted for example, by an EMS mutagenesis. Domains can be identified by suitable computer programs such as, for example, SMART or InterPRO, for example as described in Andersen P., The Journal of Biol. Chemistry, 279, 38 or 39053, (2004) or Mudgil, Y., Plant Physiology, 134, 59, (2004), and literature cited therein. The suitable mutants can then be identified for example by TILLING (for example as described by Henikoff, S., et al., Plant Physiol. 135: 630-6 (2004)).

The introduction and overexpression of a sequence according to the methods described herein into a plant or photosynthetic organism, or increasing or modifying or mutating an endogenous sequence, if appropriate of one or both untranslated regions, in a plant or photosynthetic organism is combined with increasing the polypeptide quantity, activity or function of other resistance factors, such as a *Bax inhibitor 1 protein* (BI-1), such as a Bax inhibitor 1 protein from *Hordeum vulgare* (GenBank Acc.-No.: AJ290421), from, *Nicotiana tabacum* (GenBank Acc.-No.: AF390556), rice (GenBank Acc.-No.: AB025926), *Arabidopsis* (GenBank Acc.-No.: AB025927) or tobacco and oilseed rape (GenBank Acc.-No.: AF390555, Bolduc N et al. (2003) Planta 216, 377 (2003)) or of ROR2 (for example from barley (GenBank Acc.-No.: AY246906), SnAP34 (for example, from barley (GenBank Acc.-No.: AY247208) and/or of the lumenal binding protein BiP for example from rice (GenBank Acc.-No. AF006825). An increase can be achieved for example, by mutagenesis or overexpression of a transgene, inter alia.

A nucleic acid molecule, as used herein, comprises the untranslated sequence at the 3' and at the 5' terminus of the coding gene region: at least 500, or 200, or 100 nucleotides of the sequence upstream of the 5' terminus of the coding region and at least 100, or 50, or 20 nucleotides of the sequence downstream of the 3' terminus of the coding gene region.

Moreover, nucleic acid sequences are isolated nucleic acid sequences. An "isolated" nucleic acid molecule is separated from other nucleic acid molecules which are present in the natural origin of the nucleic acid. An "isolated" nucleic acid preferably contains no sequences which naturally flank the nucleic acid in the genomic DNA of the organism from which the nucleic acid originates (for example sequences which are located at the 5' and 3' termini of the nucleic acid; however, this does not affect the above mentioned disclosures comprising 5'- and 3'-UTR regions). In different disclosures, the isolated molecule may comprise for example less than approximately 5 kb, 4 kb, 3 kb, 2 kb, 1 kb, 0.5 kb or 0.1 kb of nucleotide sequences which naturally flank the nucleic acid molecule in the genomic DNA of the cell from which the nucleic acid originates. All the nucleic acid molecules mentioned here may be for example RNA, DNA or cDNA.

The nucleic acid molecules may be isolated using standard techniques of molecular biology and the sequence information provided herein. Using comparative algorithms, it is possible to identify for example a homologous sequence, or homologous, conserved sequence regions, at the DNA or amino acid level. Essential portions of this sequence or the entire homologous sequence can be used as hybridization probe using standard hybridization techniques (such as, for example, described in Sambrook et al.: Molecular Cloning. A Laboratory Manual, 2nd ed. Cold Spring Harbor Laboratory Press, Cold Spring Harbor, N.Y., 1989) for isolating further nucleic acid sequences which are useful in the method from other organisms by screening cDNA libraries and/or genomic libraries.

Moreover, a nucleic acid molecule or a part thereof can be isolated by means of polymerase chain reaction ("PCR"), where oligonucleotide primers based on the sequences specified herein or parts thereof are used (for example, it is possible to isolate a nucleic acid molecule comprising the complete sequence or part thereof by means of PCR using oligonucleotide primers which have been generated on the basis of the very same sequence). For example, mRNA can be isolated from cells (for example by the guanidinium thiocyanate extraction method by Chirgwin et al., Biochemistry 18, 5294 (1979)) and cDNA prepared therefrom by means of reverse transcriptase (for example Moloney MLV reverse transcriptase, obtainable from Gibco/BRL, Bethesda, Md. or AMV reverse transcriptase, available from Seikagaku Amerika, Inc., St. Petersburg, Fla.). Synthetic oligonucleotide primers for the amplification by means of PCR can be generated on the basis of the sequences disclosed herein. A nucleic acid can be amplified using cDNA or, alternatively, genomic DNA as template and suitable oligonucleotide primers by means of standard PCR amplification techniques. The nucleic acid amplified thus can be cloned into a suitable vector and characterized by means of DNA sequence analysis. Oligonucleotides which correspond to a nucleotide sequence coding for a protein can be prepared by synthetic standard methods, for example, using an automated DNA synthesizer.

As used herein "introduction" or "to introduce" comprises all methods, including but not limited to as transfection, transduction or transformation, which are suitable for directly or indirectly introducing, into a plant or a cell, compartment, tissue, organ or seed, a nucleic acid sequence, or generating it therein. The introduction may lead to a transient or to a stable presence of a nucleic acid sequence.

It is also described herein the product derived from a plant or photosynthetic organism comprising a nucleic acid molecule, a DNA expression cassette, or a vector comprising the expression cassette, or comprising a cell comprising the nucleic acid molecule, the expression cassette, or the vector, or plant which is tolerant to drought stress, obtained by the method comprising using the nucleic acid molecule, a DNA expression cassette, a vector comprising the expression cassette, or a cell comprising the nucleic acid molecule, the expression cassette, or the vector, from a plant or photosynthetic organism producible by the method described herein or from a plant, plant part, transgenic seed, photosynthetic organism or transgenic plant.

It is also disclosed a tissue culture of cells produced from the plant of the disclosure (the drought stress tolerant produced by the method disclosed herein), wherein the cells of the tissue culture are produced from a plant part chosen from leaves, pollen, embryos, cotyledons, hypocotyl, meristematic cells, roots, root tips, pistils, anthers, flowers, and stems. As a skill person understands, said leaves, pollen embryos, cotyledons, hypocotyl, meristematic cells, roots, root tips, pistils, anthers, flowers, and stems comprise the FMO protein.

It is also disclosed a plant regenerated from the tissue culture of the invention. As a skill person can understand said regenerated plant comprises a FMO protein.

It is also disclosed a method for producing a photosynthetic organism overexpressing one or more FMO proteins coding sequences in said photosynthetic organism, which comprises:
growing a plant transformed with a sequence encoding an FMO protein operably linked to a promoter wherein the FMO protein and promoter are stably integrated into said photosynthetic organism's nuclear genome or said plant's chloroplast genome under conditions suitable for overexpression of the FMO protein in the photosynthetic organism, wherein the overexpression of the FMO protein coding sequence in said photosynthetic organism catalyzes the oxidation of endogenous metabolites containing nucleophilic nitrogen.

In a preferred disclosure, the one or more FMO protein comprises one or more amino acid sequences selected from SEQ ID NO: 2, SEQ ID NO: 4, SEQ ID NO:6, SEQ ID NO:8, SEQ ID NO:10, SEQ ID NO:12, SEQ ID NO:14, SEQ ID NO:16, SEQ ID NO:18, SEQ ID NO:20, SEQ ID NO:22, SEQ ID NO:24, SEQ ID NO: 26, SEQ ID NO:28, SEQ ID NO:30, SEQ ID NO:32, SEQ ID NO:34, SEQ ID NO:36, SEQ ID NO:38 SEQ ID NO:40, SEQ ID NO:42 and SEQ ID NO:43

In another preferred disclosure, the one or more FMO protein comprises at least one functionally equivalent variant of an amino acid sequence having at least 95% identity with SEQ ID NO: 2, SEQ ID NO: 4, SEQ ID NO:6, SEQ ID NO:8, SEQ ID NO:10, SEQ ID NO:12, SEQ ID NO:14, SEQ ID NO:16, SEQ ID NO:18, SEQ ID NO:20, SEQ ID NO:22, SEQ ID NO:24, SEQ ID NO: 26, SEQ ID NO:28, SEQ ID NO:30, SEQ ID NO:32, SEQ ID NO:34, SEQ ID NO:36, SEQ ID NO:38 SEQ ID NO:40, SEQ ID NO:42 and SEQ ID NO:43..

In another preferred disclosure, wherein the one or more FMO protein comprises at least one functionally equivalent variant of an amino acid sequence having at least 90% identity with SEQ ID NO: 2, SEQ ID NO: 4, SEQ ID NO:6, SEQ ID NO:8, SEQ ID NO:10, SEQ ID NO:12, SEQ ID NO:14, SEQ ID NO:16, SEQ ID NO:18, SEQ ID NO:20, SEQ ID NO:22, SEQ ID NO:24, SEQ ID NO: 26, SEQ ID NO:28, SEQ ID NO:30, SEQ ID NO:32, SEQ ID NO:34, SEQ ID NO:36, SEQ ID NO:38 SEQ ID NO:40, SEQ ID NO:42 and SEQ ID NO:43.

In another preferred disclosure, the one or more FMO protein comprises at least one functionally equivalent variant of an amino acid sequence having at least 80% identity with SEQ ID NO: 2, SEQ ID NO: 4, SEQ ID NO:6, SEQ ID NO:8, SEQ ID NO:10, SEQ ID NO:12, SEQ ID NO:14, SEQ ID NO:16, SEQ ID NO:18, SEQ ID NO:20, SEQ ID NO:22, SEQ ID NO:24, SEQ ID NO: 26, SEQ ID NO:28, SEQ ID NO:30, SEQ ID NO:32, SEQ ID NO:34, SEQ ID NO:36, SEQ ID NO:38 SEQ ID NO:40, SEQ ID NO:42 and SEQ ID NO:43.

In another preferred disclosure, wherein the one or more FMO protein comprises at least one functionally equivalent variant of an amino acid sequence having at least 70% identity with SEQ ID NO: 2, SEQ ID NO: 4, SEQ ID NO:6, SEQ ID NO:8, SEQ ID NO:10, SEQ ID NO:12, SEQ ID NO:14, SEQ ID NO:16, SEQ ID NO:18, SEQ ID NO:20, SEQ ID NO:22, SEQ ID NO:24, SEQ ID NO: 26, SEQ ID NO:28, SEQ ID NO:30, SEQ ID NO:32, SEQ ID NO:34, SEQ ID NO:36, SEQ ID NO:38 SEQ ID NO:40, SEQ ID NO:42 and SEQ ID NO:43

In another preferred disclosure, the one or more FMO protein comprises at least one functionally equivalent variant of an amino acid sequence having at least 60% identity with SEQ ID NO: 2, SEQ ID NO: 4, SEQ ID NO:6, SEQ ID NO:8, SEQ ID NO:10, SEQ ID NO:12, SEQ ID NO:14, SEQ ID NO:16, SEQ ID NO:18, SEQ ID NO:20, SEQ ID NO:22, SEQ ID NO:24, SEQ ID NO: 26, SEQ ID NO:28, SEQ ID NO:30, SEQ ID NO:32, SEQ ID NO:34, SEQ ID NO:36, SEQ ID NO:38 SEQ ID NO:40, SEQ ID NO:42 and SEQ ID NO:43.

In another preferred disclosure, the one or more FMO protein comprises at least one functionally equivalent variant of an amino acid sequence having at least 50% identity with SEQ ID NO: 2, SEQ ID NO: 4, SEQ ID NO:6, SEQ ID NO:8, SEQ ID NO:10, SEQ ID NO:12, SEQ ID NO:14, SEQ ID NO:16, SEQ ID NO:18, SEQ ID NO:20, SEQ ID NO:22, SEQ ID NO:24, SEQ ID NO: 26, SEQ ID NO:28, SEQ ID NO:30, SEQ ID NO:32, SEQ ID NO:34, SEQ ID NO:36, SEQ ID NO:38 SEQ ID NO:40, SEQ ID NO:42 and SEQ ID NO:43.

"Functionally equivalent variant", is understood to mean all those proteins derived from FMO sequence by modification, insertion and/or deletion or one or more amino acids, whenever the function is substantially maintained, particularly the ability to generate TMAO from TMO (trimethylamine).

Another disclosureprovides a method for the production of a product, herewith the method for the production of a product, comprising: a) growing a plant comprising the nucleic acid molecule disclosed herein, a DNA expression cassette as disclosed herein, or a vector comprising the expression cassette, or comprising a cell comprising the nucleic acid molecule, the expression cassette, or the vector or obtainable by the method of the disclosure; b) producing the product from or by the plant and/or part, or seeds of the plant.

Another disclosure is the method for the production of a product, which comprises: a) growing a plant comprising the nucleic acid molecule, a DNA expression cassette, or a vector comprising the expression cassette, or comprising a cell comprising the nucleic acid molecule, the expression cassette, or the vector or obtainable by the method and removing the plant, plant part, transgenic plant, or transgenic seed; and b) producing the product from or by the plant, plant part, transgenic plant, or transgenic seed of the plant.

As used herein "epitope" is understood as meaning the regions of an antigen which determine the specificity of the antibodies (the antigenic determinant). Accordingly, an epitope is the portion of an antigen which actually comes into contact with the antibody.

Such antigenic determinants are those regions of an antigen to which the T-cell receptors react and, as a consequence, produce antibodies which specifically bind the antigenic determinant/epitope of an antigen. Accordingly, antigens, or their epitopes, are capable of inducing the immune response of an organism with the consequence of the formation of specific antibodies which are directed against the epitope. Epitopes consist for example of linear sequences of amino acids in the primary structure of proteins, or of complex secondary or tertiary protein structures. A hapten is understood as meaning an epitope which is dissociated from the context of the antigen environment. Although haptens have by definition an antibody directed against them, haptens are, under certain circumstances, not capable of inducing an immune response in an organism, for example, after an injection. To this end, haptens are coupled with carrier molecules. An example which may be mentioned is dinitrophenol (DNP), which, after coupling to BSA (bovine serum albumin), has been used for generating antibodies which are directed against DNP (Bohn, A., König, W. (1982), Immunology 47 (2), 297).

Haptens are substances (frequently low-molecular weight substances or small substances) which, while they themselves do not trigger immune response, will indeed trigger such a response when coupled to a large molecular carrier. The antibodies generated thus also include those which can bind to the hapten alone.

Another disclosure relates to an antibody against an FMO protein polypeptide as described, in particular to a monoclonal antibody which binds an FMO polypeptide which comprises an amino acid sequence or consists thereof, as shown in the sequences shown in SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42 or SEQ ID NO: 43.

These antibodies can be used for identifying and isolating polypeptides disclosed herein, from organisms, including plants, such as monocotyledonous plants, as well as dicotyledonous plants. The antibodies can either be monoclonal, polyclonal or synthetic in nature or else consist of antibody fragments such as Fab, Fv or scFv fragments, which are formed by proteolytic degradation. "Single chain" Fv (scFv) fragments are single-chain fragments which, linked via a flexible linker sequence, only comprise the variable regions of the heavy and light antibody chains. Such scFv fragments can also be produced as recombinant antibody derivatives. A presentation of such antibody fragments on the surface of filamentous phages makes possible the direct selection, from combinatory phage libraries, of scFv molecules which bind with high affinity. Monoclonal antibodies can be obtained in accordance with the method described by Köhler and Milstein in Nature 256, 495 (1975).

Screening cDNA libraries or genomic libraries of other organisms, including the plant and photosynthetic organisms mentioned herein, which are suitable as transformation hosts, using the nucleic acid sequences described in SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41 or SEQ ID NO: 44; or parts of the same as probe is also a method known to the skilled worker for identifying homologs in other species. In this context, the probes derived from the nucleic acid sequence as shown in SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: , SEQ ID NO: 39, SEQ ID NO: 41 or SEQ ID NO: 44; have a length of at least 20 bp, or at least 50 bp, or at least 100 bp, or at least 200 bp, or at least 400 bp. The probe can also be one or more kilobases in length, for example, 1 kb, 1.5 kb or 3 kb. A DNA strand which is complementary to the sequences described in SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41 or SEQ ID NO: 44 or a fragment of same strand with a length of between 20 bp and several kilobases may also be employed for screening the libraries.

In an additional disclosure, the FMO protein coding sequences may hybridize under standard conditions with the nucleic acid molecules described by SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41 or SEQ ID NO: 44 ; and which code for FMO proteins, with the nucleic acid molecules which are complementary to the above or with parts of the above and which, as complete sequences, code for polypeptides which essentially have identical properties, preferred functional properties, to the polypeptides described in SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42 or SEQ ID NO: 43 may also be used.

As used herein "standard hybridization conditions" is to be understood in the broad sense and means, depending on the application, stringent or else less stringent hybridization conditions. Such hybridization conditions are described, inter alia, in Sambrook J, et al. 1989, pages 9.31-9.57 or in Current Protocols in Molecular Biology, John Wiley & Sons, N.Y. (1989), 6.3.1-6.3.6. One skilled in the art, based on his technical knowledge, would choose hybridization conditions which allow him to differentiate between specific and unspecific hybridizations.

For example, the conditions during the wash step can be selected from among low-stringency conditions (with approximately 2*SSC at 50°C.) and high-stringency conditions (with approximately 0.2*SSC at 50°C, preferably at 65°C) (20*SSC: 0.3M sodium citrate, 3M NaCl, pH 7.0). Moreover, the temperature during the wash step can be raised from low-stringency conditions at room temperature, approximately 22°C, to higher-stringency conditions at approximately 65°C. The two parameters, salt concentration and temperature, can be varied simultaneously or else singly, keeping in each case the other parameter constant. During the hybridization, it is also possible to employ denaturant agents such as, for example, formamide or SDS. In the presence of 50% formamide, the hybridization is preferably carried out at 42°C. Some examples of preferred conditions for hybridization and wash step are detailed herein below:
Hybridization conditions can be selected for example, among the following conditions:
4*SSC at 65°C,
6*SSC at 45°C,
6*SSC, 100 [mu]g/ml denatured fragmented fish sperm DNA at 68°C,
6*SSC, 0.5% SDS, 100 [mu]g/ml denatured salmon sperm DNA at 68°C,
6*SSC, 0.5% SDS, 100 [mu]g/ml denatured fragmented salmon sperm DNA, 50% formamide at 42°C,
50% formamide, 4*SSC at 42°C,
50% (vol/vol) formamide, 0.1% bovine serum albumin, 0.1% Ficoll, 0.1% polyvinylpyrrolidone, 50 mM sodium phosphate buffer pH 6.5, 750 mM NaCl, 75 mM sodium citrate at 42°C,
2* or 4*SSC at 50°C (low-stringency condition),
30 to 40% formamide, 2* or 4*SSC at 42°C (low-stringency condition), or
500 mN sodium phosphate buffer pH 7.2, 7% SDS (g/V), 1 mM EDTA, 10 [mu]g/ml single stranded DNA, 0.5% BSA (g/V) (see Church and Gilbert, Proc. Natl. Acad. Sci. U.S.A. 81:1991 (1984))
(2) Wash steps can be selected for example, among the following conditions:
a) 0.015 M NaCl/0.0015 M sodium citrate/0.1% SDS at 50°C,
b) 0.1*SSC at 65°C,
c) 0.1*SSC, 0.5% SDS at 68°C,
d) 0.1*SSC, 0.5% SDS, 50% formamide at 42°C,
e) 0.2*SSC, 0.1% SDS at 42°C, or
f) 2*SSC at 65°C (low-stringency condition).

Other examples of hybridization conditions are selected as follows:
A hybridization buffer comprising formamide, NaCl and PEG 6000 is chosen. The presence of formamide in the hybridization buffer destabilizes double-strand nucleic acid molecules, whereby the hybridization temperature can be lowered to 42°C without thereby reducing the stringency. The use of salt in the hybridization buffer increases the renaturation rate of a duplex DNA, in other words the hybridization efficiency. Although PEG increases the viscosity of the solution, which has a negative effect on the renaturation rates, the presence of the polymer in the solution increases the concentration of the probe in the remaining medium, which increases the hybridization rate. The composition of the buffer is:
Hybridization buffer:
250 mM sodium phosphate buffer pH 7.2
1 mM EDTA
7% SDS (g/v)
250 mM NaCl
10 [mu]g/ml ssDNA
5% polyethylene glycol (PEG) 6000
40% formamide

The hybridizations are carried out for approximately 12 hours at 42°C, for example overnight. The filters are then washed 3* with 2*SSC+0.1% SDS for in each case approximately 10 minutes.

As used herein the "modification" of nucleotide sequences or amino acid sequences comprises mutating them, or mutations. For the purposes described here, "mutations" means the modification of the nucleic acid sequence of a gene variant in a plasmid or in the genome of an organism. Mutations can be generated for example as the consequence of errors during replication, or by mutagens. The spontaneous mutation rate in the cell genome of organisms is very low; however, the skilled person in the art knows a multiplicity of biological, chemical or physical mutagens and methods of mutating nucleotide sequences in a random or targeted manner, and therefore ultimately potentially also for modifying the amino acid sequences which they encode.

Mutations comprise substitutions, additions, deletions of one or more nucleic acid residues. Substitutions are understood as meaning the exchange of individual nucleic acid bases, where one distinguishes between transitions (substitution of a purine base for a purine base, and of a pyrimidine base for a pyrimidine base) and transversions (substitution of a purine base for a pyrimidine base, or vice versa).

Addition or insertion is understood as meaning the incorporation of additional nucleic acid residues in the DNA, which may result in reading-frame shifts. In the case of such reading frame shifts, one distinguishes between in-frame insertions/additions and out-of-frame insertions. In the case of the in-frame insertions/additions, the reading frame is retained, and a polypeptide which is lengthened by the number of the amino acids encoded by the inserted nucleic acids is formed. In the case of out-of-frame insertions/additions, the original reading frame is lost, and the formation of a complete and functional polypeptide is in many cases no longer possible, which of course depends on the site of the mutation.

Deletions describe the loss of one or more base pairs, which likewise leads to in-frame or out-of-frame reading-frame shifts and the consequences which this entails with regard to the formation of an intact protein.

One skilled in the art would be familiar with the mutagenic agents (mutagens) which can be used for generating random or targeted mutations and both the methods and techniques which may be employed. Such methods and mutagens are described for example in van Harten A. M. ("Mutation breeding: theory and practical applications", Cambridge University Press, Cambridge, UK (1998)), Friedberg E., Walker G., Siede W. ("DNA Repair and Mutagenesis", Blackwell Publishing (1995)), or Sankaranarayanan K., Gentile J. M., Ferguson L. R. ("Protocols in Mutagenesis", Elsevier Health Sciences (2000)).

Customary methods and processes of molecular biology such as, for example, the *in vitro* mutagenesis kit, "LA PCR in vitro Mutagenesis Kit" (Takara Shuzo, Kyoto), or PCR mutagenesis using suitable primers, may be employed for introducing targeted mutations.

As mentioned above, a multiplicity of chemical, physical and biological mutagens exists. Those mentioned herein below are given by way of example, but not by limitation.

Chemical mutagens may be divided according to their mechanism of action. Thus, there are base analogs (for example 5-bromouracil, 2-aminopurine), mono- and bifunctional alkylating agents (for example monofunctional agents such as ethyl methyl sulfonate, dimethyl sulfate, or bifunctional agents such as dichloroethyl sulfite, mitomycin, nitrosoguanidine-dialkyl nitrosamine, N-nitrosoguanidine derivatives) or intercalating substances (for example acridine, ethidium bromide).

Examples of physical mutagens are ionizing radiations. Ionizing radiations are electromagnetic waves or corpuscular radiations which are capable of ionizing molecules, i.e. of removing electrons from them. The ions which remain are in most cases highly reactive so that they, in the event that they are formed in live tissue, are capable of inflicting great damage for example to the DNA and thereby inducing mutations (at low intensity). Examples of ionizing radiations are gamma radiation (photon energy of approximately one mega electron volt MeV), X-ray radiation (photon energy of several or many kilo electron volt keV) or else ultraviolet light (UV light, photon energy of over 3.1 eV). UV light causes the formation of dimers between bases, thymidine dimers are most common, and these give rise to mutations.

Examples of the generation of mutants by treating the seeds with mutagenizing agents may include ethyl methyl sulfonate (EMS) (Birchler, J. A. and Schwartz, D., Biochem. Genet. 17 (11-12), 1173 (1979); Hoffmann, G. R., Mutat. Res. 75 (1), 63 (1980)) or ionizing radiation there has now been added the use of biological mutagens, for example transposons (for example Tn5, Tn903, Tn916, Tn1000, May B. P. et al., Proc. Natl. Acad. Sci USA. 100 (20), 11541 (2003)) or molecular-biological methods such as the mutagenesis by T-DNA insertion (Feldman, K. A., Plant Journal 1, 71 (1991), Koncz, C., et al., Plant Mol. Biol. 20: 963-76 (1992)).

To generate mutated gene variants, chemical or biological mutagens may be used. Among the chemical agents, it is especially preferred to generate mutants by using EMS (ethyl methyl sulfonate) mutagenesis. Among the generation of mutants using biological mutagens, the T-DNA mutagenesis or the transposon mutagenesis may be used.

Thus, for example, it is also possible to employ polypeptides in the methods described herein, which are obtained as the result of a mutation of a nucleotide sequence such as SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 39, SEQ ID NO: 39, SEQ ID NO: 41 or SEQ ID NO: 44.

As used herein the term "recombinant" means for example with regard to a nucleic acid sequence, an expression cassette or a vector comprising the nucleic acid sequence or an organism transformed with the nucleic acid sequence, expression cassette or vector, all those constructs or organisms which are the result of recombinant methods and in which either (a) the FMO protein nucleic acid sequence or (b) a genetic control sequence, for example a promoter, which is operably linked with the FMO nucleic acid encoding sequence, or (c) (a) and (b) are not located in their-natural genetic environment or have been modified by recombinant methods, it being possible for the modification to be, for example, a substitution, addition, deletion, or insertion of one or more nucleotide residue(s).

Natural genetic environment means the natural chromosomal locus in the organism of origin, or the presence in a genomic library. In the case of a genomic library, the natural genetic environment of the nucleic acid sequence is preferably retained at least in part. The environment flanks the nucleic acid sequence at least on one side and has a sequence length of at least 50 bp, at least 500 bp, at least 1000 bp, at least 5000 bp. A naturally occurring expression cassette-for example the naturally occurring combination of the FMO protein constitutive promoter with the corresponding FMO protein gene-becomes a recombinant expression cassette when the latter is modified by means of non-natural, synthetic ("artificial") methods such as, for example, mutagenization. Suitable methods have been described (U.S. Pat. No. 5.565.350; WO 00/15815).

As used herein, the term "transgenic" refers to an organism, e.g., a plant, plant cell, callus, plant tissue, or plant part that exogenously contains the nucleic acid, recombinant construct, vector or expression cassette described herein or a part thereof which is introduced by non-essentially biological processes, preferably by Agrobacteria transformation. The recombinant construct or a part thereof is stably integrated into a chromosome, so that it is passed on to successive generations by clonal propagation, vegetative propagation or sexual propagation.

A transgenic plant, plant cell or tissue for the purposes of the methods and products described here is thus understood as meaning that an exogenous FMO nucleic acid, recombinant construct, vector or expression cassette including one or more FMO nucleic acids is integrated into the genome by means of gene technology.

In another preferred said one or more FMO protein coding sequences comprises at least one exogenous nucleic acid molecule which codes for at least one polypeptide comprising the sequence as shown in SEQ ID NO: 2, SEQ ID NO: 4, SEQ ID NO:6, SEQ ID NO:8, SEQ ID NO:10, SEQ ID NO:12, SEQ ID NO:14, SEQ ID NO:16, SEQ ID NO:18, SEQ ID NO:20, SEQ ID NO:22, SEQ ID NO:24, SEQ ID NO: 26, SEQ ID NO:28, SEQ ID NO:30, SEQ ID NO:32, SEQ ID NO:34, SEQ ID NO:36, SEQ ID NO:38 SEQ ID NO:40, SEQ ID NO:42 and SEQ ID NO:43.

In another preferred disclosuresaid one or more FMO protein coding sequences comprises an exogenous nucleic acid molecule which codes for a polypeptide wherein the polypeptide has at least 90% identity to the sequence as shown in SEQ ID NO: 2, SEQ ID NO: 4, SEQ ID NO:6, SEQ ID NO:8, SEQ ID NO:10, SEQ ID NO:12, SEQ ID NO:14, SEQ ID NO:16, SEQ ID NO:18, SEQ ID NO:20, SEQ ID NO:22, SEQ ID NO:24, SEQ ID NO: 26, SEQ ID NO:28, SEQ ID NO:30, SEQ ID NO:32, SEQ ID NO:34, SEQ ID NO:36, SEQ ID NO:38 SEQ ID NO:40, SEQ ID NO:42 and SEQ ID NO:43.

In another preferred disclosure, said one or more FMO protein coding sequences comprises an exogenous nucleic acid molecule which codes for a polypeptide wherein the polypeptide has at least 80% identity to the sequence as shown in SEQ ID NO: 2, SEQ ID NO: 4, SEQ ID NO:6, SEQ ID NO:8, SEQ ID NO:10, SEQ ID NO:12, SEQ ID NO:14, SEQ ID NO:16, SEQ ID NO:18, SEQ ID NO:20, SEQ ID NO:22, SEQ ID NO:24, SEQ ID NO: 26, SEQ ID NO:28, SEQ ID NO:30, SEQ ID NO:32, SEQ ID NO:34, SEQ ID NO:36, SEQ ID NO:38 SEQ ID NO:40, SEQ ID NO:42 and SEQ ID NO:43.

In another preferred disclosure, said one or more FMO protein coding sequences comprises an exogenous nucleic acid molecule which comprises at least one polypeptide wherein the polypeptide has at least 70% identity to the sequence as shown in SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41 or SEQ ID NO: 44.

The term "exogenous" nucleic acid refers to a nucleic acid that has been introduced in a plant by means of gene technology. An "exogenous" nucleic acid can either not occur in a plant in its natural form, be different from the nucleic acid in question as found in a plant in its natural form, or can be identical to a nucleic acid found in a plant in its natural form, but integrated not within its natural genetic environment. The corresponding meaning of "exogenous" is applied in the context of protein expression. For example, a transgenic plant containing a transgene, i.e., an exogenous nucleic acid, may, when compared to the expression of the endogenous gene, encounter a substantial increase of the expression of the respective gene or protein in total. A transgenic plant according to the methods and products described herein includes an exogenous FMO nucleic acid integrated at any genetic loci and optionally the plant may also include the endogenous gene within the natural genetic background.

In a disclosure, a method for increasing drought stress tolerance in a plant or organism may include increasing the levels of TMAO by increasing the expression of a FMO protein or a functional fragment thereof, or a splice variant thereof in the plant or photosynthetic organism, wherein the FMO protein is encoded by (i) an exogenous nucleic acid having at least 50% identity, at least 60% identity, at least 70% sequence identity, at least 80 %, at least 90%, at least 95 %, at least 98%, at least 99% sequence identity, or even 100% sequence identity with SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41 or SEQ ID NO: 44 ; or a splice variant thereof; (ii) an exogenous amino acid encoding a protein having at least 50% identity, at least 60%, at least 70% sequence identity, at least 80 %, at least 90%, at least 95 %, at least 98%, at least 99% sequence identity, or even 100% sequence identity with SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 39, SEQ ID NO: 41, a functional fragment thereof; the encoded protein confers drought stress tolerance relative to control plants; (iii) an exogenous nucleic acid capable of hybridizing under stringent conditions with a complementary sequence of any of the nucleic acids according to (i) or (ii); encoding a FMO protein; wherein the amino acid molecule codes for a polypeptide which has essentially identical properties to the polypeptide described in SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 39; SEQ ID NO: 41; the encoded protein confers enhanced drought stress tolerance relative to control plants; and / or by (iv) an exogenous nucleic acid encoding the same FMO protein as any of the nucleic acids of (i) to (iii) above, but differing from the nucleic acids of (i) to (iii) above due to the degeneracy of the genetic code is a further disclosure.

The increase of the drought stress tolerance in the plant or photosynthetic organism can also be obtained by manipulating the expression of the plant's own protein, i.e. the endogenous protein, which corresponds to the protein, or of an endogenous nucleotide sequence, which constitutes a sequence, and which may also comprise the 5'- and/or 3'-UTR region. It is, then, an endogenous nucleotide or peptide sequence which mediates an increase of the drought stress tolerance or it is an amino acid sequence which codes for such a protein. This manipulation can be achieved by any modification of the sequence, often a mutation, but also for example by a modification of the promoter DNA sequence of the protein-encoding gene. Such a modification, which results in a modified, increased, expression rate of the endogenous gene, can be effected by means of deletion or insertion of DNA sequences. As a rule, a modification of the 5'-UTR region in total and/or of the promoter sequence of endogenous genes will lead to a modification of the expressed amount of the gene and/or the function of the expressed gene or gene product, and therefore also to a modification of the activity which can be detected in the cell or in the plants. The modification of the 5'-UTR region in total and/or of the promoter sequence of the endogenous gene may also lead to a modification of the amount of, and/or the function of, a protein in the cell. Please note that an increase in the expression or function is understood as meaning herein both the activation or enhancement of the expression or function of the endogenous protein, including a de novo expression, increase of protein activity, and an increase or enhancement by expression of a transgenic protein or factor.

Another method of increasing the activity and the content of the endogenous protein may include up-regulate transcription factors which are involved in the transcription of the corresponding endogenous gene, for example by means of overexpression. The means for overexpressing transcription factors are known to the skilled worker and are also disclosed for proteins within the context of the present disclosure.

Moreover, an increased expression of the endogenous gene as described herein can be achieved by a regulator protein, which is not present in the untransformed organism, interacting with the promoter of these genes. Such a regulator may take the form of a chimeric protein which consists of a DNA binding domain and a transcription activator domain, as described for example in WO 96/06166.

The protein-encoding cDNA (or the mRNA including the UTR sequence(s)) sequence for expression in a cell of a plant or photosynthetic organism that, upon expression of the DNA to RNA and transcription of the RNA to produce an encoded peptide or polypeptide, enhances the ability of the plant or photosynthetic organism or plant cell to withstand an abiotic or biotic stress, or enhances the yield or value of the plant or photosynthetic organism, or a crop or product produced from the plant or photosynthetic organism.

The introduction into a plant or organism of an expression cassette comprising, for example, the FMO protein (SEQ ID NO: 1-44) into a photosynthetic organism or plant cells, plant tissue, plant organs such as chloroplast, parts or seeds thereof can advantageously be carried out using vectors which comprise the expression cassettes. The expression cassette can be introduced into the vector (for example the pROK2 vector, or the pCAMBIA vector) via a suitable restriction cleavage site. The plasmid obtained is first introduced into *E. coli* cells. Correctly transformed E. *coli* cells are selected, cultured, and the recombinant plasmid is obtained using methods with which the skilled worker is familiar. Restriction analysis and sequencing may be used for verifying the cloning step.

The vectors may take the form of, for example, plasmids, cosmids, phages, viruses or else agrobacteria and may be introduced by means of plasmid vectors. Examples of vectors are those which make possible a stable integration of the expression cassette into the host genome.

A variety of methods (Keown et al., Methods in Enzymology 185, 527(1990)) are available for the introduction of a desired construct into a plant or organism, which is referred to as transformation (or transduction or transfection). Thus, the DNA or RNA can be introduced for example, directly by means of microinjection or by bombardment with DNA-coated microparticles. Also, it is possible to chemically permeabilize the cell, for example using polyethylene glycol, so that the DNA can reach the cell by diffusion. The DNA can also be introduced into the cell by means of protoplast fusion with other DNA-comprising units such as minicells, cells, lysosomes or liposomes. A further suitable method of introducing DNA is electroporation, where the cells are reversibly permeabilized by means of an electrical pulse. Examples of such methods have been described in Bilang et al., Gene 100, 247 (1991); Scheid et al., Mol. Gen. Genet. 228, 104 (1991); Guerche et al., Plant Science 52, 111 (1987); Neuhause et al., Theor. Appl. Genet. 75, 30 (1987); Klein et al., Nature 327, 70(1987); Howell et al., Science 208, 1265 (1980); Horsch et al., Science 227, 1229 (1985); DeBlock et al., Plant Physiology 91, 694 (1989); *"*Methods for Plant Molecular Biology" (Weissbach and Weissbach, eds.) Academic Press Inc. (1988); and *"*Methods in Plant Molecular Biology" (Schuler and Zielinski, eds.) Academic Press Inc. (1989).

In plants, the above-described methods for the transformation and regeneration of plants from plant tissue or plant cells are exploited for the purposes of transient or stable transformation. Suitable methods are mainly protoplast transformation by means of polyethylene-glycol-induced DNA uptake, the biolistic method with the gene gun, known as the particle bombardment method, electroporation, the incubation of dry embryos in DNA-comprising solution, and microinjection.

Transformation may also be effected by bacterial infection by means of *Agrobacterium tumefaciens* or *Agrobacterium rhizogenes.* The methods are described for example in Horsch et al. Science 225, 1229 (1985).

If agrobacteria are used for transformation, the expression cassette may be integrated into specific plasmids, which may either be a shuttle or intermediate vector or a binary vector. If a Ti or Ri plasmid is used for the transformation, at least the right border, but in most cases both the right and the left border, of the Ti or Ri plasmid T-DNA as flanking region is linked with the expression cassette to be introduced.

Binary vectors are capable of replicating in a variety of organisms including but not limited to *E. coli* and in agrobacterium. As a rule, they comprise a selection marker gene and a linker or polylinker flanked by the right and left T-DNA border sequence. They can be transformed directly into agrobacterium (Holsters et al., Mol. Gen. Genet. 163, 181 (1978)). The selection marker gene, for example the *nptII* gene, which mediates resistance to kanamycin, permits transformed agrobacteria to be selected. The agrobacterium which, in the present case, acts as the host organism should already comprise a helper Ti plasmid with the vir region, which is required for transferring the T-DNA to the plant cell. An agrobacterium thus transformed can be used for transforming plant cells. The use of T-DNA for the transformation of plant cells has been studied and described in great detail (EP 120 516; Hoekema, in "The Binary Plant Vector System", Offsetdrukkerij Kanters B. V., Alblasserdam, Chapter V; An et al. EMBO J. 4, 277 (1985)). Various binary vectors are known and in some cases commercially available, such as, for example, pBI101.2 or pBIN19 (Clontech Laboratories, Inc. USA).

In the event that DNA or RNA is injected or electroporated into plant cells, the plasmid used need not meet particular requirements. Simple plasmids such as those from the pUC series may be used. If intact plants are to be regenerated from the transformed cells, it is necessary for an additional selection marker gene to be located on the plasmid.

Stably transformed cells, i.e. those which comprise the introduced DNA integrated into the DNA of the host cell, can be distinguished from untransformed cells when a selection marker is constituent of the introduced DNA (McCormick et al, Plant Cell Reports 5, 81 (1986)). For example, any gene which is capable of mediating a resistance to antibiotics or herbicides (such as kanamycin, G 418, bleomycin, hygromycin or phosphinothricin) may act as a marker. Transformed cells which express such a marker gene are capable of surviving in the presence of concentrations of a suitable antibiotic or herbicide which destroy an untransformed wildtype. Examples include the bar gene, which mediates resistance to the herbicide phosphinothricin (Rathore et al., Plant Mol. Biol. 21 (5), 871 (1993)), the nptII gene, which mediates resistance to kanamycin, the hpt gene, which mediates resistance to hygromycin, or the EPSP gene, which mediates resistance to the herbicide glyphosate. The resulting plants can be bred and hybridized in the customary manner. Two or more generations should be cultivated in order to ensure that the genomic integration is stable and hereditary.

Additional methods may be described in Jones et al. ("Techniques for Gene Transfer", in "Transgenic Plants", Vol. 1, Engineering and Utilization, edited by Kung S. D. and Wu R., Academic Press, p. 128-143 (1993), and in Potrykus, Annu. Rev. Plant Physiol. Plant Molec. Biol. 42, 205 (1991)). It is preferred to clone the construct to be expressed into a vector which is suitable for transforming *Agrobacterium tumefaciens,* for example into pBin 19 (Bevan et al., Nucl. Acids Res. 12, 8711 (1984)).

When a transformed plant cell has been generated, an intact plant can be obtained using methods known to one skilled in the art. An example of a starting material used here are callus cultures. The formation of shoot and root from this as yet undifferentiated cell biomass can be induced in a known manner. The plantlets obtained can be planted out and bred.

A person skilled in the art also knows methods for regenerating plant parts and intact plants from plant cells. For example, methods described by Fennell et al., Plant Cell Rep, 11, 567 (1992); Stoeger et al., Plant Cell Rep. 14, 273 (1995); Jahne et al., Theor. Appl. Genet. 89, 525 (1994), are used for this purpose.

The recombinant nucleic acid molecules described herein comprise the following elements in 5'-3' orientation: regulatory sequences of a promoter which is active in plant cells, a DNA sequence in operative linkage therewith, if appropriate, regulatory sequences which, in the plant cell, may act as transcription, termination and/or polyadenylation signals in operable linkage therewith.

In the recombinant expression constructs/expression cassettes, a nucleic acid molecule whose expression (transcription and, if appropriate, translation) generates a FMO protein is in operable linkage with at least one genetic control element (for example a promoter) which ensures overexpression in plants. If the expression construct is to be introduced directly into the plant or photosynthetic organism and the FMO protein generated therein in plants or photosynthetic organisms, then plant-specific genetic control elements (for example promoters) are preferred. However, the FMO protein can also be generated in other organisms or in vitro and then introduced into the plant. In this context, preference is given to all prokaryotic or eukaryotic genetic control elements (for example promoters) which permit the overexpression in the plant selected in each case for the production.

A recombinant vector construct or expression construct/cassette is provided comprising: (i) a nucleic acid having at least 50% identity, at least 60% identity, at least 70% sequence identity, at least 80 %, at least 90%, at least 95 %, at least 98%, at least 99% sequence identity, or even 100% sequence identity with SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41 or SEQ ID NO: 44 ; or a splice variant thereof; (ii) an amino acid coding for a protein having at least 50% identity, at least 60% identity, at least 70% sequence identity, at least 80 %, at least 90%, at least 95 %, at least 98%, at least 99% sequence identity, or even 100% sequence identity with SEQ ID NOs: 2, 4, 6, 8, 10, 12, 14, 16, 18, 20, 22, 24, 26, 28, 30, 32, 34, 36, 38, 39 or 41; the encoded protein confers enhanced drought stress tolerance relative to control plants; (iii) a nucleic acid capable of hybridizing under stringent conditions with a complementary sequence of any of the nucleic acids according to (i) or (ii); encoding an FMO protein; wherein the nucleic acid molecule codes for a polypeptide which has essentially identical properties to the polypeptide described in SEQ ID NOs: 2, 4, 6, 8, 10, 12, 14, 16, 18, 20, 22, 24, 26, 28, 30, 32, 34, 36, 38, 39 or 41; the encoded protein confers enhanced drought stress tolerance relative to control plants; and / or (iv)a nucleic acid encoding the same FMO protein as any of the nucleic acids of (i) to (iii) above, but differing from the nucleic acids of (i) to (iii) above due to the degeneracy of the genetic code, operably linked with (b) a promoter and (c) a transcription termination sequence.

Arrangements are those in which the nucleic acid sequence to be expressed recombinantly is positioned after the sequence which acts as the promoter, so that the two sequences are bonded covalently with one another. In this context, it is the distance between the promoter sequence and the nucleic acid sequence to be expressed recombinantly is less than 200 base pairs, or less than 100 base pairs, or less than 50 base pairs.

The generation of a functional linkage and the generation of an expression cassette can be carried out by means of customary recombination and cloning techniques as described for example in Sambrook J. (1989), in Silhavy T. J., Berman M. L. and Enquist L. W. "Experiments with Gene Fusions", Cold Spring Harbor Laboratory, Cold Spring Harbor (N.Y.) (1984), in Ausubel F. M. et al., "Current Protocols in Molecular Biology", Greene Publishing Assoc. and Wiley Interscience (1987) and in Gelvin et al., in "Plant Molecular Biology Manual" (1990). However, it is also possible to position, between the two sequences, further sequences which exert for example the function of a linker with specific restriction enzyme cleavage sites, or of a signal peptide. The insertion of sequences may also lead to the expression of fusion proteins. It is preferred that the expression cassette, consisting of a linkage of promoter and nucleic acid sequence to be expressed, can be present in vector-integrated form and inserted into a plant genome by, for example, transformation.

The method described herein can advantageously be combined with other methods which bring about a pathogen resistance (for example against insects, fungi, bacteria, nematodes and the like), stress tolerance or another improvement of the plant characteristics. Examples are mentioned inter alia in Dunwell J. M., J. Exp. Bot. 51, (Spec No) 487 (2000).

The nucleic acid molecules described herein may comprise nucleic acid molecules coding for FMO *GS-OX5* proteins from *Arabidopsis* according to the polynucleotides SEQ. ID NO: 1, and the nucleic acid sequences which are complementary thereto as shown in Figure 2, and the sequences which are derived due to the degeneracy of the genetic code, where the nucleic acid molecules do not consist of SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41 or SEQ ID NO: 44. The nucleic molecules described herein may comprise nucleic acid molecules coding for FMO *GS-OX3* proteins from cucumber plants according to the polynucleotides SEQ ID NOs: 5, 7, 9, 11, and the nucleic acid sequences which are complementary thereto, and the sequences which are derived due to the degeneracy of the genetic code, where the nucleic acid molecules do not consist of SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41 or SEQ ID NO: 44.

Transgenic expression cassettes may also be developed for the expression of FMO proteins where the cassettes may comprise one of the nucleic acid sequences nucleic acid molecule including but not limited to SEQ ID No: 1 SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41 or SEQ ID NO: 44; or a fragment thereof. In the transgenic expression cassettes, the nucleic acid sequence coding for the FMO proteins from *Arabidopsis* is linked with at least one genetic control element as defined above in such a manner that the expression (transcription and, if appropriate, translation) can be effected in any organism, usually in dicotyledonous plants. Genetic control elements which are suitable for this purpose are described above. The transgenic expression cassettes may also comprise further functional elements as defined above.

Such expression cassettes may comprise a nucleic acid sequence which is essentially identical to a nucleic acid molecule as shown in SEQ ID No.:1 SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41 or SEQ ID NO: 44; or a fragment thereof, where the nucleic acid sequence is in sense orientation or in antisense orientation relative to a promoter and can therefore lead to the expression of sense or antisense RNA, the promoter being a promoter which is active in plants, usually a promoter which can be induced by pathogen attack. Also provided herein are transgenic vectors which encompass the transgenic expression cassettes.

A promoter is a DNA region, which includes sequences sufficient to cause transcription of an associated (downstream) sequence. The promoter may be regulated, i.e., not constitutively acting to cause transcription of the associated sequence. If inducible, there are sequences present therein which mediate regulation of expression so that the associated sequence is transcribed only when an inducer molecule is present. The promoter may be any DNA sequence which shows transcriptional activity in the chosen plant cells, plant parts, or plants. The promoter may be inducible or constitutive. It may be naturally-occurring, may be composed of portions of various naturally-occurring promoters, or may be partially or totally synthetic. Also, the location of the promoter relative to the transcription start may be optimized. Many suitable promoters for use in plants or photosynthetic organisms are well known in the art, as are nucleotide sequences, which enhance expression of an associated expressible sequence.

A variety of promoters may be used in the methods described herein including a drought-inducible promoter, an epidermis promoter, a mesophyll-specific promoter, or a stress induced promoter (for example *RD29* (Singh et al. Plant Cell Rep 30:1019-1028(2011)). The promoter may be selected from the group consisting of a promoter induced by: osmotic stress, drought stress, cold stress, heat stress, oxidative stress, nutrient deficiency, infection by a fungus, infection by an oomycete, infection by a virus, infection by a bacterium, nematode infestation, pest infestation, weed infestation, and herbivory.

The promoters can be selected based on the desired outcome. That is, the nucleic acids can be combined with constitutive, tissue-preferred, or other promoters for expression in the host cell of interest. The promoter may be inducible or constitutive. It may be naturally-occurring, may be composed of portions of various naturally occurring promoters, or may be partially or totally synthetic. Guidance for the design of promoters is commonly known in the art. In addition, the location of the promoter relative to the transcription start may be optimized. Many suitable promoters for use in plants are well known in the art, as are nucleotide sequences, which enhance expression of an associated expressible sequence. An example of a DNA construct with a suitable promoter may include a nucleotide sequence in operable linkage with a stress-inducible promoter or an epidermis- and/or mesophyll-specific promoter.

Plant-specific promoters mean in principle any promoter which is capable of controlling the expression of genes, in particular foreign genes, in plants or plant parts, plant cells, plant tissues, plant cultures. Here, the expression can be for example constitutional, inducible or development-dependent.

In a preferred disclosure, the promoter is a constitutive promoter, preferably a stress inducible promoter, more preferably a water stress inducible promoter.

As used herein "constitutive" promoter means those promoters which ensure overexpression in numerous tissues over a relatively large period of plant development, at all times during plant development. In particular, a plant promoter or a promoter derived from a plant virus with the methods described herein including but not limited to the 35S transcript of the *CaMV* cauliflower mosaic virus (Franck et al. Cell 21, 285 (1980); Odell et al. Nature 313, 810 (1985); Shewmaker et al. Virology 140, 281 (1985); Gardner et al. Plant Mol Biol 6, 221 (1986)) or the 19S *CaMV* Promoter (U.S. Pat. No. 5,352,606; WO 84/02913; Benfey et al. EMBO J. 8, 2195-2202 (1989)). A further suitable constitutive promoter is the rubisco small subunit (SSU) promoter (U.S. Pat. No. 4,962,028), the promoter of *Agrobacterium nopaline* synthase, the TR double promoter, the *Agrobacterium* OCS (octopine synthase) promoter, the ubiquitin promoter (Holtorf S et al. Plant Mol Biol 29, 637 (1995)), the ubiquitin 1 promoter (Christensen et al. Plant Mol Biol 18, 675 (1992); Bruce et al. Proc Natl Acad Sci USA 86, 9692 (1989)), the Smas promoter, the cinnamyl-alcohol dehydrogenase promoter (U.S. Pat. No. 5,683,439), the promoters of vacuolar ATPase subunits or the promoter of a proline-rich protein from wheat (WO 91/13991), and further promoters of genes whose constitutive expression in plants is known to the skilled worker including the promoter of nitrilase-1 (nit1) gene from *A. thaliana* (GenBank Acc.-No.: Y07648.2, Nucleotide 2456-4340, Hillebrand et al. Gene 170, 197 (1996)).

Thus, in a preferred disclosure, the overexpression of the one or more FMO protein coding sequences in the methods of the disclosure is a constitutive overexpression. In another

Seed-specific promoters are, for example, the promoter of phaseolin (U.S. Pat. No. 5.504,200; Bustos et al. Plant Cell 1(9), 839 (1989)), of the 2S albumin gene (Joseffson et al. J Biol Chem 262, 12196 (1987)), of legumin (Shirsat et al. Mol Gen Genet 215(2), 326 (1989)), of the USP (unknown seed protein; Bäumlein et al. Mol Gen Genet 225(3), 459 (1991)), of the napin gene (U.S. Pat. No. 5,608,152; Stalberg et al. L Planta 199, 515 (1996)), of the gene coding for the sucrose binding protein (WO 00/26388) or the legumin B4 promoter (LeB4; Bäumlein et al. Mol Gen Genet 225, 121 (1991); Bäumlein et al. Plant Journal 2(2), 233 (1992); Fiedler et al. Biotechnology (NY) 13(10), 1090 (1995)), the oleosin promoter from *Arabidopsis* (WO 98/45461), the Bce4 promoter from *Brassica* (WO 91/13980). Further suitable seed-specific promoters are those of the genes coding for the high molecular weight glutenin (HMWG), gliadin, branching enzyme, ADP glucose pyrophosphatase (AGPase) or starch synthase. Further promoters may include those allowing seed-specific expression in monocotyledons such as maize, barley, wheat, rye, rice etc. It is possible and advantageous to employ the promoter of the Ipt2 or Ipt1 gene (WO 95/15389, WO 95/23230) or the promoters described in WO 99/16890 (promoters of the hordein gene, of the glutelin gene, of the oryzin gene, of the prolamin gene, of the gliadin gene, of the zein gene, of the kasirin gene or of the secalin gene).

Tuber-, storage root- or root-specific promoters are, for example, the patatin class I promoter (B33) or the promoter of the potato cathepsin D inhibitor.

Leaf-specific promoters are, for example, the promoter of the cytosolic FBPase from potato (WO 97/05900), the SSU promoter (small subunit) of the rubisco (ribulose-1.5-bisphosphate carboxylase) or the ST-LSI promoter from potato (Stockhaus et al. EMBO J. 8, 2445 (1989)). Epidermis-specific promoters are, for example the promoter of the OXLP gene ("oxalate oxidase like protein"; Wei et al. Plant Mol. Biol. 36, 101 (1998)) and a promoter consisting of the GSTA1 promoter and the WIRla intron (WO 2005/035766) and the GLP4 promoter (WO 2006/1288832 PCT/EP 2006/062747).

Examples of other tissue-specific promoters are: flower-specific promoters, for example the phytoene synthase promoter (WO 92/16635) or the promoter of the Prr gene (WO 98/22593) and anther-specific promoters, for example the 5126 promoter (U.S. Pat. No. 5,689,049, 5,689,051), the glob-I promoter and the [gamma]-zein promoter.

The expression cassettes may also comprise a chemically inducible promoter (review article: Gatz et al. Annu. Rev. Plant Physiol Plant Mol Biol 48, 89 (1997)) through which expression of the exogenous gene in the plant can be controlled at a particular point in time. Promoters of this type, such as, for example, the PRP1 promoter (Ward et al. Plant Mol Biol 22, 361 (1993)), a salicylic acid-inducible promoter (WO 95/19443), a benzenesulfonamide-inducible promoter (EP 0 388 186), a tetracycline-inducible promoter (Gatz et al. Plant J 2, 397 (1992)), an abscisic acid-inducible promoter (EP 0 335 528) and an ethanol- or cyclohexanone-inducible promoter (WO 93/21334) can likewise be used.

Pathogen-inducible promoters which make possible an expression only when required (i.e. in the case of attack by pathogens).In one disclosure, the method therefore uses promoters which are active in plants which are pathogen-inducible promoters. Pathogen-inducible promoters comprise the promoters of genes which are induced as a result of pathogen attack, such as, for example, genes of PR proteins, SAR proteins, [beta]-1.3-glucanase, chitinase etc. (for example Redolfi et al. Neth J Plant Pathol 89, 245 (1983); Uknes, et al. Plant Cell 4, 645 (1992); Van Loon Plant Mol Viral 4, 111 (1985); Marineau et al. Plant Mol Bid 9, 335 (1987); Matton et al. Molecular Plant-Microbe Interactions 2, 325 (1987); Somssich et al. Proc Natl Acad Sci USA 83, 2427 (1986); Somssich et al. Mol Gen Genetics 2, 93 (1988); Chen et al. Plant J 10, 955 (1996); Zhang and Sing Proc Natl Acad Sci USA 91, 2507 (1994); Warner, et al. Plant J 3, 191 (1993); Siebertz et al. Plant Cell 1, 961 (1989)).

An additional promoter for the overexpression of the FMO proteins as described herein may include wounding-inducible promoters such as that of the pinII gene (Ryan Ann Rev Phytopath 28, 425 (1990); Duan et al. Nat Biotech 14, 494 (1996)), of the wun1 and wun2 gene (U.S. Pat. No. 5,428,148), of the win1 and win2 gene (Stanford et al. Mol Gen Genet 215, 200 (1989)), of the systemin gene (McGurl et al. Science 225, 1570 (1992)), of the WIP1 gene (Rohmeier et al. Plant Mol Biol 22, 783 (1993); Eckelkamp et al. FEBS Letters 323, 73 (1993)), of the MPI gene (Corderok et al. Plant J 6(2), 141 (1994)) and the like.

A source of further pathogen-inducible promoters may include the pathogenesis-related (PR) gene family. A series of elements in these promoters have proved advantageous. Thus, the nucleotide region of nucleotide -364 to nucleotide -288 in the promoter of PR-2d mediates salicylate specificity (Buchel et al. Plant Mol Biol 30, 493 (1996)). In tobacco, this region binds a nuclear protein whose abundance is increased by salicylate. The PR-1 promoters from tobacco and *Arabidopsis* (EP-A 0 332 104, WO 98/03536) are also suitable as pathogen-inducible promoters. Also useful, since particularly specifically induced by pathogens, are the "acidic PR-5"-(aPR5) promoters from barley (Schweizer et al. Plant Physiol 114, 79 (1997)) and wheat (Rebmann et al. Plant Mol Biol 16, 329 (1991)). A PR5 proteins accumulate within approximately 4 to 6 hours after attack by pathogens and only show very little background expression (WO 99/66057). One approach for obtaining an increased pathogen-induced specificity is the generation of synthetic promoters from combinations of known pathogen-responsive elements (Rushton et al. Plant Cell 14, 749 (2002); WO 00/01830; WO 99/66057). Other pathogen-inducible promoters from different species are known to the skilled worker (EP-A 1 165 794; EP-A 1 062 356; EP-A 1 041 148; EP-A 1 032 684).

Further pathogen-inducible promoters comprise the Flachs Fis1 promoter (WO 96/34949), the Vst1 promoter (Schubert et al. Plant Mol Biol 34, 417 (1997)) and the tobacco EAS4 sesquiterpene cyclase promoter (U.S. Pat. No. 6,100,451).

Other promoters are those which are induced by biotic or abiotic stress, such as, for example, the pathogen-inducible promoter of the PRP1 gene (or gst1 promoter), for example from potato (WO 96128561; Ward et al. Plant Mol Biol 22, 361 (1993)), the heat-inducible hsp70 or hsp80 promoter from tomato (U.S. Pat. No. 5,187,267), the chill-inducible alpha-amylase promoter from potato (WO 96/12814) and the light-inducible PPDK promoter or the wounding-inducible pinII promoter (EP-A 0 375 091).

In one disclosure, the methods described herein employ mesophyll-tissue-specific promoters such as, for example, the promoter of the wheat germin 9f-3.8 gene (GenBank Acc.-No.: M63224) or the barley GerA promoter (WO 02/057412). The promoters are particularly advantageous since they are both mesophyll-tissue-specific and pathogen-inducible. Also suitable is the mesophyll-tissue-specific *Arabidopsis* CAB-2 promoter (GenBank Acc.-No.: X15222), and the *Zea mays* PPCZm1 promoter (GenBank Acc.-No.: X63869) or homologs thereof. Mesophyll-tissue-specific means that the transcription of a gene is limited to as few as possible plant tissues which comprise the mesophyll tissue as the result of the specific interaction of cis elements present in the promoter sequence and transcription factors binding to these elements; preferably, it means a transcription which is limited to the mesophyll tissue.

Additional mesophyll-specific promoters include PPCZm1 (=PEPC; Kausch, Plant Mol. Biol. 45, 1 (2001)); OsrbcS (Kyozuka et al., Plant Phys. 102, 991- (1993)); OsPPDK, acc. AC099041; TaGF-2.8, acc. M63223 (Schweizer, Plant J. 20, 541 (1999)); TaFBPase, acc. X53957;TaWIS1, acc. AF467542 (US 20021115849); HvBIS1, acc. AF467539 (US 2002/115849); ZmMIS1, acc. AF467514 (US 2002/115849); HvPR1a, acc. X74939 (Bryngelsson et al., Molecular Plant-Microbe Interactions 7 (2), 267 (1994); HvPR1b, acc. X74940 (Bryngelsson et al., Molecular Plant-Microbe Interactions 7 (2), 267 (1994)); HvB1.3gluc; acc. AF479647; HvPrx8, acc. AJ276227 (Kristensen et al., Molecular Plant Pathology 2 (6), 311(2001)); and HvPAL, acc. X97313 (Wei, Plant Molecular Biology 36, 101 (1998)).

Examples of epidermis-specific promoters are, for example, WIR5 (=GstA1), acc. X56012 (Dudler & Schweizer, unpublished); GLP4, acc. AJ310534 (Wei, Plant Molecular Biology 36, 101 (1998)); GLP2a, acc. AJ237942 (Schweizer, Plant J. 20, 541 (1999).); Prx7, acc. AJ003141 (Kristensen, Molecular Plant Pathology 2 (6), 311(2001)); GerA, acc. AF250933 (Wu, Plant Phys. Biochem. 38 or 685 (2000)); OsROC1, acc. AP004656; RTBV, acc. AAV62708, AAV62707 (Klöti, PMB 40, 249 (1999)) and Cer3 (Hannoufa, Plant J. 10 (3), 459 (1996)).

Examples of additional promoters suitable for the expression of FMO proteins include fruit ripening-specific promoters such as, for example, the fruit ripening-specific promoter from tomato (WO 94/21794, EP 409 625). Development-dependent promoters include some of the tissue-specific promoters because the development of individual tissues naturally takes place in a development-dependent manner.

Constitutive, and leaf- and/or stem-specific, pathogen-inducible, root-specific, mesophyll-tissue-specific promoters may be used with constitutive, pathogen-inducible, mesophyll-tissue-specific and root-specific promoters.

A further possibility for further promoters which make expression possible in further plant tissues or in other organisms such as, for example, *E. coli* bacteria to be operably linked to the nucleic acid sequence to be expressed or overexpressed. All the promoters described above are in principle suitable as plant or photosynthetic organism promoters.

Other promoters which are suitable for expression in plants are described (Rogers et al. Meth in Enzymol 153, 253 (1987); Schardl et al. Gene 61, 1 (1987); Berger et al. Proc Natl Acad Sci USA 86, 8402 (1989)).

Moreover, the average person skilled in the art is capable of isolating further suitable promoters by means of routine methods. Thus, the person skilled in the art can identify for example further epidermis-specific regulatory nucleic acid elements, with the aid of customary methods of molecular biology, for example with hybridization experiments or with DNA-protein binding studies. Here, a first step involves, for example, the isolation of the desired tissue from the desired organism from which the regulatory sequences are to be isolated, wherefrom the total poly(A)+RNA is isolated and a cDNA library is established. In a second step, those clones from the first library whose corresponding poly(A)+RNA molecules only accumulate in the desired tissue are identified by means of hybridization with the aid of cDNA clones which are based on poly(A)+RNA molecules from another tissue. Then, promoters with tissue-specific regulatory elements are isolated with the aid of these cDNAs thus identified. Moreover, a person skilled in the art has available further PCR-based methods for the isolation of suitable tissue-specific promoters.

The nucleic acid sequences present in the expression cassettes or vectors described herein may be operably linked to further genetic control sequences besides a promoter. The term genetic control sequences has a wide meaning and means all sequences which have an influence on the coming into existence or the function of the recombinant nucleic acid molecule of the disclosure. For example, genetic control sequences modify transcription and translation in prokaryotic or eukaryotic organisms. The expression cassettes may further comprise a promoter with an abovementioned specificity 5'-upstream from the particular nucleic acid sequence which is to be expressed transgenically, and a terminator sequence as additional genetic control sequence 3'-downstream, and if appropriate further conventional regulatory elements, in each case operably linked to the nucleic acid sequence to be expressed transgenically.

Genetic control sequences also comprise further promoters, promoter elements or minimal promoters capable of modifying the expression-controlling properties. It is thus possible for example through genetic control sequences for tissue-specific expression to take place additionally dependent on particular stress factors. Corresponding elements are described, for example, for water stress, abscisic acid (Lam E and Chua N H, J Biol Chem 266(26): 17131(1991)) and heat stress (Schoffl. F et al., Molecular & General Genetics 217(2-3): 246, 1989).

It is possible in principle for all natural promoters with their regulatory sequences like those mentioned above to be used for the method of the disclosure. It is additionally possible also for synthetic promoters to be used advantageously.

Genetic control sequences further comprise also the 5'-untranslated regions (5'-UTR), introns or noncoding 3' region of genes such as, for example, the actin-1 intron, or the Adh1-S introns 1, 2 and 6 (generally: The Maize Handbook, Chapter 116, Freeling and Walbot, Eds., Springer, New York (1994)). It has been shown that these may play a significant function in the regulation of gene expression. It has thus been shown that 5'-untranslated sequences are capable of enhancing transient expression of heterologous genes. An example of a translation enhancer which may be mentioned is the 5' leader sequence from the tobacco mosaic virus (Gallie et al. Nucl Acids Res 15, 8693 (1987)) and the like. They may in addition promote tissue specificity (Rouster J et al. Plant J 15, 435 (1998)). For example, is the natural 5'-UTR of the AtFMO GS-OX5 or ZmFMO gene, however the use of the promoter of the methods described herein induces the expression levels higher, for example drought stress induces an increase of three fold in the expression level, in particular that with the sequence of SEQ ID NO: 1, 25, or a sequence with at least 50%, 60%, 70%, 80%, 90%, 95%, 97% or in particular 99% or more identity thereto. The recombinant nucleic acid molecule may advantageously comprise one or more so-called enhancer sequences in operable linkage with the promoters, which make increased transgenic expression of the nucleic acid sequence possible. Additional advantageous sequences such as further regulatory elements or terminators can also be inserted at the 3' end of the nucleic acid sequences to be expressed recombinantly. The nucleic acid sequences to be expressed recombinantly may be present in one or more copies in the gene construct.

Polyadenylation signals suitable as control sequences are plant polyadenylation signals may include those which correspond essentially to T-DNA polyadenylation signals from *Agrobacterium tumefaciens,* in particular to gene 3 of the T-DNA (octopine synthase) of the Ti plasmid pTiACHS (Gielen et al. EMBO J 3:835(1984)) or functional equivalents thereof. Examples of particularly suitable terminator sequences are the OCS (octopine synthase) terminator and the NOS (nopaline synthase) terminator.

Control sequences mean those sequences which make homologous recombination or insertion into the genome of a host organism possible or allow deletion from the genome. In homologous recombination, for example, the natural promoter of a particular gene can be specifically replaced by a promoter with specificity for the embryonal epidermis and/or the flower.

A recombinant nucleic acid molecule and a vector derived from the molecule may comprise further functional elements. The term functional element has a wide meaning and means all elements which have an influence on the production, replication or function of the nucleic acid molecules, the vectors or the transgenic organisms of the disclosure. Non-restrictive examples which may be mentioned are selection markers which confer a resistance to a metabolism inhibitor such as 2-deoxyglucose 6-phosphate (WO 98/45456), antibiotics or biocides, herbicides, for example kanamycin, G 418, bleomycin, hygromycin or phosphinotricin. Examples which may be mentioned are: DNA sequences which code for phosphinothricin acetyltransferases (PAT), which inactivate glutamine synthase inhibitors (bar and pat gene), 5-enolpyruvylshikimate-3-phosphate synthase (EPSP synthase genes) which confer resistance to Glyphosat(R) (N-(phosphonomethyl)glycine), the gox gene, which codes for the Glyphosat(R)-degrading enzyme (glyphosate oxidoreductase), the deh gene (coding for a dehalogenase which inactivates dalapon), and bxn genes which code for bromoxynil-degrading nitrilase enzymes, the aasa gene, which confers a resistance to the antibiotic spectinomycin, the streptomycin phosphotransferase (SPT) gene, which makes possible a resistance to streptomycin, the neomycin phosphotransferase (NPTII) gene, which confers a resistance to kanamycin or geneticidin, the hygromycin phosphotransferase (HPT) gene, which mediates a resistance to hygromycin, the acetolactate synthase gene (ALS), which mediates a resistance to sulfonylurea herbicides (for example mutated ALS variants with, for example, the S4 and/or Hra mutation), and the acetolactate synthase gene (ALS), which mediates a resistance to imidazolinone herbicides.

Reporter genes or selectable markers are genes which code for easily quantifiable proteins and ensure via an intrinsic color or enzymic activity an assessment of the transformation efficiency or of the location or timing of expression including but not limited to reporter proteins (Schenborn E. and Groskreutz D. Mol Biotechnol.; 13(1):29 (1999) such as the green fluorescence protein (GFP) (Sheen et al. Plant Journal 8(5):777 (1995); Haselhoff et alProc Natl Acad Sci USA 94(6):2122 (1997); Reichel et al. Proc Natl Acad Sci USA 93(12):5888 (1996); Tian et al. Plant Cell Rep 16:267 (1997); WO 97/41228; Chui et al. Curr Biol 6:325 (1996); Leffel et al. Biotechniques. 23(5):912-8 (1997)), the chloramphenicoltransferase, a luciferase (Ow et al. Science 234:856 (1986); Millar et al. Plant Mol Biol Rep 10:324 (1992)), the aequorin gene (Prasher et al. Biochem Biophys Res Commun 126(3):1259 (1985)), the [beta]-galactosidase, R-locus gene (codes for a protein which regulates the production of anthocyanin pigments (red coloration) in plant tissue and thus makes possible the direct analysis of the promoter activity without the addition of additional adjuvants or chromogenic substrates; Dellaporta et al., In: Chromosome Structure and Function: Impact of New Concepts, 18th Stadler Genetics Symposium, 11:263, (1988), with [beta]-glucuronidase (Jefferson et al., EMBO J., 6, 3901, 1987).

Origins of replication (ORI) which ensure replication of the expression cassettes or vectors may include for example *E. coli.* Examples which may be mentioned are ORI (origin of DNA replication), the pBR322 ori or the P15A ori (Sambrook et al.: 1989).

Elements which are necessary for agrobacterium-mediated plant transformation, such as, for example, the right or left border of the T-DNA or the vir region.

To select successfully transformed cells, it is generally required to introduce a selection or selectable marker which confers to the successfully transformed cells a resistance to a biocide (for example a herbicide), a metabolism inhibitor such as 2-deoxyglucose 6-phosphate (WO 98/45456) or an antibiotic. The selection marker permits the selection of the transformed cells from untransformed cells (McCormick et al. Plant Cell Reports 5:81 (1969)).

It is also disclosed a plant produced by the method of the disclosure. As the skill person understands, said plant comprises the FMO protein,

It is also disclosed a tissue culture of cells produced from the plant of claim 45, wherein said cells of the tissue culture are produced from a plant part chosen from leaves, pollen, embryos, cotyledons, hypocotyl, meristematic cells, roots, root tips, pistils, anthers, flowers, and stems. As the skill person understands, said leaves, pollen embryos, cotyledons, hypocotyl, meristematic cells, roots, root tips, pistils, anthers, flowers, and stems comprise the FMO protein.

In a preferred disclosure, said plant is a monocotyledonous or dicotyledonous plant.

An additional disclosure relates to plants which, as the result of natural processes or artificial induction, comprise one or more mutations in a nucleic acid molecule which comprises the nucleic acid sequence as shown in SEQ ID NOs: 1, 3, 5, 7, 9, 11, 13, 15, 17, 19, 21, 23, 25, 27, 29, 31, 33, 35, 37, 39, 41 or 44, where the mutation brings about an increase of the activity, function or polypeptide quantity of one of the polypeptide encoded by the nucleic acid molecules as shown in SEQ ID NOs: 1, 3, 5, 7, 9, 11, 13, 15, 17, 19, 21, 23, 25, 27, 29, 31, 33, 35, 37, 39, 41 or 44. For example a mutation generated, and identified, by TILLING.

As a consequence, another disclosure may include a plant comprising a nucleic acid sequence which comprises a mutation which brings about, in the plants or parts thereof, an increase of the activity of one of the proteins encoded by the nucleic acid molecules of the disclosure. For example, the mutation concerns one or more amino acid residues which are identified in the consensus sequence in the figures as being conserved or highly conserved.

Consequently, a disclosure described herein provides a transgenic plant, transgenic plant part, or transgenic plant cell overexpressing an exogenous FMO, including the FMO protein overexpressed in the plant, plant part or plant cell is encoded by (i)an exogenous nucleic acid having at least 50% identity with SEQ ID NOs: 1, 3, 5, 7, 9, 11, 13, 15, 17, 19, 21, 23, 25, 27, 29, 31, 33, 35, 37 or 40, or a splice variant thereof; or by (ii)an exogenous amino acid encoding a protein having at least 50% identity with SEQ ID NOs: 2, 4, 6, 8, 10, 12, 14, 16, 18, 20, 22, 24, 26, 28, 30, 32, 34, 36, 38, 40 or 42, the encoded protein confers enhanced drought stress tolerance relative to control plants; (iii) an exogenous nucleic acid capable of hybridizing under stringent conditions with a complementary sequence of any of the nucleic acids according to (i) or (ii); encoding a FMO protein; wherein the amino acid molecule codes for a polypeptide which has essentially identical properties to the polypeptide described in SEQ ID NO: 2, 4, 6, 8, 10, 12, 14, 16, 18, 20, 22, 24, 26, 28, 30, 32, 34, 36, 38, 40 or 42; the encoded protein confers enhanced drought stress tolerance relative to control plants; and / or by (iv) an exogenous nucleic acid encoding the same FMO protein as any of the nucleic acids of (i) to (iii) above, but differing from the nucleic acids of (i) to (iii) above due to the degeneracy of the genetic code.

Also provided herein are transgenic plants transformed with at least a) a nucleic acid sequence which comprises the nucleic acid molecules as shown in SEQ ID NOs: 1, 3, 5, 7, 9, 11, 13, 15, 17, 19, 21, 23, 25, 27, 29, 31, 33, 35, 37, 39, 41 or 44; the nucleic acid sequences which are complementary thereto, or the amino acid molecules which code for the polypeptides as shown in SEQ ID NOs: 2, 4, 6, 8, 10, 12, 14, 16, 18, 20, 22, 24, 26, 28, 30, 32, 34, 36, 38, 40, 42 or 43; b) a transgenic expression cassette which comprises one of the nucleic acid sequences , or a vector, and cells, cell cultures, tissue, parts-such as for example leaves, roots and the like or propagation material in the case of plant organisms-derived from such organisms. Of note, this disclosuremay also include a plant other than *Arabidopsis thaliana.*

Host organisms or starting organisms, herein "transgenic organisms" are plants as defined above. In one disclosure, the transgenic organism is a mature plant, seed, shoot and seedling, and parts, propagation material and cultures derived therefrom, for example cell cultures. As used herein "mature plant" means plants at any developmental stage beyond the seedling stage. "Seedling" means a young immature plant in an early developmental stage. Plants which are particularly preferred as host organisms are plants to which the method for obtaining a drought stress tolerance in accordance with the abovementioned criteria can be applied. In one disclosure, the plant is a dicotyledonous plant as discussed above. In another disclosure, the plant is a monocotyledonous plant as discussed above. The transgenic organisms can be generated with the above-described methods for the transformation or transfection of organisms.

Further disclosuresdescribed herein include the use of the transgenic organisms and of the cells, cell cultures, parts-such as, for example, roots, leaves and the like in the case of transgenic plant organisms, and transgenic propagation material such as seeds or fruits for the preparation of foodstuffs or feeding stuffs, pharmaceuticals or fine chemicals. Stack varieties are also included in which a plurality of advantageous characters such as the classic herbicide characters mentioned above or herbicide tolerance genes, harmful insect resistance genes, antipathogenic substance producing genes, characters improved in oil stuff ingredients or characters having reinforced amino acid content are combined.

Parts of the transgenic plant are also provided herein and comprise the FMO nucleic acid or FMO protein. The may be seeds, roots, leaves and/or flowers comprising the FMO nucleic acid or FMO protein or parts thereof. Preferred parts of soy plants are soy beans comprising the FMO nucleic acid or FMO protein. Products derived from transgenic plants as described herein, parts thereof or harvestable parts thereof are also provided, including meal or oil, such as soybean meal or soybean oil, comprising the FMO nucleic acid or FMO protein. One disclosureis the method for the production of a product, wherein the product is meal or oil, preferably, soybean meal or soybean oil.

In one disclosuredescribed herein, the method for the production of a product comprise: a) growing the plants described herein or obtainable by the methods of described herein and b) producing the product from or by the plants of the disclosure and/or parts, e.g. seeds, of these plants.

In another disclosurethe products produced by the methods described herein are plant products such as, but not limited to, a foodstuff, feedstuff, a food supplement, feed supplement, fiber, cosmetic and/or pharmaceutical. Foodstuffs are regarded as compositions used for nutrition and/or for supplementing nutrition. Animal feedstuffs and animal feed supplements, in particular, are regarded as foodstuffs.

In a further disclosurethe method comprises the steps a) growing the plants of the disclosure, b) removing the harvestable parts as defined above from the plants and c) producing the product from or by the parts of the transgenic plant or organism.

The product may be produced at the site where the plant has been grown, the plants and/or parts thereof may be removed from the site where the plants have been grown to produce the product. Typically, the plant is grown, the desired harvestable parts are removed from the plant, if feasible in repeated cycles, and the product made from the harvestable parts of the plant. The step of growing the plant may be performed only once each time the methods disclosed herein is performed, while allowing repeated times the steps of product production e.g. by repeated removal of harvestable parts of the plants of the disclosure and if necessary further processing of these parts to arrive at the product. It is also possible that the step of growing the plants of the disclosure is repeated and plants or harvestable parts are stored until the production of the product is then performed once for the accumulated plants or plant parts. Also, the steps of growing the plants and producing the product may be performed with an overlap in time, even simultaneously to a large extend or sequentially. Generally the plants are grown for some time before the product is produced.

In another disclosurethe methods for the production are used to make agricultural products such as, but not limited to, plant extracts, proteins, amino acids, carbohydrates, fats, oils, polymers, vitamins, and the like. Please note that it is possible that a plant product consists of one or more agricultural products to a large extent.

The transgenic plants produced as described herein may be crossed with similar transgenic plants or with transgenic plants lacking the nucleic acids of the disclosure or with non-transgenic plants, using known methods of plant breeding, to prepare seeds. Further, the transgenic plant cells or plants described herein may comprise, and/or be crossed to another transgenic plant that comprises one or more exogenous nucleic acids, thus creating a "stack" of transgenes in the plant and/or its progeny. The seed is then planted to obtain a crossed fertile transgenic plant comprising the FMO nucleic acid. The crossed fertile transgenic plant may have the particular expression cassette inherited through a female parent or through a male parent. The second plant may be an inbred plant. The crossed fertile transgenic may be a hybrid. Also disclosed herein are seeds of any of these crossed fertile transgenic plants. The seeds disclosed herein can be harvested from fertile transgenic plants and be used to grow progeny generations of transformed plants of this disclosure including hybrid plant lines comprising the exogenous nucleic acid.

Therefore another disclosuremay include a method for breeding a drought stress tolerant plant comprising the steps of: (a) crossing a transgenic plant described herein or a plant obtainable by a method described herein with a second plant; (b)obtaining a seed or seeds resulting from the crossing step described in (a); (c) planting the seed or seeds and growing the seed or seeds to plants; and (d) selecting from the plants the plants expressing a FMO protein, encoded by (i) an exogenous nucleic acid having at least 60% identity with SEQ ID NOs: 1, 3, 5, 7, 9, 11, 13, 15, 17, 19, 21, 23, 25, 27, 29, 31, 33, 35, 37, 39, 41 or 44, or a splice variant thereof; (ii) an exogenous nucleic acid encoding a protein having at least 60% identity with SEQ ID NOs: 2, 4, 6, 8, 10, 12, 14, 16, 18, 20, 22, 24, 26, 28, 30, 32, 34, 36, 38, 40, 42 or 43; the encoded protein confers enhanced water tolerance relative to control plants; (iii) an exogenous nucleic acid capable of hybridizing under stringent conditions with a complementary sequence of any of the nucleic acids according to (i) or (ii); encoding a FMO protein; wherein the nucleic acid molecule codes for a polypeptide which has essentially identical properties to the polypeptide described in SEQ ID NOs: 2, 4, 6, 8, 10, 12, 14, 16, 18, 20, 22, 24, 26, 28, 30, 32, 34, 36, 38, 40, 42 or 43; preferably the encoded protein confers enhanced water stress tolerance relative to control plants; and / or by (iv) an exogenous nucleic acid encoding the same FMO protein as any of the nucleic acids of (i) to (iii) above, but differing from the nucleic acids of (i) to (iii) above due to the degeneracy of the genetic code.

Another disclosureprovided herein is a method for plant improvement comprising (a) obtaining a transgenic plant by any of the methods of the present disclosure; (b) combining within one plant cell the genetic material of at least one plant cell of the plant of (a) with the genetic material of at least one cell differing in one or more gene from the plant cells of the plants of (a) or crossing the transgenic plant of (a) with a second plant; (c) obtaining seed from at least one plant generated from the one plant cell of (b) or the plant of the cross of step (b); (d) planting the seeds and growing the seeds to plants; and (e) selecting from the plants, plants expressing the nucleic acid encoding the FMO GS-OX5 protein; and optionally (f) producing propagation material from the plants expressing the nucleic acid encoding the FMO GS-OX5 protein. The transgenic plants may be selected by known methods as described above (e.g., by screening for the presence of one or more markers which are encoded by plant-expressible genes co-transferred with the FMO gene or screening for the FMO nucleic acid itself), the expression of a structural gene can, of course, also be effected, or influenced, independently of the methods described herein or the use of the subject matter described herein.

The practice described herein employs, unless otherwise indicated, conventional techniques of chemistry, molecular biology, microbiology, recombinant DNA, genetics, immunology, cell biology, cell culture and transgenic biology, which are within the skill of the art. (See, e.g., Maniatis, et al., Molecular Cloning, Cold Spring Harbor Laboratory Press, Cold Spring Harbor, NY (1982); Sambrook, et al., (1989); Sambrook and Russell, Molecular Cloning, 3rd Ed., Cold Spring Harbor Laboratory Press, Cold Spring Harbor, NY (2001); Ausubel, et al., Current Protocols in Molecular Biology, John Wiley & Sons (including periodic updates) (1992); Glover, DNA Cloning, IRL Press, Oxford (1985); Russell, Molecular biology of plants: a laboratory course manual, Cold Spring Harbor Laboratory Press, Cold Spring Harbor, NY (1984); Anand, Techniques for the Analysis of Complex Genomes, Academic Press, NY (1992); Guthrie and Fink, Guide to Yeast Genetics and Molecular Biology, Academic Press, NY (1991); Harlow and Lane, Antibodies, Cold Spring Harbor Laboratory Press, Cold Spring Harbor, NY (1988); Nucleic Acid Hybridization, B.D. Hames & S.J. Higgins eds. (1984); Transcription And Translation, B.D. Hames & S.J. Higgins eds. (1984); Culture Of Animal Cells, R.I. Freshney, A.R. Liss, Inc. (1987); Immobilized Cells And Enzymes, IRL Press (1986); B. Perbal, A Practical Guide To Molecular Cloning (1984); the treatise, Methods In Enzymology, Academic Press, Inc., NY); Methods In Enzymology, Vols. 154 and 155, Wu, et al., eds.; Immunochemical Methods In Cell And Molecular Biology, Mayer and Walker, eds., Academic Press, London (1987); Handbook Of Experimental Immunology, Volumes I-IV, D.M. Weir and C.C. Blackwell, eds. (1986); Riott, Essential Immunology, 6th Edition, Blackwell Scientific Publications, Oxford (1988); Fire, et al., RNA Interference Technology: From Basic Science to Drug Development, Cambridge University Press, Cambridge (2005); Schepers, RNA Interference in Practice, Wiley VCH (2005); Engelke, RNA Interference (RNAi): The Nuts & Bolts of siRNA Technology, DNA Press (2003); Gott, RNA Interference, Editing, and Modification: Methods and Protocols (Methods in Molecular Biology), Human Press, Totowa, NJ (2004); and Sohail, Gene Silencing by RNA Interference: Technology and Application, CRC (2004)).

All the terms and embodiments previously described are equally applicable to this aspect of the invention.

The invention will be described by way of the following examples which are to be considered as merely illustrative and not limitative of the scope of the invention

### EXAMPLES

The following examples are provided to illustrate further the various applications and are not intended to limit the invention beyond the limitations set forth in the appended claims.

### General Methods

### Biological material and growth conditions

For the FMO protein overexpression, transgenic *Arabidopsis* plants over expressing the FMO GS-OX5 gene (SEQ ID NO: 1 or SEQ ID NO:2) and described as RCI5-OE (ES 2347399B1) (FMOX3 and FMOX8 genotypes shown in Figure 3) and wild type (Col-0) seeds of *Arabidopsis thaliana* were obtained using the following method.

RCI5 cDNA was ligated into the Smal site, downstream of the CaMv35S promoter in the pROK2 vector (Baulcombe et al., 1986) (shown in the construct of Figure 4a), to obtain the X3 and X8. Once the presence of the construct (such as the construct described in Figure 4a and Figure 4b) was verified in the recombinant plasmid by DNA sequencing, were introduced into the *Agrobacterium tumefaciens* strain C58C1 (Deblaere et al., 1985). Transformation of *Arabidopsis* Col was performed following the floral dip method (Clough and Bent, 1998). The plants were sown in plastic pots containing the same amount of water saturated substrate. Trays containing 16 pots with 5 plants per pot were placed in a grow chamber under short-day light conditions until the plants developed 12 leaves. Then, the trays were transferred to the greenhouse under long-day light conditions and the pots were individually placed in transparent plastic glasses in order to avoid water spillage during irrigations. Normal irrigated plants for each genotype were also placed on the trays, as controls. A total of 4 trays were used, with differently distributed genotypes within each tray. No phenotypic differences were observed among genotypes.

In order to determine the plant biomass analysis, *Arabidopsis* plants were grown for three (3) weeks under short day (10 hours light, 14 hours dark, 21°C light and 20°C at night, 65% humidity) conditions. Fresh weight from individual rosettes was obtained, Col-0 (n=10) and RCI5-OE (ES 2347399B1) (FMOX3 and FMOX8 genotypes) two weeks after sowing (n=10). Seeds yield of fully grown plants that were grown for 3 weeks under short day conditions and then transferred for 3 additional weeks to long day conditions was recorded. Seeds were harvested 4 weeks later from individual plants (n=10).

### Nuclear Magnetic Resonance spectroscopy (NMR)

TMAO content in plants was determined by harvesting three leaves per treatment and freezing them in liquid nitrogen before the NMR determination. At least three independent plants were treated per experiment.

### Biological material and growth conditions for greenhouse

For each wilting or limited water experiment 480 seeds (of either pepper, barley, tomato, cucumber or corn) were sown, producing 384 plants in 512 cm³ pots (4 plants per pot). Plants were grown under chamber conditions at 21°C for 3 weeks. Then, the plants were moved to a greenhouse, where average temperature was 25°C to 28°C. Treatments as described herein were done when the plants had two extended leaves and the next pair of leaves were coming up.

Treatments: Twelve (12) pots (containing 48 plants) were irrigated with 40 ml of either: water, 0.1g/L TMAO di-hydrate solution, 1.0g/L TMAO di-hydrate solution, or 5.5g/L TMAO di-hydrate solution. Another set of 12 pots containing 48 plants were sprayed with 40 ml of either water (3.3ml in average per pot), a solution containing 0.1g/L TMAO di-hydrate solution, 1.0g/L TMAO di-hydrate, or 5.5g/L TMAO di-hydrate. All pots were also watered with 40ml of water. The sprayed plants were watered with the same volume of water as the "irrigated plants). The pots were located on plastic glass to maintain constant moisture and to avoid liquid spillage during watering. Trays containing the pots were located on greenhouse tables. The distribution of the trays on the table and the position on the pots in the tray was changed every week to avoid position effects.

After the treatments described above, the plants were not watered until the pots completely lost their moisture, taking about 4 to 8 days depending on the season, at which point the plants were extremely wilted for the extreme drought experiments. The plants were then watered once with solutions containing the different amounts of TMAO di-hydrate described above (0.1g/L, 1.0g/L or 5.5g/L) or just water, after which the plants were left to lose their moisture completely again for three consecutive cycles of watering after wilting. For the "limited water" experiments they were watered with 20 ml of water or solution instead of 40 ml when the first plants started to wilt. The plant survival rate was recorded and analyzed for the "extreme drought" experiments in which plants were allowed to wilt severely before watering, while the stem length was recorded as analyzed for the limited water experiments in which the plants are watered with 30% of the water that the plant requires.

### Strawberries, leek, lettuce, broccoli, celery or kohlrabi

In order to determine the plant yield productivity under normal conditions, 'Sabrina', 'Candonga' and 'Fortuna' variety strawberry, leek, lettuce, "Iceberg" variety, broccoli "Parthenon" variety, celery or kohlrabi plants, were grown under standard production conditions and 120 plants of each variety per treatment (where the treatment was a Control comprising standard watering or 1g/L of TMAO spray every four weeks) were analyzed. Plants were located in four (4) different positions for each group of 30 plants from the same treatment. Fruits, leaves or roots were harvested from individual plants and total weight was determined for each plant.

### Tomatoes

In order to determine the drought or water stress tolerance after seed treatments with TMAO di-hydrate and germination in the presence of TMAO di-hydrate, tomato 'Moneymaker' seeds were surface sterilized for 3 minutes in ethanol 70%, then rinsed twice and finally included in a pre-treatment solution of 0.1g/L TMAO di-hydrate solution (or just water) under shaking for 3 hours. Then, they were rinsed and included in the germination plates under sterile conditions. Polyethylene glycol (PEG-6000) was added to the germination medium (Murashige and Skoog salts medium) at 152 and 182 g/L. The increasing amount of PEG reduces ψ (hydric potential) values, simulating drought conditions for germination. Each germination plate had at least 30 seeds, and each treatment/pretreatment plate was replicated five times (150 seeds per treatment/pretreatment). Seeds were left to germinate for 10 days under dark conditions in a culture chamber (21°C light and 20°C night, 65% humidity). Then, germinated seeds were recorded by visual inspection and data analysis was performed using Statgraphics software.

### Corn, Barley and sunflower field trials

In order to determine the drought or water stress tolerance after seed treatments with TMAO di-hydrate and germination in the presence of TMAO di-hydrate, barley "Hispanic" seeds or com "FAO700" seeds, or "Sambro" sunflower seeds were surface sterilized for 3 minutes in ethanol 70%, then rinsed twice and finally included in a pre-treatment solution of 1g/L TMAO di-hydrate solution (or just water) under shaking for 3 hours at a dose of 1litre per Kg of seeds. Then, the seeds were sown in randomized plots of 10 sqm in a surface of 2.000 sqm. Chlorophyll content was measured 1 month before harvest. In corn irrigation was applied in half of the plots while the order half only received an initial establishment watering. The barley plots received 2001 of rain per m² through the growing season. Some of the plots received a second spray treatment with 1g/liter of TMAO.

### Nuclear Magnetic Resonance spectroscopy (NMR)

TMAO content in plants was determined by harvesting three leaves per treatment and freezing them in liquid nitrogen before the NMR determination. At least three independent plants were treated per experiment.

Example 1: TMAO accumulates in pepper and barley after 1 week drought treatment. 'Murano' pepper and 'Bomi' barley seeds were sown and grown as described above. Control plants (six weeks old) were irrigated with 40 ml of water twice in the week, while "drought" treated plants were not irrigated. Leaves were harvested and TMAO was determined by NMR as described. As shown in Table 1, TMAO levels increase almost three fold compared to the control in both pepper and barley after drought treatment.

**Table 1. TMAO accumulation after 1 week drought**

| Crop | TMAO (µM) | SD | % Control |
|---|---|---|---|
| Pepper Control. | 446.68 | 215.86 | 100 |
| Pepper Drought 7days | 1224.23 | 243.10 | 274 |
| Barley Control | 422.10 | 43.36 | 100 |
| Barley Drought 7days | 1252.73 | 251.99 | 297 |

As shown in Table 1, in row 1, the control pepper shows 446.68 µM of TMAO, while in row 2 it is shown that 7 days of drought treatment increases TMAO levels in pepper 2.74 fold to 1224.23 µM. Analogously in row 3 control barley shows 422.10 µM of TMAO while in row 4 it is shown that 7 days of drought treatment increases TMAO levels in barley 2.97 fold to 1252.73 µM.

Example 2: TMAO accumulates in pepper and barley when applied exogenously. 'Murano' pepper seeds and' Bomi' barley seeds were sown and grown as described above. Control plants (six weeks old) were sprayed with water and pepper treated plants were sprayed with 1g/l of TMAO while barley plants were sprayed with 1g/l of TMAO formulated with 0.1% of C8-C10 Alkylpolysaccharide. Leaves were harvested and TMAO was determined by NMR. The percentage of TMAO increase compared to untreated controls was determined for each time point. TMAO levels increase in pepper and barley with exogenous treatment of TMAO at 1g/l to higher levels than drought treatment and furthermore, the TMAO levels are high up to 40 days post spray in pepper.

**Table 2. TMAO accumulation after TMAO di-hydrate spray treatments**

| Crop | % Control |
|---|---|
| Pepper 1 day post spray | 529 |
| Pepper 10 days post spray | 373 |
| Pepper 20 days post spray | 286 |
| Pepper 30 days post spray | 135 |
| Pepper 40 days post spray | 213 |
| Barley 1 day post spray | 822 |

As shown in Table 2, in row 1, pepper sprayed with TMAO increases its TMAO level 5.29 fold 1 day post spray when compared with control sprayed with water. The level decreases after 10 days to 3.73 fold of control (row 2), after 20 days to 2.86 fold of control (row 3), after 30 days to 1.35 fold of unsprayed control (row 4), staying above the water sprayed control even 40 days after spray (row 5). Analogously in row 6, barley sprayed with TMAO and an alkylpolysaccharide, increases its TMAO level 8.22 fold 1 day post spray when compared with control sprayed with just the alkypolysaccharide due to better penetration of the TMAO formulated with the alkylpolysaccharide.

Example 3: Exogenous application of TMAO di-hydrate does not have trade-offs in strawberry. Fruit yield was determined in 'Sabrina', 'Candonga' and 'Fortuna' strawberry plants treated with 1g/l of TMAO di-hydrate or water as described above in order to evaluate the trade-off costs of the treatment with no water stress. However, no significant difference was observed in the fruit production which was always slightly higher in the TMAO di-hydrate treated plants.

**Table 3. Strawberry fruit production after TMAO di-hydrate spray treatments every 4 weeks for 3 months**

| Strawberry Variety | % Control 2013 |
|---|---|
| Sabrina | 106 |
| Candonga | 102 |
| Fortuna | 101 |
| Total | 105 |

Table 3 shows that TMAO can be applied exogenously for three months without a fitness cost. In row 1 the total production weight of Sabrina variety plants treated with TMAO di-hydrate produced 106% when compared with water treated controls, in row 2 the total production weight of Candonga variety plants treated with TMAO di-hydrate produce 102% when compared with controls, in row 3 the total production weight of Fortuna variety plants treated with TMAO di-hydrate produce 101% when compared with controls, while in row 4 the total production weight of the three variety plants treated with TMAO di-hydrate produce 105% when compared with water treated controls of the three varieties.

Example 4: TMAO di-hydrate applied exogenously increases germination both in pre-treatment of the seeds and as an additive to the medium. Tomato 'Moneymaker' seeds were sown, grown and treated as described above.

**Table 4. Tomato seed germination rates ± S.E. and ANOVA P-values for two independent experiments where TMAO di-hydrate effect was evaluated on seed germination under drought conditions generated by adding Polyethylene glycol (PEG-6000) to the germination medium.**

| PEG CONCENTR ATION | ψ VALUE | NUMBER OF SEEDS | TREATMENT | GERMINA TION RATE | ANOVA *P*-value |
|---|---|---|---|---|---|
| 152g/L | -0.2564 | 150 (5X30) | No treatment | 25.80 ± 4.62% | - |
| | | 150 (5X30) | Pre-treatment with TMAO di-hydrate 0.1g/L solution (3 hours) | 70.62 ± 4.62% | 0.0000* |
| | | 150 (5X30) | TMAO di-hydrate 0.1 g/L in the germination medium | 71.09 ± 4.26% | 0.0000* |
| 182g/L | -0.3676 | 150 (5X30) | No treatment | 15.37 ± 3.20 % | - |
| | | 150 (5X30) | Pre-treatment with TMAO di-hydrate 0.1g/L solution (3 hours) | 18.53 ± 3.20% | 0.2310 |
| | | 150 (5X30) | TMAO di-hydrate 0.1 g/L in the germination medium | 45.00 ± 3.20% | 0.0086* |

| | | | | | |
|---|---|---|---|---|---|
| Asterisks (*) indicate statistical significant differences between control and treated seeds (α=0,05) | | | | | |

This example shows that TMAO can be applied exogenously in seed treatments to improve germination under drought conditions before the water stress occurs. Seeds are germinated in the presence of PEG to induce hydric stress. Two concentrations are used in the first 3 rows 152g/L (which corresponds to a ψ value of -0.2564) and a higher dose 182g/L (which corresponds to a ψ value of -0.3676) to show that at increasing values of water stress germination decreases. In the first and in the fourth rows no treatment is applied to the seeds which are germinated directly in the presence of PEG. It is shown that indeed water stress affects germination which is only of 25.80% for 152g/L PEG (row 1) and 15.37% for 182g/L PEG (row 4). Pre-treatment of the seeds for 3 hours with TMAO di-hydrate 0.1g/L solution significantly increases the germination rates to 70.62% for 152g/L PEG (row 2), and 18.53% for 182g/L PEG (row 5) when compared to untreated controls. Furthermore if TMAO di-hydrate 0.1g/L is added to the germination medium the germination rates significantly increase even higher to 71.09% for 152g/L PEG (row 3), and specially to 45.00% for the higher water stress condition of 182g/L PEG (row 6) when compared to untreated controls.

Example 5: TMAO di-hydrate applied exogenously increases plant survival in pepper under extreme drought conditions. 'Murano' pepper seeds were sown, grown and treated as described above. 10g/L and 1g/L TMAO di-hydrate sprayed was the best treatment when irrigation was done with water, with 83.3% of plant survival while 100% plant survival rate was observed when plants were sprayed with 0.1g/L or 1g/L and watered with 5g/L.

**Table 5. Average survival rate and ANOVA analysis for TMAO di-hydrate treated pepper plants under drought growing conditions.**

| IRRIGATION | *N* | INITIAL SPRAY TREATMENT | SURVIVAL RATE (%) | ANOVA *P*-value |
|---|---|---|---|---|
| ALL REGIMES | 384 | WATER | 42.7 ± 3.6 | 0.0000 |
| | | 0.1g/L TMAO | 51.0 ± 3.6 | |
| | | 1g/L TMAO | 62.5 ± 3.6 | |
| | | 10g/L TMAO | 71.8 ± 3.6 | |
| WATER | 96 | WATER | 45.8 ± 8.1 | 0.0025 |
| | | 0.1g/L TMAO | 37.5 ± 8.1 | |
| | | 1g/L TMAO | 62.5 ± 8.1 | |
| | | 10g/L TMAO | 79.1 ± 8.1 | |
| 0.1g/L TMAO | 96 | WATER | 29.1 ± 8.3 | 0.0000 |
| | | 0.1g/L TMAO | 33.3 ± 8.3 | |
| | | 1g/L TMAO | 54.1 ± 8.3 | |
| | | 10g/L TMAO | 83.3 ± 8.3 | |
| 1g/L TMAO | 96 | WATER | 0,0 ± 7.7 | 0.0028 |
| | | 0.1g/L TMAO | 33.3 ± 7.7 | |
| | | 1g/L TMAO | 33.3 ± 7.7 | |
| | | 10g/L TMAO | 37.5 ± 7.7 | |
| 5g/L TMAO | 96 | WATER | 95.8 ± 3.8 | 0.0812 |
| | | 0.1g/L TMAO | 100 ± 3.8 | |
| | | 1g/L TMAO | 100 ± 3.8 | |
| | | 10g/L TMAO | 87.5 ± 3.8 | |

Table 5 shows that TMAO can be applied exogenously by spray and/or irrigation before water stress occurs increasing the plant survival rate under extreme water stress conditions in a vegetable crop species. In rows 1-4 the spray treatments are compared combined independently from the irrigation treatments. The survival rate after drought significantly increases with the concentration of the TMAO spray being the lowest in row 1 without TMAO (42.7%) and the highest in row 4 with 10g/L of TMAO (71.8%). In rows 5-8 the spray treatments are compared when the plants are irrigated only with water. Analogously survival rate after drought significantly increases with the concentration of the TMAO spray being the lowest in row 5 without TMAO (45.8%) and the highest in row 8 with 10g/L of TMAO (79.1%). In rows 9-12 the spray treatments are compared when the plants are irrigated with 0.1g/L of TMAO. Survival rate after drought significantly increases with the concentration of the TMAO spray being the lowest in row 9 without TMAO (29.1%) and the highest in row 12 with 10g/L of TMAO (83.3%). In rows 13-16 the spray treatments are compared when the plants are irrigated with 1g/L of TMAO. Survival rate after drought also significantly increases with the concentration of the TMAO spray being the lowest in row 13 without TMAO (0%) and the highest in row 16 with 10g/L of TMAO (37.5%). The best results are achieved when plants are irrigated with TMAO at 5g/L (rows 17-20). Even without spray treatment the survival rate is 95.8% (row 17), which increases up to 100% survival with 0.1g/L and 1g/L spray treatments (rows 18-19). Combining the highest doses of spray 10g/L and irrigation 5g/L lowers the survival rate to 87.5% (row 20) due to a TMAO overdose.

Example 6: TMAO di-hydrate applied exogenously increases plant survival in tomato under extreme drought conditions. Moneymaker tomato seeds were sown, grown and treated as described. No statistical differences between modes of application (sprayed or TMAO di-hydrate watered) were observed on this experiment. 5g/L TMAO di-hydrate sprayed was the best treatment when irrigation was done with water, with 74.2% of plant survival. At higher test rates, both treatments showed a clear increase of shoot dry mass when compared with untreated plants. Additionally, TMAO treated plants behaved extremely healthy compared to untreated control, though plants withstand drought much better after drought treatment (Figure 1). Additionally, as shown in Figure 1, TMAO treated plants behaved extremely healthy compared to untreated control, though plants withstand drought much better after drought treatment. In Figure 1, on the left-hand side control plants irrigated with water and on the right-hand side treated plants irrigated with 5.5g/L TMAO di-hydrate after drought recovery.

**Table 6. Average survival rate and ANOVA analysis for TMAO di-hydrate treated tomato plants under drought conditions.**

| IRRIGATION | *N* | INITIAL SPRAY TREATMENT | SURVIVAL RATE (%) | ANOV A *P-*value |
|---|---|---|---|---|
| ALL REGIMES | 384 | WATER | 12.5 ± 4.1 | 0.0000 |
| | | 0.1g/L TMAO | 12.5 ± 4.1 | |
| | | 1g/L TMAO | 37.5 ± 4.1 | |
| | | 5g/L TMAO | 56.6 ± 4.1 | |
| WATER | 96 | WATER | 16.6 ± 9.1 | 0.0000 |
| | | 0.1g/L TMAO | 29.1 ± 9.1 | |
| | | 1g/L TMAO | 62.5 ± 9.1 | |
| | | 5g/L TMAO | 74.2 ± 9.1 | |
| 0.1g/L TMAO | 96 | WATER | 16.6 ± 8.5 | 0.0000 |
| | | 0.1g/L TMAO | 12.5 ± 8.5 | |
| | | 1g/L TMAO | 41.6 ± 8.5 | |
| | | 5g/L TMAO | 68.9 ± 8.5 | |
| 1g/L TMAO | 96 | WATER | 4.1 ± 7.5 | 0.0013 |
| | | 0.1g/L TMAO | 0.0 ± 7.5 | |
| | | 1g/L TMAO | 29.1 ± 7.5 | |
| | | 5g/L TMAO | 33.3 ± 7.5 | |
| 5g/L TMAO | 96 | WATER | 8.3 ± 8.0 | 0.0015 |
| | | 0.1g/L TMAO | 12.5 ± 8.0 | |
| | | 1g/L TMAO | 16.6 ± 8.0 | |
| | | 5g/L TMAO | 50.0 ± 8.0 | |

Table 6 shows that TMAO can be applied exogenously by spray and/or watering before the water stress occurs increasing the plant survival rate in the *Solanaceae* family, under extreme water stress conditions. In rows 1-4 the spray treatments are compared combined independently from the irrigation treatments. The survival rate after drought significantly increases with the concentration of the TMAO spray being the lowest in row 1 without TMAO (12.5%) and the highest in row 4 with 5g/L of TMAO (56.6%). In rows 5-8 the spray treatments are compared when the plants are irrigated only with water. Analogously survival rate after drought significantly increases with the concentration of the TMAO spray being the lowest in row 5 without TMAO 16.6%) and the highest in row 8 with 5g/L of TMAO (74.2%). In rows 9-12 the spray treatments are compared when the plants are irrigated with 0.1g/L of TMAO. Survival rate after drought significantly increases with the highest concentrations of the TMAO spray being the lowest in rows 9 and 10, without TMAO (16.6%) and 0.1g/L TMAO spray (12.5%) respectively, and the highest in row 12 with 5g/L of TMAO (68.9%). In rows 13-16 the spray treatments are compared when the plants are irrigated with 1g/L of TMAO. Survival rate after drought also significantly increases with the highest concentrations of the TMAO spray being the lowest in rows 13 and 14, without TMAO (4.1%) and 0.1g/L TMAO spray (0%) respectively, and the highest in row 16 with 5g/L of TMAO (33.3%). Increasing the TMAO irrigation treatment to 5g/L (rows 17-20) improves the survival rates when compared to low dose irrigation treatments combined with spray treatments, but in tomato is not as good as the spray treatments alone, probably due to a TMAO overdose, although increasing concentrations of TMAO spray still increase the overall survival rate. Combining the highest doses of spray 5g/L and irrigation 5g/L renders a survival rate of 50% (row 20).

Example 7: TMAO di-hydrate applied exogenously increases plant survival in cucumber under extreme drought conditions. 'Marketer' cucumber seeds were sown, grown and treated as described. Watered applications seem to produce better performance on survival rate (P value 0,05). 5g/L TMAO sprayed was the best treatment when irrigation was done with 5g/L TMAO, with 95.8% of plant survival.

**Table 7. Average survival rate and ANOVA analysis for TMAO treated cucumber plants under drought growing conditions.**

| IRRIGATION | *N* | INITIAL SPRAY TREATMENT | SURVIVAL RATE (%) | ANOVA *P*-value |
|---|---|---|---|---|
| ALL REGIMES | 384 | WATER | 66.6 ± 3.4 | 0.0000 |
| | | 0.1g/L TMAO | 80.1 ± 3.4 | |
| | | 1g/L TMAO | 92.7 ± 3.4 | |
| | | 5g/L TMAO | 94.7 ± 3.4 | |
| WATER | 96 | WATER | 54.1 ± 7.2 | 0.0004 |
| | | 0.1g/L TMAO | 83.3 ± 7.2 | |
| | | 1g/L TMAO | 91.6 ± 7.2 | |
| | | 5g/L TMAO | 95.8 ± 7.2 | |
| 0.1g/L TMAO | 96 | WATER | 45.8 ± 7.4 | 0.0000 |
| | | 0.1g/L TMAO | 82.9 ± 7.4 | |
| | | 1g/L TMAO | 91.6 ± 7.4 | |
| | | 5g/L TMAO | 95.8 ± 7.4 | |
| 1g/L TMAO | 96 | WATER | 87.5 ± 5.9 | 0.0028 |
| 5g/L TMAO | 96 | 0.1g/L TMAO | 91.6 ± 5.9 | 0.0812 |
| | | 1g/L TMAO | 91.6 ± 5.9 | |
| | | 5g/L TMAO | 91.6 ± 5.9 | |
| | | WATER | 66.6 ± 7.2 | |
| | | 0.1g/L TMAO | 75.0 ± 7.2 | |
| | | 1g/L TMAO | 95.8 ± 7.2 | |
| | | 5g/L TMAO | 95.8 ± 7.2 | |

Table 7 shows that TMAO can be applied exogenously by spray and/or watering before the water stress occurs increasing the plant survival rate in the *Cucurbitaceae* family, under extreme water stress conditions. In rows 1-4 the spray treatments are compared combined independently from the irrigation treatments. The survival rate after drought significantly increases with the concentration of the TMAO spray being the lowest in row 1 without TMAO (66.6%) and the highest in row 4 with 5g/L of TMAO (94.7%). In rows 5-8 the spray treatments are compared when the plants are irrigated only with water. Analogously survival rate after drought significantly increases with the concentration of the TMAO spray being the lowest in row 5 without TMAO (54.1%) and the highest in row 8 with 5g/L of TMAO (95.8 %). In rows 9-12 the spray treatments are compared when the plants are irrigated with 0.1g/L of TMAO. Survival rate after drought significantly increases with the concentration of the TMAO spray being the lowest in row 9 without TMAO (45.8%) and the highest in row 12 with 5g/L of TMAO (95.8%). In rows 13-16 the spray treatments are compared when the plants are irrigated with 1g/L of TMAO. Survival rate after drought also significantly increases with the any of the TMAO spray treatments being the lowest in row 13 without TMAO (87.5%) and higher in rows 14-16 with 0.1, 1 or 5g/L of TMAO giving the same 91.6% survival rate. The best results are achieved when plants are irrigated with TMAO at 5g/L (rows 17-20). Even without spray treatment the survival rate is 66.6% (row 17), which increases up to 95.8% survival with 5g /L spray treatment (rows 20).

Example 8: TMAO di-hydrate applied exogenously increases plant survival in tomato under limited water irrigation. 'Moneymaker' tomato seeds were sown, grown and treated as described. Both spray and irrigation treatments with TMAO increased significantly plant size.

**Table 8. Average stem size and ANOVA analysis for TMAO and water irrigated tomato plants under limited water growing conditions.**

| INITIAL TREATMENT | *N* | IRRIGATIONS | AVERAGE STEM SIZE (cm) | ANOVA *P*-value |
|---|---|---|---|---|
| WATER | 94 | WATER | 10.57 ± 0.56 | 0.0000 |
| | | 1g/L TMAO | 12.97 ± 0.55 | |
| 0.1g/L TMAO | 93 | WATER | 11.06 ± 0.55 | 0.1034 |
| | | 1g/L TMAO | 12.32 ± 0.56 | |
| 1g/L TMAO | 96 | WATER | 11.59 ± 0.55 | 0.0000 |
| | | 1g/L TMAO | 13.77 ± 0.55 | |
| 5g/L TMAO | 92 | WATER | 14.2 ± 0.56 | 0.7230 |
| | | 1g/L TMAO | 14.6 ± 0.55 | |

Table 8 shows that TMAO can be applied exogenously by spray and watering before the water stress occurs increasing the shoot biomass in the *Solanaceae* family, under limited water stress conditions. In rows 1-2 the irrigation treatments are compared combined independently from the spray treatments. The shoot length significantly increases after limited irrigation with 1g/L TMAO spray being the lowest in row 1 without TMAO (10.57 cm) and the highest in row 2 with 1g/L of TMAO spray (12.97 cm). In rows 1, 3, 5 and 7 the spray treatments are compared when the plants are irrigated only with water. Shoot length after limited water irrigation significantly increases with the concentration of the TMAO spray being the lowest in row 1 without TMAO (10.57 cm) and the highest in row 7 with 5g/L of TMAO (14.2 cm). In rows 2, 4, 6 and 8 the spray treatments are compared when the plants are irrigated with 1g/L of TMAO. Again shoot length significantly increases after limited water irrigation with the increasing concentrations of the TMAO spray being the lowest in row 2, without TMAO spray (12.97 cm) and the highest in row 8 when both treatments are combined with 5g/L of TMAO spray treatment and 1g/L irrigation treatment (14.6 cm).

Example 9: TMAO di-hydrate applied exogenously increases plant production in tomato under limited water irrigation. 'Rio Grande' tomato seeds were sown, grown and treated as described. Spray treatments with 1g/L TMAO increased plant production.

**Table 9. Average fruit production and ANOVA analysis for TMAO spray treated tomato plants under limited water growing conditions.**

| IRRIGATION | *N* | INITIAL TREATMENT | AVERAGE WEIGTH (grams/fruit) | ANOVA *P*-value |
|---|---|---|---|---|
| 100% WATER | 36 | WATER | 73.85 ± 17.84 | - |
| 30% WATER | 36 | WATER | 52.9 ± 17.28 | 0.4243 |
| 30% WATER | 36 | 1g/L TMAO | 76.73 ± 17.67 | 0.3406 |

Table 9 shows that TMAO can be applied exogenously by spray before the water stress occurs increasing the production in the *Solanaceae* family, under limited water stress conditions. In row 2 it is shown that 30% water irrigation significantly lowers plant production (52.9 g/fruit) when compared with plants in row 1 under normal water irrigation (73.85 g/fruit). However, as shown in row 3, spray treatment with 1g/L of TMAO di-hydrate applied exogenously every 4 weeks restores plant production with an increase of fruit production of 45% even under limited water irrigation (76.73 g/fruit) over the untreated plants with a 30% irrigation.

Example 10: TMAO di-hydrate applied exogenously increases plant survival and biomass in barley under limited water irrigation. 'Bomi' barley seeds were sown, grown and treated as described.

**Table 10. Average dry weight ± S.E. and ANOVA analysis for TMAO di-hydrate and water irrigated barley plants under drought growing conditions.**

| INITIAL TREATMENT | *N* | IRRIGATIONS | AVERAGE DRY WEIGHT (mg) | ANOVA *P-*value |
|---|---|---|---|---|
| CONTROL | 10 | WATER | 1017.7 ± 66.13 | - |
| 1g/L SPRAYED TMAO di-hydrate SOLUTION | 12 | WATER | 1205.4 ± 60.37 | 0,0212* |
| 1g/L WATERED TMAO di-hydrate SOLUTION | 10 | WATER | 1371.4 ± 66.13 | 0,0073* |
| - | 70 | CONTROL | 1109.3 ± 33.93 | - |
| - | 68 | 1g/L TMAO di-hydrate SOLUTION | 1216.1 ± 33.44 | 0,0265* |

Table 10 shows that TMAO can be applied exogenously by spray and watering before the water stress occurs increasing the plant survival rate and shoot biomass in monocotyledonous plants, under extreme water stress conditions. In the first three rows the initial treatments are compared, both 1g/L TMAO di-hydrate spray (row 2) and 1g/L TMAO di-hydrate irrigation treatments (row 3) significantly increase the mean dry biomass per plant, under extreme drought conditions, to 1205.4 mg and 1371.4 respectively when compared with water treated control plants in row 1 (1017.7 mg). Furthermore, analogous results can be obtained when plants are only irrigated with 1g/L TMAO di-hydrate (row 5: 1216.1 mg per plant) when compared with the same amount of limited irrigation with water without TMAO in row 4 (1109.3 mg).

Example 11: TMAO di-hydrate applied exogenously increases plant production in corn under limited water irrigation. 'FAO700" corn seeds were sown, grown and treated as described. Spray treatments with 1g/L TMAO increased plant number of green leaves, total chlorophyll content and grain production.

**Table 11. Average number of green leaves and ANOVA analysis for TMAO spray or seed treated corn plants under limited water growing conditions.**

| IRRIGATION REGIME | *N* | TREATMENT | AVERAGE NUMBER OF GREEN LEAVES | *P* VALUE |
|---|---|---|---|---|
| 100% WATER | 30 | - | 11.03 ± 0.33 | - |
| 30% WATER | 23 | - | 5.78 ± 0.38 | - |
| 30% WATER | 53 | 1g/L TMAO SPRAY | 8.50 ± 0.25 | 0.0000 * |
| 30% WATER | 20 | 1g/L TMAO SEED | 8.50 ± 0.41 | 0.0001 * |

Table 11 shows that TMAO can be applied exogenously by spray before the water stress occurs, or by seed incubation, increasing the biomass production in the monocotyledonous plants, under limited water stress conditions. In row 2 it is shown that 30% water irrigation significantly lowers the number of green leaves when compared with plants in row 1 under normal water irrigation. However, as shown in rows 3 and 4, spray treatment with 1g/L of TMAO di-hydrate when applied exogenously every 4 weeks significantly restores the number of green leaves under limited water irrigation with a 47% increase in biomass production, shown in green leaf production over the untreated plants with a 30% irrigation.

Example 12: TMAO di-hydrate applied exogenously increases plant production in corn under limited water irrigation. 'FAO700" corn seeds were sown, grown and treated as described above. As shown in Table 12, spray treatments with 1g/L TMAO increased plant total chlorophyll content.

**Table 12. Average chlorophyll content and ANOVA analysis for TMAO spray or seed treated corn plants under limited water growing conditions.**

| IRRIGATION REGIME | | TREATMENT | TOTAL CHLOROPHYL CONTENT | *P* VALUE |
|---|---|---|---|---|
| 100% WATER | 0 | - | 0.9163 ± 0.052 | - |
| 30% WATER | 3 | - | 0.5194 ± 0.107 | - |
| 30% WATER | 3 | 1g/L TMAO SPRAY | 0.7278 ± 0.076 | 0.1214 |
| 30% WATER | 0 | 1g/L TMAO SEED | 0.8977 ± 0.195 | 0.1854 |

Table 12 shows that TMAO can be applied exogenously by spray before the water stress occurs, or by seed incubation, increasing the biomass production in the monocotyledonous plants, under limited water stress conditions. In row 2 it is shown that 30% water irrigation significantly lowers total chlorophyll content when compared with plants in row 1 under normal water irrigation. However, as shown in rows 3 and 4, spray treatment with 1g/L of TMAO di-hydrate when applied exogenously every 4 weeks significantly restores the chlorophyll content under limited water irrigation with an increase in biomass production between 40% and 72%, shown in chlorophyll content over the untreated plants with a 30% irrigation.

Example 13: TMAO di-hydrate applied exogenously increases plant production in corn under limited water irrigation. 'FAO700" corn seeds were sown, grown and treated as described. Spray treatments with 1g/L TMAO increased plant grain production.

**Table 13. Average number of grains per cob and ANOVA analysis for TMAO spray or seed treated corn plants under limited water growing conditions.**

| IRRIGATION REGIME | *N* | TREATMENT | AVERAGE NUMBER OF GRAINS PER COB | *P* VALUE |
|---|---|---|---|---|
| 100% WATER | 30 | - | 533.95 ± 22.48 | - |
| 30% WATER | 23 | - | 429.13 ± 45.31 | - |
| 30% WATER | 53 | 1g/L TMAO SPRAY | 511.34 ± 19.70 | 0.0495 * |
| 30% WATER | 20 | 1g/L TMAO SEED | 542.89 ± 41.22 | 0.0757 |

Table 13 shows that TMAO can be applied exogenously by spray before the water stress occurs, or by seed incubation, increasing the biomass production in the monocotyledonous plants, under limited water stress conditions. In row 2 it is shown that 30% water irrigation significantly lowers total number of grains per corn cob when compared with plants in row 1 under normal water irrigation. However, as shown in rows 3 and 4, spray treatment with 1g/L of TMAO di-hydrate when applied exogenously every 4 weeks significantly restores the total number of grains per corn cob under limited water irrigation with an increase in biomass production between 19% and 27%, shown in chlorophyll content over the untreated plants with a 30% irrigation. Of note, row 4 actually shows a 2% increase in the total number of grains per corn cob for corn plants under 30% water irrigation with a spray treatment of 1g/L of TMAO di-hydrate when compared to corn plants with no water stress or 100% irrigation.

Example 14: Exogenous application of TMAO di-hydrate does not have trade-offs in leek, lettuce, broccoli, celery or kohlrabi. Root or leaves yield was determined in the plants treated with 1g/l of TMAO di-hydrate or water as described above in order to evaluate the trade-off costs of the treatment with no water stress. However, no significant difference was observed in the yield production which was in most cases slightly higher in the TMAO di-hydrate treated plants.

**Table 14. Yield production after TMAO di-hydrate spray treatments every 4 weeks for 3 months**

| Crop | % Control |
|---|---|
| Leek | 102 |
| Lettuce | 112 |
| Broccoli | 120 |
| Celery | 100 |
| Kohlrabi | 103 |

Table 14 shows that TMAO can be applied exogenously for three months without a fitness cost. In row 1 the total production weight of leek plants treated with TMAO di-hydrate produced 102% when compared with water treated controls, in row 2 the total production weight of lettuce plants treated with TMAO di-hydrate produced 112% when compared with controls, in row 3 the total production weight of broccoli plants treated with TMAO di-hydrate produce 120% when compared with controls, while in row 4 the total production weight of the celery plants treated with TMAO di-hydrate produce the same as water treated controls, and finally in row 5 kohlrabi plants produced 103% when compared with water treated controls.

Example 15: TMAO di-hydrate applied exogenously increases plant production in broccoli under limited water irrigation. 'Parthenon' broccoli seeds were sown, grown and treated as described. Spray and irrigation treatments with 1g/L TMAO increased plant production.

**Table 15. Average inflorescence production and ANOVA analysis for TMAO spray treated broccoli plants under limited water growing conditions.**

| IRRIGATION | *N* | TREATMENT | AVERAGE WEIGTH (grams/fruit) | ANOVA *P-*value | % Control |
|---|---|---|---|---|---|
| 100% WATER | 36 | WATER | 202.8 ± 17.5 | - | 250 |
| 30% WATER | 36 | WATER | 80.5 ± 8.9 | 0.4243 | - |
| 30% WATER | 36 | 1g/L TMAO spray | 87.3 ± 6.7 | 0.3406 | 108 |
| 30% WATER | 36 | 1g/L TMAO irrigation | 85.2 ± 4.6 | 0.3406 | 106 |

Table 15 shows that TMAO can be applied exogenously by spray before the water stress occur increasing the production in the *Brassicaceae* family, under limited water stress conditions. In row 2 it is shown that 30% water irrigation significantly lowers plant production (80.5 g/plant) when compared with plants in row 1 under normal water irrigation (202.8 g/plant). However, as shown in rows 3 and 4, spray or irrigation treatment with 1g/L of TMAO di-hydrate applied exogenously every 4 weeks partially restores plant production with an increase of inflorescence production of 8 % or 6% respectively even under limited water irrigation (87.3 g/plant and 85.2 g/plant) over the untreated plants with a 30% irrigation.

Example 16: TMAO di-hydrate applied exogenously increases plant production in barley grown in the field without irrigation. 'Hispanic" barley seeds were sown, grown and treated as described. Both, seed (1g/l/Kg TMAO) and a combination of seed and spray treatments with 1g/L TMAO increased plant grain production.

**Table 16. Average seed production in grams per square meter and ANOVA analysis for TMAO seed or seed and spray treated barley plants grown in the field without external irrigation and with 200 1/m² of rain water in total through the season.**

| | TREATMENT | AVERAGE NUMBER OF GRAMS PER SQUARE METER | *P* VALUE | % Control |
|---|---|---|---|---|
| 8 | - | 190.63 ± 26.24 | - | - |
| 8 | 1g TMAO/1 Kg SEED | 225.98 ± 11.89 | 0.04615 * | 18 |
| 8 | 1g TMAO/1 Kg SEED+1g/L TMAO spray | 256.36 ± 12.78 | 0.0438 * | 35 |

Table 16 shows that TMAO can be applied exogenously by spray before the water stress occurs, or by seed incubation, increasing the seed production in cereal plants grown in the open field without additional irrigation. In row 2 it is shown that seed treatment with 1gTMAO per 1 Kg seeds significantly increases up to 18% the yield when compared with plants in row 1 without treatment. Furthermore, as shown in row 4, an additional spray treatment with 1g/L of TMAO di-hydrate applied exogenously every 4 weeks significantly increases the total yield per square meter up to 35% when compared with the untreated control.

Example 17: TMAO di-hydrate applied exogenously increases plant production in sunflower grown in the field without external irrigation. 'Sambra" sunflower seeds were sown, grown and treated as described. Seed treatment (1g/l/Kg TMAO) increased plant chlorophyll content and seed production.

**Table 16. Effects of seed treatment with TMAO on plant fitness in sunflower under natural stress conditions. The table shows the chlorophyll content, weight of seeds and P-values for the ANOVA test. Both chlorophyll and weight differences between control and TMAO groups are statistically significant. Relative chlorophyll content values are obtained by optical absorbance in two different wavebands: 653nm (chlorophyll) and 931nm (Near Infra-Red)**

| TRAIT | GROUP | *N* | AVERAGE VALUE | % GAIN/LOSS RESPECT TO THE CONTROL | ANOVA *P-*VALUE |
|---|---|---|---|---|---|
| CHLOROPHYLL CONTENT | CONTROL | 100 | 16,28 ± 0,42 | 30,0% | 0,0000 |
| | SEED TREATMENT | 100 | 21,17 ± 0,54 | | |
| WEIGHT OF SEEDS FROM 1 PLANT | CONTROL | 8 | 90,8 ± 9,0 | 77,7% | 0,0005 |
| | SEED TREATMENT | 8 | 161,3 ± 13,1 | | |

Table 17 shows that TMAO can be applied exogenously by seed treatment before the water stress occurs, increasing the seed production in and oil bearing crop plants such as sunflower grown in the open field without additional irrigation. In column 5 it is shown that seed treatment with 1gTMAO per 1 Kg seeds significantly increases up to 30% the chlorophyll content and the yield up to 77% when compared with control plants without treatment.

Example 18: DDAO applied exogenously increases plant survival in pepper under extreme drought conditions. 'Murano' pepper seeds were sown, grown and treated as described above. 0.5g/L DDAO applied as seed, irrigation or spray treatment improved survival rate when compared to untreated controls. 0.5g/L DDAO sprayed was the best treatment, with 83.3% of plant survival.

**Table 18. Average survival rate and ANOVA analysis for DDAO treated pepper plants under drought growing conditions.**

| *N* | TREATMENT | SURVIVAL RATE (%) | ANOVA *P-*value |
|---|---|---|---|
| 96 | WATER SEED | 22.9 ± 4.1 | 0.0000 |
| 96 | 0.5g/Kg DDAO SEED | 76.1 ± 4.1 | |
| 96 | WATER SPRAY | 22.9 ± 4.1 | |
| 96 | 0.5g/L DDAO SPRAY | 83.3 ±4.1 | |
| 96 | WATER IRRIGATION | 8.3 ± 4.1 | |
| 96 | 0.5g/L DDAO IRRIGATION | 35.4 ±4.1 | |

Table 18 shows that DDAO can be applied exogenously by seed and/or spray and/or irrigation before water stress occurs increasing the plant survival rate under extreme water stress conditions in a vegetable crop species. In rows 1-2 the seed treatments are compared and DDAO treatment increases survival from 22.9% to 76.1%. Survival rate after drought also significantly increases to 35.4% when the DDAO is applied in irrigation as shown in row6, while the lowest survival was in row 5 the irrigation control without DDAO (8.3%). The best results are achieved when plants are irrigated with DDAO at 0.5g/L (row 4).

### Comparative Example 19:

As shown in Table 19 and Table 20 below, over-expression of FMO GS-OX5 increasing endogenous production of TMAO di-hydrate does not have trade-offs in *Arabidopsis.* Plant biomass and seed yield was determined in transgenic (X3 and X8 genotypes) and wild type (Col-0) seeds of *Arabidopsis thaliana* were sown, grown and treated as described above in order to evaluate the trade-off costs of the increase of the TMAO endogenous production with no water stress. However, as shown in Tables 19 and 20, no significant difference was observed in the plant biomass or seed weight or yield. The vegetative mean weight increased with the number of copies of the FMO GS-OX5, being significantly larger when 8 copies of the gene are present compared to the 3 copies genotype. The seed mean weight increased with the number of copies of the FMO GS-OX5, being larger when 8 copies of the gene are present compared to the 3 copies genotype.

**Table 19. Plant Biomass was evaluated as average weight value (in grams) ± S.E. for three different groups of plants grown under no stress conditions: wild type (Col-0) and transgenic (X3 and X8) plants of Arabidopsis thaliana.**

| Genotype | N | BIOMASS MEAN WEIGHT VALUE ± S.E | ANOVA P-value |
|---|---|---|---|
| Col-0 | 10 | 2.0637 ± 0.2240 | |
| RCI5-OE.FMOX3 | 10 | 1.9199 ± 0.1383 | 0.5917 |
| RCI5-OE.FMOX8 | 10 | 2.5815 ± 0.1191 | 0.023* |

**Table 20. Plant seed weight or yield was evaluated as average weight value (in mg) ± S.E. for three different groups of seeds and siliques from Arabidopsis plants grown under no stress conditions: wild type (Col-0) and transgenic (38.3 and 38.8) plants of Arabidopsis thaliana.**

| Genotype | N | SEED MEAN WEIGHT VALUE ± S.E | ANOVA P-value |
|---|---|---|---|
| Col-0 | 10 | 522.8 ± 22.64 | |
| RCI5-OE.FMOX3 | 10 | 495.1 ± 37.22 | 0.5330 |
| RCI5-OE.FMOX 8 | 10 | 546.3 ± 35.09 | 0.5806 |

### Comparative Example 20:

As shown in Table 21 below, over-expression of FMO *GS-OX5* increases plant survival in *Arabidopsis* under limited water irrigation: Control plants (six weeks old) were irrigated with 40 ml of water twice in the week, while "limited water irrigation" treated plants were irrigated with 30 ml of water once a week. Transgenic (X3 and X8 genotypes) and wild type (Col-0) seeds of *Arabidopsis thaliana* were sown, grown and treated as described. The fitness value increased with the number of copies of the FMO GS-OX5, being larger when 8 copies of the gene are present compared to the 3 copies genotype. Fitness values were assigned using the following criteria: 0: Dead plant; 1: Critically damaged plant symptoms; 2: Moderate damaged plant symptoms; 3: Slightly damaged plant symptoms; 4: Healthy plant. As shown in Table 20, the transgenic plants had a significantly higher fitness value than the non-transgenic plants.

**Table 21. Average fitness value ± S.E. for three different genotypes grown under limited water irrigation: wild type (Col-0) and transgenic (X3 and X8) plants of Arabidopsis thaliana.**

| GENOTYPE | NUMBER OF PLANTS | FITNESS VALUE | ANOVA P-value |
|---|---|---|---|
| Col-0 | 60 | 1.75 ± 0.09 | - |
| RCI5-OE.FMOX3 | 60 | 2.533 ± 0.09 | 0.0000* |
| RCI5-OE.FMOX8 | 60 | 3.066 ± 0.09 | 0.0000* |

### Comparative Example 21

Over-expression of FMO GS-OX5 increases plant survival in *Arabidopsis* under drought conditions: Control plants (six weeks old) were irrigated with 40 ml of water twice in the week; while "drought" treated plants were not irrigated until all the plants were wilted. (TMAO dihydrate applied exogenously is able to recover plant survival in wild type drought stressed plants. Transgenic (FMOX3 and FMOX8 genotypes) and wild type (Col-0) seeds of *Arabidopsis thaliana* were sown, grown and treated as described. After the first cycle of wilting wild type plants were sprayed with 1g/L TMAO di-hydrate to determine if the wilted wild type plants could recover and perform as well as the transgenic plants in the following cycles of wilting with the exogenous application. Fitness values were assigned using the following criteria: 0: Dead plant; 1: Critically damaged plant symptoms; 2: Moderate damaged plant symptoms; 3: Slightly damaged plant symptoms; 4: Healthy plant. As shown in Table 21, the transgenic plants treated with TMAO had a significantly higher fitness value than the non-transgenic plants treated with TMAO.

**Table 22. Average fitness value ± S.E. for three different genotypes grown under drought conditions: wild type (Col-0) and transgenic (X3 and X8) plants of Arabidopsis thaliana.**

| GENOTYPE | NUMBER OF PLANTS | MEAN FITNESS VALUE ± S.E. | ANOVA P-value |
|---|---|---|---|
| Col-0 | 36 | 1.14 ± 0.17 | - |
| Col-0 + 1g/L SPRAYED TMAO di-hydrate SOLUTION | 36 | 1.83 ± 0.21 | 0.0129* |
| RCI5-OE.FMOX3 | 36 | 2.67 ± 0,08 | 0.0000* |
| RCI5-OE.FMOX8 | 36 | 2.64 ± 0,08 | 0.0000* |

While a number of exemplary aspects and embodiments have been discussed above, those of skill in the art will recognize certain modifications, permutations, additions and sub-combinations thereof. It is therefore intended that the following appended claims and claims hereafter introduced are interpreted to include all such modifications, permutations, additions, and sub-combinations.

The foregoing discussion of the disclosure has been presented for purposes of illustration and description. The foregoing is not intended to limit the disclosure to the form or forms disclosed herein. In the foregoing Detailed Description for example, various features of the disclosure are grouped together in one or more embodiments for the purpose of streamlining the disclosure. This method of disclosure is not to be interpreted as reflecting an intention that the claimed disclosure requires more features than are expressly recited in each claim. Rather, as the following claims reflect, inventive aspects lie in less than all features of a single foregoing disclosed embodiment. Thus, the following claims are hereby incorporated into this Detailed Description, with each claim standing on its own as a separate preferred embodiment of the disclosure.

As used herein "gene expression" and "expression" are to be understood as being synonymous and mean the realization of the information which is stored in a nucleic acid molecule. The terms "polypeptide" and "protein" are used herein interchangeably.

Various components are referred to herein as "operably linked", "linked" or "operably associated." As used herein, "operably linked", "operative linkage", "linked" or "operably associated" refers to nucleic acid sequences on a single nucleic acid fragment so that the function of one is affected by the other. For example, a promoter is operably linked with a coding sequence when it is capable of affecting the expression of that coding sequence.

As used herein, "at least one," "one or more," and "and/or" are open-ended expressions that are both conjunctive and disjunctive in operation. For example, each of the expressions "at least one of A, B and C," "at least one of A, B, or C," "one or more of A, B, and C," "one or more of A, B, or C" and "A, B, and/or C" means A alone, B alone, C alone, A and B together, A and C together, B and C together, or A, B and C together.

As used herein, "sometime" means at some indefinite or indeterminate point of time. So for example, as used herein, "sometime after" means following, whether immediately following or at some indefinite or indeterminate point of time following the prior act

The use of the terms "a," "an," and "the," and similar referents in the context of describing the disclosure (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. For example, if the range 10-15 is disclosed, then 11, 12, 13, and 14 are also disclosed. All methods described herein can be performed in any suitable order unless otherwise indicated herein or otherwise clearly contradicted by context. The use of any and all examples, or exemplary language (e.g., "such as") provided herein, is intended merely to better illuminate the disclosure and does not pose a limitation on the scope of the disclosure unless otherwise claimed. No language in the specification should be construed as indicating any non-claimed element as essential to the practice of the disclosure.

## Claims

1. A method for producing a drought tolerant plant or photosynthetic organism comprising:
applying at least one treatment of an effective amount of trimethylamine N-oxide (TMAO), Trimethylamine N-oxide di-hydrate or N,N-Dimethyldecylamine N-oxide (DDAO) to a plant, plant part, photosynthetic organism or seed, wherein the effective amount of the TMAO, TMAO di-hydrate or DDAO is from 0.1 to 10 g per liter for spray or irrigation, or wherein the at least one treatment is a seed treatment and the effective amount of the TMAO, TMAO di-hydrate or DDAO is from 0.1 g to 1000 g per 100 kg of seed; and
growing said plant, plant part, photosynthetic organism or seed, wherein a drought tolerant plant or photosynthetic organism is produced.

2. The method of claim 1, and further comprising:
applying at least one second treatment of an effective amount of TMAO, TMAO di-hydrate or DDAO to said drought tolerant plant or photosynthetic organism, previously treated with TMAO, TMAO di-hydrate or DDAO.

3. The method of claims 1 or 2, wherein said at least one treatment of said effective amount TMAO, TMAO di-hydrate or DDAO is an irrigation treatment or a spray treatment.

4. The method of any of claims 1 to 3, wherein said at least one treatment of said effective amount of TMAO, TMAO di-hydrate or DDAO comprises two or more different compounds selected from the group consisting of TMAO, TMAO di-hydrate and DDAO.

5. The method of any of claims 1 to 4, wherein said drought tolerant plant or photosynthetic organism has a biomass, fruit or seed production that is between 6% and 30% or between 31% and 50% more than the biomass, fruit or seed production of drought stressed plants or photosynthetic organisms where an effective amount of TMAO, TMAO di-hydrate or DDAO has not been applied to the non-tolerant water stressed plant or photosynthetic organism.

6. The method of any of claims 1 to 5, further comprising applying salts or any other additive to said plant, plant part, photosynthetic organism or seed and growing said plant, plant part, photosynthetic organism or seed wherein a drought tolerant plant or photosynthetic organism is produced.

## Patentansprüche

1. Ein Verfahren zur Herstellung einer dürretoleranten Pflanze oder eines dürretoleranten photosynthetischen Organismus, umfassend:
Anwendung von mindestens einer Behandlung mit einer effektiven Menge von Trimethylamin-N-oxid (TMAO), Trimethylamin-N-oxid-Dihydrat oder N,N-Dimethyldecylamin N-oxid (DDAO) auf eine Pflanze, einen Pflanzenteil, einen photosynthetischen Organismus oder Saatgut, wobei die effektive Menge TMAO, TMAO Dihydrat oder DDAO 0,1 bis 10 g pro Liter zum Sprühen oder zur Bewässerung beträgt oder wobei die mindestens eine Behandlung eine Samenbehandlung ist und die effektive Menge des TMAO, TMAO Dihydrat oder DDAO 0,1 g bis 1000 g pro 100 kg Saatgut beträgt und
Anbau der besagten Pflanze, des Pflanzenteils, des photosynthetischen Organismus oder Saatguts, wobei eine dürretolerante Pflanze oder ein photosynthetischer Organismus hergestellt wird.

2. Das Verfahren gemäß Anspruch 1, weiterhin umfassend:
Anwendung von mindestens einer zweiten Behandlung mit einer effektiven Menge an TMAO, TMAO Dihydrat oder DDAO auf besagte dürretolerante Pflanze oder auf besagten photosynthetischen Organismus, welche vorher mit TMAO, TMAO Dihydrat oder DDAO behandelt wurden.

3. Das Verfahren gemäß Anspruch 1 oder 2, wobei besagte mindestens eine Behandlung mit einer effektiven Menge TMAO, TMAO Dihydrat oder DDAO eine Bewässerungsbehandlung oder eine Spraybehandlung ist.

4. Das Verfahren gemäß einem der Ansprüche 1 bis 3, wobei besagte mindestens eine Behandlung mit einer effektiven Menge TMAO, TMAO Dihydrat oder DDAO, zwei oder mehr verschiedene Verbindungen ausgewählt aus der Gruppe bestehend aus TMAO, TMAO Dihydrat und DDAO umfasst.

5. Das Verfahren gemäß einem der Ansprüche 1 bis 4, wobei besagte dürretolerante Pflanze oder besagter dürretoleranter photosynthetischer Organismus eine Biomasse, Frucht oder Saatgutproduktion hat, die zwischen 6% und 30% liegt oder zwischen 31% und 50% mehr Biomasse, Frucht und Saatgutproduktion als dürregestresste Pflanzen oder photosynthetische Organismen hat, wobei der nicht-toleranten wassergestressten Pflanze oder dem nicht-toleranten wassergestressten photosynthetischen Organismus keine effektive Menge TMAO, TMAO Dihydrat oder DDAO verabreicht wurden.

6. Das Verfahren gemäß einem der Ansprüche 1 bis 5, weiterhin umfassend die Behandlung der besagten Pflanze, des Pflanzenteils, des photosynthetischen Organismus oder des Saatguts mit Salzen oder anderen Additiven und Anbau der besagten Pflanze, des Pflanzenteils, des photosynthetischen Organismus oder Saatguts, wobei eine dürretolerante Pflanze oder ein dürretoleranter Organismus produziert wird.

## Revendications

1. Un procédé de production d'une plante ou d'un organisme photosynthétique tolérant la sécheresse, comprenant :
le fait d'appliquer au moins un traitement d'une quantité efficace de N-oxyde de triméthylamine (TMAO), de N-oxyde de triméthylamine di-hydraté ou de N,N-diméthyldécylamine N-oxyde (DDAO) à une plante, une partie de plante, un organisme photosynthétique ou des semences, la quantité efficace du TMAO, TMAO di-hydraté ou DDAO étant de 0,1 à 10 g par litre pour la pulvérisation ou l'irrigation, ou ledit au moins un traitement étant un traitement de semences et la quantité efficace de TMAO, TMAO di-hydrate ou DDAO étant de 0,1 g à 1000 g pour 100 kg de semences ; et
le fait de cultiver ladite plante, ladite partie de plante, ledit organisme photosynthétique ou lesdites semences, une plante ou un organisme photosynthétique résistant à la sécheresse étant produit.

2. Le procédé selon la revendication 1, et comprenant en outre :
le fait d'appliquer au moins un deuxième traitement d'une quantité efficace de TMAO, TMAO di-hydrate ou DDAO sur ladite plante ou ledit organisme photosynthétique résistant à la sécheresse, préalablement traité avec du TMAO, du TMAO di-hydraté ou du DDAO.

3. Le procédé selon les revendications 1 ou 2, dans lequel ledit au moins un traitement de ladite quantité efficace de TMAO, de TMAO di-hydrate ou de DDAO est un traitement par irrigation ou un traitement par pulvérisation.

4. Le procédé selon l'une quelconque des revendications 1 à 3, dans lequel ledit au moins un traitement de ladite quantité efficace de TMAO, TMAO di-hydrate ou DDAO comprend deux composés différents, ou plus de deux, choisis dans le groupe constitué de TMAO, TMAO di-hydrate et DDAO.

5. Le procédé selon l'une quelconque des revendications 1 à 4, dans lequel ladite plante ou ledit organisme photosynthétique tolérant à la sécheresse a une production de biomasse, de fruits ou de semences qui est comprise entre 6 % et 30 % ou entre 31 % et 50 % de plus que la production de biomasse, de fruits ou de semences de plantes ou d'organismes photosynthétiques soumis à la sécheresse sur lesquels une quantité efficace de TMAO, TMAO di-hydrate ou DDAO n'a pas été appliquée aux plantes ou organismes photosynthétiques exposés à la sécheresse, non tolérants.

6. Le procédé selon l'une quelconque des revendications 1 à 5, comprenant en outre le fait d'appliquer des sels ou de tout autre additif sur ladite plante, ladite partie de plante, ledit organisme photosynthétique ou lesdites semences et le fait de cultiver ladite plante, ladite partie de plante, ledit organisme photosynthétique ou lesdites semence, une plante ou un organisme photosynthétique tolérant la sécheresse étant produit.


### SEQUENCE LISTING

<110> PLANT RESPONSE BIOTECH, S.L. CONSEJO SUPERIOR DE INVESTIGACIONES CIENTIFICAS
<120> METHOD FOR ENHANCING DROUGHT TOLERANCE IN PLANTS
<130> P11165PC00
<150> US61865549
   <151> 2013-08-13
<150> US14142285
   <151> 2013-12-27
<150> US14203261
   <151> 2014-03-10
<160> 44
<170> PatentIn version 3.5
<210> 1
   <211> 1380
   <212> DNA
   <213> Arabidopsis thaliana
<400> 1
   atggcaccag cacgaacccg agtcaactca ctcaacgtgg cagtgatcgg agccggagcc 60
   gccggactcg tagctgcaag agagctccgc cgcgagaatc acaccgtcgt cgttttcgaa 120
   cgtgactcaa aagtcggagg tctctgggta tacacaccta acagcgaacc agacccgctt 180
   agcctcgatc caaaccgaac catcgtccat tcaagcgtct atgattctct ccgaaccaat 240
   ctcccacgag agtgcatggg ttacagagac ttccccttcg tgcctcgacc tgaagatgac 300
   gaatcaagag actcgagaag gtaccctagt cacagagaag ttcttgctta ccttgaagac 360
   ttcgctagag aattcaaact tgtggagatg gttcgattta agaccgaagt agttcttgtc 420
   gagcctgaag ataagaaatg gagggttcaa tccaaaaatt cagatgggat ctccaaagat 480
   gagatctttg atgctgttgt tgtttgtaat ggacattata cagaacctag agttgctcat 540
   gttcctggta tagattcatg gccagggaag cagattcata gccacaatta ccgtgttcct 600
   gatcaattca aagaccaggt ggtggtagtg ataggaaatt ttgcgagtgg agctgatatc 660
   agcagggaca taacgggagt ggctaaagaa gtccatatcg cgtctagatc gaatccatct 720
   aagacatact caaaacttcc cgggtcaaac aatctatggc ttcactctat gatagaaagt 780
   gtacacgaag atgggacgat tgtttttcag aacggtaagg ttgtacaagc tgataccatt 840
   gtgcattgca ctggttacaa atatcacttc ccatttctca acaccaatgg ctatattact 900
   gttgaggata actgtgttgg accgctttac gaacatgtct ttccgcctgc gcttgctccc 960
   gggctttcct tcatcggttt accctggatg acactgcaat tctttatgtt tgagctccaa 1020
   agcaagtggg tggctgcagc tttgtctggc cgggtcacac ttccttcaga agagaaaatg 1080
   atggaagacg ttaccgccta ctatgcaaag cgtgaggctt tcgggcaacc taagagatac 1140
   acacatcgac ttggtggagg tcaggttgat taccttaatt ggatagcaga gcaaattggt 1200
   gcaccgcccg gtgaacaatg gagatatcag gaaataaatg gcggatacta cagacttgct 1260
   acacaatcag acactttccg tgataagtgg gacgatgatc atctcatagt tgaggcttat 1320
   gaggatttct tgagacagaa gctgattagt agtcttcctt ctcagttatt ggaatcttga 1380
<210> 2
   <211> 459
   <212> PRT
   <213> Arabidopsis thaliana
<400> 2
   Met Ala Pro Ala Arg Thr Arg Val Asn Ser Leu Asn Val Ala Val Ile
   1 5 10 15
   Gly Ala Gly Ala Ala Gly Leu Val Ala Ala Arg Glu Leu Arg Arg Glu
   20 25 30
   Asn His Thr Val Val Val Phe Glu Arg Asp Ser Lys Val Gly Gly Leu
   35 40 45
   Trp Val Tyr Thr Pro Asn Ser Glu Pro Asp Pro Leu Ser Leu Asp Pro
   50 55 60
   Asn Arg Thr Ile Val His Ser Ser Val Tyr Asp Ser Leu Arg Thr Asn
   65 70 75 80
   Leu Pro Arg Glu Cys Met Gly Tyr Arg Asp Phe Pro Phe Val Pro Arg
   85 90 95
   Pro Glu Asp Asp Glu Ser Arg Asp Ser Arg Arg Tyr Pro Ser His Arg
   100 105 110
   Glu Val Leu Ala Tyr Leu Glu Asp Phe Ala Arg Glu Phe Lys Leu Val
   115 120 125
   Glu Met Val Arg Phe Lys Thr Glu Val Val Leu Val Glu Pro Glu Asp
   130 135 140
   Lys Lys Trp Arg Val Gln Ser Lys Asn Ser Asp Gly Ile Ser Lys Asp
   145 150 155 160
   Glu Ile Phe Asp Ala Val Val Val Cys Asn Gly His Tyr Thr Glu Pro
   165 170 175
   Arg Val Ala His Val Pro Gly Ile Asp Ser Trp Pro Gly Lys Gln Ile
               180                 185                 190
   His Ser His Asn Tyr Arg Val Pro Asp Gln Phe Lys Asp Gln Val Val
           195                 200                 205
   Val Val Ile Gly Asn Phe Ala Ser Gly Ala Asp Ile Ser Arg Asp Ile
       210                 215                 220
   Thr Gly Val Ala Lys Glu Val His Ile Ala Ser Arg Ser Asn Pro Ser
   225                 230                 235                 240
   Lys Thr Tyr Ser Lys Leu Pro Gly Ser Asn Asn Leu Trp Leu His Ser
                   245                 250                 255
   Met Ile Glu Ser Val His Glu Asp Gly Thr Ile Val Phe Gln Asn Gly
               260                 265                 270
   Lys Val Val Gln Ala Asp Thr Ile Val His Cys Thr Gly Tyr Lys Tyr
           275                 280                 285
   His Phe Pro Phe Leu Asn Thr Asn Gly Tyr Ile Thr Val Glu Asp Asn
       290                 295                 300
   Cys Val Gly Pro Leu Tyr Glu His Val Phe Pro Pro Ala Leu Ala Pro
   305                 310                 315                 320
   Gly Leu Ser Phe Ile Gly Leu Pro Trp Met Thr Leu Gln Phe Phe Met
                   325                 330                 335
   Phe Glu Leu Gln Ser Lys Trp Val Ala Ala Ala Leu Ser Gly Arg Val
               340                 345                 350
   Thr Leu Pro Ser Glu Glu Lys Met Met Glu Asp Val Thr Ala Tyr Tyr
           355                 360                 365
   Ala Lys Arg Glu Ala Phe Gly Gln Pro Lys Arg Tyr Thr His Arg Leu
       370                 375                 380
   Gly Gly Gly Gln Val Asp Tyr Leu Asn Trp Ile Ala Glu Gln Ile Gly
   385                 390                 395                 400
   Ala Pro Pro Gly Glu Gln Trp Arg Tyr Gln Glu Ile Asn Gly Gly Tyr
                   405                 410                 415
   Tyr Arg Leu Ala Thr Gln Ser Asp Thr Phe Arg Asp Lys Trp Asp Asp
               420                 425                 430
   Asp His Leu Ile Val Glu Ala Tyr Glu Asp Phe Leu Arg Gln Lys Leu
   435 440 445
   Ile Ser Ser Leu Pro Ser Gln Leu Leu Glu Ser
   450 455
<210> 3
   <211> 1552
   <212> DNA
   <213> Brassica rapa
<400> 3
   gcacgaggca aaaaaacaaa cataacatta acatttgaaa aatggcacca gctcaaaacc 60
   tagtcagttc gaaacacgta gcggtgatcg gagccggagc atccgggtta atagcggcca 120
   gagagctcca tcgtgaaggt cacaccgtcg tcgtttttga gcgggagaaa caagtgggag 180
   gtctctggat ttactcacct aaatctgaat ccgacccgct tggtctcgac ccgacaagac 240
   ctatagttca ctcgagtgtc tacgagtctc tccgaaccaa cctcccgaga gagtgtatgg 300
   gtttcaggga ttttccgttc gtgccatgtg ttgatgactt ttcaagagac tcgagaaggt 360
   atccgagcca cagggaagtt cttgcgtacc ttcaagactt tgctagagag tttaaaatag 420
   aggagatggt ccggttcgag accgaggtgg ttcgggttga gccggttgat ggaaaatgga 480
   gggtccgatc caaaaactcc gatgatctct ccgaagatga gatctttgac gcagtcgttg 540
   tttgcagtgg gcattatacc gaaccttatg ttgctcatat tcctgggata aaatcatggc 600
   caggaaagca gatccatagc cataactaca gagttccggg tccattcaaa aatgaggtgg 660
   tggtggtcat cggaaatttt gcgagcggtg ccgatattag tagagacgta gctaaggtcg 720
   ccaaagaagt ccacgttgcg tctagaggga gtgaagctag tacgtatgag aagctttccg 780
   tgcccaccaa caatctatgg attcattctg agatagagac tgcatgtgat gatggttcaa 840
   ttgttttcaa aaatgggaag gcggttcatg cagatactgt tgtgtattgt accgggtaca 900
   agtataagtt tccatttctt gaaaccaatg gttatatgag cattgatgat aaccgcgttg 960
   aacctttgta caaacatgtc tttccaccgg cgcttgcccc agggctttct tttgttggtt 1020
   taccagggat gggcatacaa ttcgtcatgt ttgaaatcca aagcaaatgg gtagctgcag 1080
   ttttgtctgg acgagttaca cttcctgcac cagaaaaaat gatggaagat cttattgcat 1140
   cgtatgccat gcttgaagcg ttaggtattc ccaagagata tacacataaa ttgggtaaaa 1200
   ttcagtctaa ttatcttgac tgggtcgcag aagaatgtgg ttgtcagcct gttgagcctt 1260
   ggagaactca acaagttgac cgtggttatg agagacttgt ttctaaccct gaaaattacc 1320
   gcgatgaatg ggacgacgat gatctcataa aagaagcgta cgaggatttt gctagtaaga 1380
   agttgattag ctttcttcct tcttatttcc ccaaatcagg aagatgatat cccataatgg 1440
   tgcctacttg tttttaaggg tctacttgta ttatttttaa aaatgttggt tttaataaag 1500
   ctgaatgtaa gggttgcttg ttatacaatg gctactactt ttccctcgtg cc 1552
<210> 4
   <211> 461
   <212> PRT
   <213> Brassica rapa
<400> 4
   Met Ala Pro Ala Gln Asn Leu Val Ser Ser Lys His Val Ala Val Ile
   1               5                   10                  15
   Gly Ala Gly Ala Ser Gly Leu Ile Ala Ala Arg Glu Leu His Arg Glu
               20                  25                  30
   Gly His Thr Val Val Val Phe Glu Arg Glu Lys Gln Val Gly Gly Leu
           35                  40                  45
   Trp Ile Tyr Ser Pro Lys Ser Glu Ser Asp Pro Leu Gly Leu Asp Pro
       50                  55                  60
   Thr Arg Pro Ile Val His Ser Ser Val Tyr Glu Ser Leu Arg Thr Asn
   65                  70                  75                  80
   Leu Pro Arg Glu Cys Met Gly Phe Arg Asp Phe Pro Phe Val Pro Cys
                   85                  90                  95
   Val Asp Asp Phe Ser Arg Asp Ser Arg Arg Tyr Pro Ser His Arg Glu
               100                 105                 110
   Val Leu Ala Tyr Leu Gln Asp Phe Ala Arg Glu Phe Lys Ile Glu Glu
           115                 120                 125
   Met Val Arg Phe Glu Thr Glu Val Val Arg Val Glu Pro Val Asp Gly
       130                 135                 140
   Lys Trp Arg Val Arg Ser Lys Asn Ser Asp Asp Leu Ser Glu Asp Glu
   145                 150                 155                 160
   Ile Phe Asp Ala Val Val Val Cys Ser Gly His Tyr Thr Glu Pro Tyr
                   165                 170                 175
   Val Ala His Ile Pro Gly Ile Lys Ser Trp Pro Gly Lys Gln Ile His
               180                 185                 190
   Ser His Asn Tyr Arg Val Pro Gly Pro Phe Lys Asn Glu Val Val Val
           195                 200                 205
   Val Ile Gly Asn Phe Ala Ser Gly Ala Asp Ile Ser Arg Asp Val Ala
       210                 215                 220
   Lys Val Ala Lys Glu Val His Val Ala Ser Arg Gly Ser Glu Ala Ser
   225                 230                 235                 240
   Thr Tyr Glu Lys Leu Ser Val Pro Thr Asn Asn Leu Trp Ile His Ser
                   245                 250                 255
   Glu Ile Glu Thr Ala Cys Asp Asp Gly Ser Ile Val Phe Lys Asn Gly
               260                 265                 270
   Lys Ala Val His Ala Asp Thr Val Val Tyr Cys Thr Gly Tyr Lys Tyr
           275                 280                 285
   Lys Phe Pro Phe Leu Glu Thr Asn Gly Tyr Met Ser Ile Asp Asp Asn
       290                 295                 300
   Arg Val Glu Pro Leu Tyr Lys His Val Phe Pro Pro Ala Leu Ala Pro
   305                 310                 315                 320
   Gly Leu Ser Phe Val Gly Leu Pro Gly Met Gly Ile Gln Phe Val Met
                   325                 330                 335
   Phe Glu Ile Gln Ser Lys Trp Val Ala Ala Val Leu Ser Gly Arg Val
               340                 345                 350
   Thr Leu Pro Ala Pro Glu Lys Met Met Glu Asp Leu Ile Ala Ser Tyr
           355                 360                 365
   Ala Met Leu Glu Ala Leu Gly Ile Pro Lys Arg Tyr Thr His Lys Leu
       370                 375                 380
   Gly Lys Ile Gln Ser Asn Tyr Leu Asp Trp Val Ala Glu Glu Cys Gly
   385                 390                 395                 400
   Cys Gln Pro Val Glu Pro Trp Arg Thr Gln Gln Val Asp Arg Gly Tyr
                   405                 410                 415
   Glu Arg Leu Val Ser Asn Pro Glu Asn Tyr Arg Asp Glu Trp Asp Asp
               420                 425                 430
   Asp Asp Leu Ile Lys Glu Ala Tyr Glu Asp Phe Ala Ser Lys Lys Leu
           435                 440                 445
   Ile Ser Phe Leu Pro Ser Tyr Phe Pro Lys Ser Gly Arg
       450                 455                 460
<210> 5
   <211> 1526
   <212> DNA
   <213> Cucumis sativus
<400> 5
   gaaaacatga ataaacgaat cttatcataa tttgcaaaaa tcgaaaccaa attagttgac      60
   aaccacatcg aacaagaatc atcaataatc caattccctt ttctaatcgg aaaatcaaac     120
   ggatgttatc tcctctcaat ttcctcccaa cttcccgccg cgtggcagta atcggcgccg     180
   gtgccggtgg cctcgtcact gcccgtgagc tcggccgcga gggccaccat gtcgtcgttt     240
   tcgaacgtaa tactcgaatc ggagggacct gggtatattc ctcagagatt gaatccgacc     300
   cacttggact cgacccaaat cggacccgaa ttcacagcag tctctacaaa tctctacgca     360
   ccaatctccc cagagaactc atgggggtcc gcgattaccc ttttgttcct cgagaagggg     420
   aggatcgaga tcccaggcga tttccaagtc accgggaggt tctgaagtat ttagaagatt     480
   tcgctaatga atttgggatt tgtaaattgg tgagatttgg aactgaggtg gtatttgctg     540
   gtctggagga ggttgggaaa tggaggattg aatttagatg tgaaaatggg gatgttgaag     600
   aagacctttt tgatgctctg gttgtttgtg ttggcaatta ttcacagcct cgagtggcag     660
   agattcctgg gattgatgga tggcctgggg agcaagtgca tagtcacaat tatcgtgatc     720
   ccgaaccatt tcggggtaag gttgttgtct tgataggtta ttcttcgagt ggtacagaca     780
   tttctcagga gctcattggg gttgccaaag aaattcatat tgcttggaga tcaactaaaa     840
   cagagctttt gaacacagaa tcaattaaca gtaatgtgtc atttcatcca atgattgaaa     900
   gtgtccataa agatggggca gtggtttttc aagacgggtg cgttgttttg gctgatatta     960
   ttctgcattg cactgggtac aaatatcatt tcccttttct tgaaaccaat ggcattgtta    1020
   cggtggacaa caaccgtgta ggacccctat acaagcatgt cttcccccca gcattggccc    1080
   cagggctttc ctttgttggg ttaccattta aggctgttcc tttgcccatc tttgagcttc    1140
   aaagcaattg gattgctggt gttttatcaa acaggattgc acttccatca aaagaggaaa    1200
   tgttggcaga tgttaaagct ttctatgaaa atcttgaagc ttttgggaag cccaagcatc    1260
   ggacccatga attgggtgat gatatgcctg tgtattgtaa ctggcttgca acaacttgtg    1320
   gttgtccagc ctttgaagaa tggaggaaga aaatgtacat tgctattggt atttataaaa    1380
   aggccaatct cgagacatat cgtgatgatt ggcaggacaa tgagttgatt cgtcaagctt    1440
   acgaggaatt cagcaagtat aaatacaaat gaaaggacac tcaaaaccac atagttttga    1500
   atgcttcata agattggttc tatatg                                         1526
<210> 6
   <211> 449
   <212> PRT
   <213> Cucumis sativus
<400> 6
   Met Leu Ser Pro Leu Asn Phe Leu Pro Thr Ser Arg Arg Val Ala Val
   1               5                   10                  15
   Ile Gly Ala Gly Ala Gly Gly Leu Val Thr Ala Arg Glu Leu Gly Arg
               20                  25                  30
   Glu Gly His His Val Val Val Phe Glu Arg Asn Thr Arg Ile Gly Gly
           35                  40                  45
   Thr Trp Val Tyr Ser Ser Glu Ile Glu Ser Asp Pro Leu Gly Leu Asp
       50                  55                  60
   Pro Asn Arg Thr Arg Ile His Ser Ser Leu Tyr Lys Ser Leu Arg Thr
   65                  70                  75                  80
   Asn Leu Pro Arg Glu Leu Met Gly Val Arg Asp Tyr Pro Phe Val Pro
                   85                  90                  95
   Arg Glu Gly Glu Asp Arg Asp Pro Arg Arg Phe Pro Ser His Arg Glu
               100                 105                 110
   Val Leu Lys Tyr Leu Glu Asp Phe Ala Asn Glu Phe Gly Ile Cys Lys
           115                 120                 125
   Leu Val Arg Phe Gly Thr Glu Val Val Phe Ala Gly Leu Glu Glu Val
       130                 135                 140
   Gly Lys Trp Arg Ile Glu Phe Arg Cys Glu Asn Gly Asp Val Glu Glu
   145                 150                 155                 160
   Asp Leu Phe Asp Ala Leu Val Val Cys Val Gly Asn Tyr Ser Gln Pro
                   165                 170                 175
   Arg Val Ala Glu Ile Pro Gly Ile Asp Gly Trp Pro Gly Glu Gln Val
               180                 185                 190
   His Ser His Asn Tyr Arg Asp Pro Glu Pro Phe Arg Gly Lys Val Val
           195                 200                 205
   Val Leu Ile Gly Tyr Ser Ser Ser Gly Thr Asp Ile Ser Gln Glu Leu
       210                 215                 220
   Ile Gly Val Ala Lys Glu Ile His Ile Ala Trp Arg Ser Thr Lys Thr
   225                 230                 235                 240
   Glu Leu Leu Asn Thr Glu Ser Ile Asn Ser Asn Val Ser Phe His Pro
   245 250 255
   Met Ile Glu Ser Val His Lys Asp Gly Ala Val Val Phe Gln Asp Gly
   260 265 270
   Cys Val Val Leu Ala Asp Ile Ile Leu His Cys Thr Gly Tyr Lys Tyr
   275 280 285
   His Phe Pro Phe Leu Glu Thr Asn Gly Ile Val Thr Val Asp Asn Asn
   290 295 300
   Arg Val Gly Pro Leu Tyr Lys His Val Phe Pro Pro Ala Leu Ala Pro
   305 310 315 320
   Gly Leu Ser Phe Val Gly Leu Pro Phe Lys Ala Val Pro Leu Pro Ile
   325 330 335
   Phe Glu Leu Gln Ser Asn Trp Ile Ala Gly Val Leu Ser Asn Arg Ile
   340 345 350
   Ala Leu Pro Ser Lys Glu Glu Met Leu Ala Asp Val Lys Ala Phe Tyr
   355 360 365
   Glu Asn Leu Glu Ala Phe Gly Lys Pro Lys His Arg Thr His Glu Leu
   370 375 380
   Gly Asp Asp Met Pro Val Tyr Cys Asn Trp Leu Ala Thr Thr Cys Gly
   385 390 395 400
   Cys Pro Ala Phe Glu Glu Trp Arg Lys Lys Met Tyr Ile Ala Ile Gly
   405 410 415
   Ile Tyr Lys Lys Ala Asn Leu Glu Thr Tyr Arg Asp Asp Trp Gln Asp
   420 425 430
   Asn Glu Leu Ile Arg Gln Ala Tyr Glu Glu Phe Ser Lys Tyr Lys Tyr
   435 440 445
   Lys
<210> 7
   <211> 1541
   <212> DNA
   <213> Cucumis sativus
<400> 7
   atggaattca tcgctacttg ccaccctgac tttcctcccc ctccggcctc acctcaaccc        60
   acgacgatgc aacactcccg ccgcgtggca gtgatcggcg ccggtggcgc aggcctcatc       120
   tccgcccgcc aactttcccg ggagggccac caagtcgtgg tcttcgaacg gaataatcag       180
   atcggagggg tctgggtata ttcgcccgaa attgaatccg acccacttgg agttcaccct       240
   aagcggactc gaatacatag cagcctctac aaatctctac gaaccaatat ccccagagaa       300
   gtcatggggg tccgtgattt cccctttgtt cctcgagaag gggaggatcg agatcccagg       360
   cgatttccaa gtcaccggga ggttctgaag tatttagaag atttcgctaa tgaatttggg       420
   atttgtaaat tggtgagatt tagaactgag gtggtgtttg ctggtttgga gaagcttggc       480
   aaatggaggg ttgaattcag atgtgagaat ggggatgttc attatgacat ttttgatgct       540
   gtagttgttt gtgttggcaa tttttcgcag cctcgagtag cagagattcc agggattgat       600
   ggatggcctg gggagcaagt gcatagtcac aattatcgtg atcccgaacc atttcgcggt       660
   aaggttgttg tgttgatagg ttattcttcg agtggtacgg acatttctca ggagctcatt       720
   ggggttgcca aagaaattca tattgcttgc aggccagcta aaacagagtc ttcggacgaa       780
   aaatcaatta ttagtaacgt ctcatttcat ccaatgatcg aaagtgtcca taaagatgga       840
   acggtggtct ttcaagacgg gtccgtcgtt tcggctgatg ttattctgca ttgtactggg       900
   tacaaatatc atttcccgtt tcttgaaacc aatggcactg ttacggtgga cgacaaccgt       960
   gtaggacctc ttttcaagca tgtcttcccc ccagcattgg ccccagggct ttccttcgtt      1020
   gggttaccat ttaaggttgt tccttttgtc atatttgagc ttcaaagcaa ttggattgct      1080
   ggtgttttat caaacaggat tgcacttcca tcaaaagagg aaatgttggc agatgttaaa      1140
   gctttttatg aagaactcga agctcgtggc aagcccaagc atcggaccca taaattgggt      1200
   ggttatacgc ctgcctactg taactggctt gcagcaactt gtggttgtcc tccctatgaa      1260
   gaatggagaa aggaaatgtt tgttgctact gatattaata aagtggccaa tcttgagtca      1320
   taccgtgatg attggcatga cgatgagttg attcatcaag cttatgaaga atttggcaag      1380
   tatactacta caaatgaagg aagtcaaaac cactcgaatt tgaatgttta ataagtttgg      1440
   ttctatatat ttgtacattg cacaatcatg tgtcttgatt ataaatgttg gatcttgatt      1500
   tataaataaa aatgaaaata atattagacc agattatgac a                          1541
<210> 8
   <211> 476
   <212> PRT
   <213> Cucumis sativus
<400> 8
   Met Glu Phe Ile Ala Thr Cys His Pro Asp Phe Pro Pro Pro Pro Ala
   1               5                   10                  15
   Ser Pro Gln Pro Thr Thr Met Gln His Ser Arg Arg Val Ala Val Ile
               20                  25                  30
   Gly Ala Gly Gly Ala Gly Leu Ile Ser Ala Arg Gln Leu Ser Arg Glu
           35                  40                  45
   Gly His Gln Val Val Val Phe Glu Arg Asn Asn Gln Ile Gly Gly Val
       50                  55                  60
   Trp Val Tyr Ser Pro Glu Ile Glu Ser Asp Pro Leu Gly Val His Pro
   65                  70                  75                  80
   Lys Arg Thr Arg Ile His Ser Ser Leu Tyr Lys Ser Leu Arg Thr Asn
                   85                  90                  95
   Ile Pro Arg Glu Val Met Gly Val Arg Asp Phe Pro Phe Val Pro Arg
               100                 105                 110
   Glu Gly Glu Asp Arg Asp Pro Arg Arg Phe Pro Ser His Arg Glu Val
           115                 120                 125
   Leu Lys Tyr Leu Glu Asp Phe Ala Asn Glu Phe Gly Ile Cys Lys Leu
       130                 135                 140
   Val Arg Phe Arg Thr Glu Val Val Phe Ala Gly Leu Glu Lys Leu Gly
   145                 150                 155                 160
   Lys Trp Arg Val Glu Phe Arg Cys Glu Asn Gly Asp Val His Tyr Asp
                   165                 170                 175
   Ile Phe Asp Ala Val Val Val Cys Val Gly Asn Phe Ser Gln Pro Arg
               180                 185                 190
   Val Ala Glu Ile Pro Gly Ile Asp Gly Trp Pro Gly Glu Gln Val His
           195                 200                 205
   Ser His Asn Tyr Arg Asp Pro Glu Pro Phe Arg Gly Lys Val Val Val
       210                 215                 220
   Leu Ile Gly Tyr Ser Ser Ser Gly Thr Asp Ile Ser Gln Glu Leu Ile
   225                 230                 235                 240
   Gly Val Ala Lys Glu Ile His Ile Ala Cys Arg Pro Ala Lys Thr Glu
                   245                 250                 255
   Ser Ser Asp Glu Lys Ser Ile Ile Ser Asn Val Ser Phe His Pro Met
               260                 265                 270
   Ile Glu Ser Val His Lys Asp Gly Thr Val Val Phe Gln Asp Gly Ser
           275                 280                 285
   Val Val Ser Ala Asp Val Ile Leu His Cys Thr Gly Tyr Lys Tyr His
       290                 295                 300
   Phe Pro Phe Leu Glu Thr Asn Gly Thr Val Thr Val Asp Asp Asn Arg
   305                 310                 315                 320
   Val Gly Pro Leu Phe Lys His Val Phe Pro Pro Ala Leu Ala Pro Gly
                   325                 330                 335
   Leu Ser Phe Val Gly Leu Pro Phe Lys Val Val Pro Phe Val Ile Phe
               340                 345                 350
   Glu Leu Gln Ser Asn Trp Ile Ala Gly Val Leu Ser Asn Arg Ile Ala
           355                 360                 365
   Leu Pro Ser Lys Glu Glu Met Leu Ala Asp Val Lys Ala Phe Tyr Glu
       370                 375                 380
   Glu Leu Glu Ala Arg Gly Lys Pro Lys His Arg Thr His Lys Leu Gly
   385                 390                 395                 400
   Gly Tyr Thr Pro Ala Tyr Cys Asn Trp Leu Ala Ala Thr Cys Gly Cys
                   405                 410                 415
   Pro Pro Tyr Glu Glu Trp Arg Lys Glu Met Phe Val Ala Thr Asp Ile
               420                 425                 430
   Asn Lys Val Ala Asn Leu Glu Ser Tyr Arg Asp Asp Trp His Asp Asp
           435                 440                 445
   Glu Leu Ile His Gln Ala Tyr Glu Glu Phe Gly Lys Tyr Thr Thr Thr
       450                 455                 460
   Asn Glu Gly Ser Gln Asn His Ser Asn Leu Asn Val
   465                 470                 475
<210> 9
   <211> 1570
   <212> DNA
   <213> Cucumis sativus
<400> 9
   atgtggagta gagttggtag cacttcagct atcattcata cttttatcaa aaaagattcc      60
   catttcttac ccataaatcc atgttctact caattagcta cactcaattt cctcccctcc     120
   cctcaaccat caacgatgcc tcactccagc cgcgtggcag tgatcggcgc cggcgccgga     180
   ggcctcgtct cagcccggga actttcccgg gaggaccacc atgtggttgt attcgaacgg     240
   aatactcaaa ttggaggggc ctgggtatat tcaccggaaa ttgaatccga cccacttgga     300
   gtcgacccgg atcggacccg aatccatagc agcctcttca aatctcttcg aaccaatata     360
   cctagagaac tcatgggggt ccgggatttc ccgtttgttc ctcgagaagg ggaggatcga     420
   gatccgaggc gatttccaag tcatcaggag gttcgcaagt atttggaaga tttcgctaat     480
   gaatttgggg tttacaaatt tgtgagattt ggaactgagg ttgtgtttgc tggtttggag     540
   gagcttggga aatggaggat tgaatttaga tgtgaaaatg gggacgttga ttatgagatt     600
   tttgatgctg tggttgtttg tgttgggaat tattcgcagc ctcgagtagc agagattcct     660
   gggattgatg gatggcctgg agagcaagtg catagtcaca attatcgtga tcccgaacca     720
   tttcggggta aggttgttgt gttgataggt tattcttcga gtggaacaga catttctcag     780
   gagctcattg gggttgccaa agaaattcat attgtttgga gatcacctaa aacagagctt      840
   ttggacagag aatcaattat tagtaatgtt tcatttcatc caatgattga aagtgtgtgt     900
   aaagatggga cagtggtctt tcaagacggg tgtgttgttt cggctgatgt aattttgcat     960
   tgcactgggt acaactatca tttccctttc cttgaaacca atggcaatgt tacagtggac    1020
   gacaaccgtg taggacctct atacaagcat gtcttccccc cagcattggc cccggggctt    1080
   tcctttgttg gattaccatt caaggttatt ccttttccct tgtttgagct tcaaagcaat    1140
   tgggttgctg gtgttttatc aaaaaggatt gcacttccat caaaagagga aatgttggca    1200
   gatgttaaag ctttctatga agatcttgaa gctcttggca agcccaagca tcggacccat    1260
   ttattgggtg attatatgat gcctgcctat tgtaattggg ttgcaacaac ttgtggttgt    1320
   cctccctatg aagaatggag aaaggaaatg aacatttctg ttcatcttta tagattgccc    1380
   aatctcaaga cgtaccgtga tgattggcac gatgatgagt tgattcgtca agcttacgag    1440
   gagtttagca agtataatac aaatgtaaga agtcaaaaca actcaaattt gaatgcttca    1500
   taagatttgt tgtatatgtg tacatttaca tatttatgtt gtcattgatc cttcctcctc    1560
   gttacaaata                                                           1570
<210> 10
   <211> 500
   <212> PRT
   <213> Cucumis sativus
<400> 10
   Met Trp Ser Arg Val Gly Ser Thr Ser Ala Ile Ile His Thr Phe Ile
   1               5                   10                  15
   Lys Lys Asp Ser His Phe Leu Pro Ile Asn Pro Cys Ser Thr Gln Leu
               20                  25                  30
   Ala Thr Leu Asn Phe Leu Pro Ser Pro Gln Pro Ser Thr Met Pro His
           35                  40                  45
   Ser Ser Arg Val Ala Val Ile Gly Ala Gly Ala Gly Gly Leu Val Ser
       50                  55                  60
   Ala Arg Glu Leu Ser Arg Glu Asp His His Val Val Val Phe Glu Arg
   65                  70                  75                  80
   Asn Thr Gln Ile Gly Gly Ala Trp Val Tyr Ser Pro Glu Ile Glu Ser
                   85                  90                  95
   Asp Pro Leu Gly Val Asp Pro Asp Arg Thr Arg Ile His Ser Ser Leu
               100                 105                 110
   Phe Lys Ser Leu Arg Thr Asn Ile Pro Arg Glu Leu Met Gly Val Arg
           115                 120                 125
   Asp Phe Pro Phe Val Pro Arg Glu Gly Glu Asp Arg Asp Pro Arg Arg
       130                 135                 140
   Phe Pro Ser His Gln Glu Val Arg Lys Tyr Leu Glu Asp Phe Ala Asn
   145                 150                 155                 160
   Glu Phe Gly Val Tyr Lys Phe Val Arg Phe Gly Thr Glu Val Val Phe
                   165                 170                 175
   Ala Gly Leu Glu Glu Leu Gly Lys Trp Arg Ile Glu Phe Arg Cys Glu
               180                 185                 190
   Asn Gly Asp Val Asp Tyr Glu Ile Phe Asp Ala Val Val Val Cys Val
           195                 200                 205
   Gly Asn Tyr Ser Gln Pro Arg Val Ala Glu Ile Pro Gly Ile Asp Gly
       210                 215                 220
   Trp Pro Gly Glu Gln Val His Ser His Asn Tyr Arg Asp Pro Glu Pro
   225                 230                 235                 240
   Phe Arg Gly Lys Val Val Val Leu Ile Gly Tyr Ser Ser Ser Gly Thr
                   245                 250                 255
   Asp Ile Ser Gln Glu Leu Ile Gly Val Ala Lys Glu Ile His Ile Val
               260                 265                 270
   Trp Arg Ser Pro Lys Thr Glu Leu Leu Asp Arg Glu Ser Ile Ile Ser
           275                 280                 285
   Asn Val Ser Phe His Pro Met Ile Glu Ser Val Cys Lys Asp Gly Thr
       290                 295                 300
   Val Val Phe Gln Asp Gly Cys Val Val Ser Ala Asp Val Ile Leu His
   305                 310                 315                 320
   Cys Thr Gly Tyr Asn Tyr His Phe Pro Phe Leu Glu Thr Asn Gly Asn
                   325                 330                 335
   Val Thr Val Asp Asp Asn Arg Val Gly Pro Leu Tyr Lys His Val Phe
               340                 345                 350
   Pro Pro Ala Leu Ala Pro Gly Leu Ser Phe Val Gly Leu Pro Phe Lys
           355                 360                 365
   Val Ile Pro Phe Pro Leu Phe Glu Leu Gln Ser Asn Trp Val Ala Gly
       370                 375                 380
   Val Leu Ser Lys Arg Ile Ala Leu Pro Ser Lys Glu Glu Met Leu Ala
   385                 390                 395                 400
   Asp Val Lys Ala Phe Tyr Glu Asp Leu Glu Ala Leu Gly Lys Pro Lys
                   405                 410                 415
   His Arg Thr His Leu Leu Gly Asp Tyr Met Met Pro Ala Tyr Cys Asn
               420                 425                 430
   Trp Val Ala Thr Thr Cys Gly Cys Pro Pro Tyr Glu Glu Trp Arg Lys
           435                 440                 445
   Glu Met Asn Ile Ser Val His Leu Tyr Arg Leu Pro Asn Leu Lys Thr
       450                 455                 460
   Tyr Arg Asp Asp Trp His Asp Asp Glu Leu Ile Arg Gln Ala Tyr Glu
   465                 470                 475                 480
   Glu Phe Ser Lys Tyr Asn Thr Asn Val Arg Ser Gln Asn Asn Ser Asn
                   485                 490                 495
   Leu Asn Ala Ser
               500
<210> 11
   <211> 1590
   <212> DNA
   <213> Cucumis sativus
<400> 11
   aacatgaata aacgaatctt atcataattt gcaaaaatcg aaaccaaatt agttgacaac      60
   cacatcgaac aagaatcatc aataatccaa ttcccttttc taatcggaaa atcaaacgga      120
   tgttatctcc tctcaatttc ctcccaactt cccgccgcgt ggcagtaatc ggcgccggtg      180
   ccggtggcct cgtcactgcc cgtgagctcg gccgcgaggg ccaccatgtc gtcgttttcg      240
   aacgtaatac tcgaatcgga gggacctggg tatattcctc agagattgaa tccgacccac      300
   ttggactcga cccaaatcgg acccgaattc acagcagtct ctacaaatct ctacgcacca      360
   atctccccag agaactcatg ggggtccgcg attacccttt tgttcctcga gaaggggagg      420
   atagagatcc gaggcgattt ccaagtcacc gggaggttct gaagtattta gaagatttcg      480
   ctaatgaatt tgggatttgt aaattggtga gatttggaac tgaggtggta tttgctggtc      540
   tggaggaggt tgggaaatgg aggattgaat ttagatgtga aaatggggat gttgaagaag      600
   acctttttga tgctctggtt gtttgtgttg gcaattattc acagcctcga gtggcagaga      660
   ttcctgggat tgatggatgg cctggggagc aattacatag tcacaattat cgtgatcccg      720
   aaccatttcg gggtaaggtt gttgtcttga taggttattc ttcgagtggt acagacattt      780
   ctcaggagct cattggggtt gccaaagaaa ttcatattgc ttggagatca actaaaacag      840
   agcttttgaa cacagaatca attaacagta atgtgtcatt tcatccaatg attgaaagtg      900
   tccataaaga tggggcagtg gtttttcaag acgggtgcgt tgttttggct gatattattc      960
   tgcattgcac tgggtacaaa tatcatttcc cttttcttga aaccaatggc attgttacgg     1020
   tggacaacaa ccgtgtaggg cccctataca agcatgtctt ccccccagca ttggccccag     1080
   ggctttcctt tgttgggtta ccatttaagg ttgttccttt tcccttgttt gagcttcaaa     1140
   gcaattggat tgctggtgtt ttatcaaaca ggattgcact tccatcaaaa gaggaaatgt     1200
   tggcagatgt taaagctttc tatgaaaatc ttgaagcttt tgggaagccc aagcatcgga     1260
   cccatgaatt gggtgatgat atgcctgcct acttggactg gcttgcagca gtatgtggtt     1320
   gtcctgccta tgaagaatgg agaaaggaaa tgtacattgc tactcatatg aataaagtgg     1380
   ccaatctcag gtcataccgt gacgattggc acgacaatga gttgattcgt caagcttatg     1440
   aagaatttag caagtatgca acaaatgaag gaagtgggaa ccactcaaaa ttgagtgttt     1500
   gataagattg gttgtataca tgttacataa tttatgtgtt gttgattaat gaaaataata     1560
   gtagtatggg atcgcccatt ttctttacaa                                      1590
<210> 12
   <211> 460
   <212> PRT
   <213> Cucumis sativus
<400> 12
   Met Leu Ser Pro Leu Asn Phe Leu Pro Thr Ser Arg Arg Val Ala Val
   1               5                   10                  15
   Ile Gly Ala Gly Ala Gly Gly Leu Val Thr Ala Arg Glu Leu Gly Arg
               20                  25                  30
   Glu Gly His His Val Val Val Phe Glu Arg Asn Thr Arg Ile Gly Gly
           35                  40                  45
   Thr Trp Val Tyr Ser Ser Glu Ile Glu Ser Asp Pro Leu Gly Leu Asp
       50                  55                  60
   Pro Asn Arg Thr Arg Ile His Ser Ser Leu Tyr Lys Ser Leu Arg Thr
   65                  70                  75                  80
   Asn Leu Pro Arg Glu Leu Met Gly Val Arg Asp Tyr Pro Phe Val Pro
                   85                  90                  95
   Arg Glu Gly Glu Asp Arg Asp Pro Arg Arg Phe Pro Ser His Arg Glu
               100                 105                 110
   Val Leu Lys Tyr Leu Glu Asp Phe Ala Asn Glu Phe Gly Ile Cys Lys
           115                 120                 125
   Leu Val Arg Phe Gly Thr Glu Val Val Phe Ala Gly Leu Glu Glu Val
       130                 135                 140
   Gly Lys Trp Arg Ile Glu Phe Arg Cys Glu Asn Gly Asp Val Glu Glu
   145                 150                 155                 160
   Asp Leu Phe Asp Ala Leu Val Val Cys Val Gly Asn Tyr Ser Gln Pro
                   165                 170                 175
   Arg Val Ala Glu Ile Pro Gly Ile Asp Gly Trp Pro Gly Glu Gln Leu
               180                 185                 190
   His Ser His Asn Tyr Arg Asp Pro Glu Pro Phe Arg Gly Lys Val Val
           195                 200                 205
   Val Leu Ile Gly Tyr Ser Ser Ser Gly Thr Asp Ile Ser Gln Glu Leu
       210                 215                 220
   Ile Gly Val Ala Lys Glu Ile His Ile Ala Trp Arg Ser Thr Lys Thr
   225                 230                 235                 240
   Glu Leu Leu Asn Thr Glu Ser Ile Asn Ser Asn Val Ser Phe His Pro
                   245                 250                 255
   Met Ile Glu Ser Val His Lys Asp Gly Ala Val Val Phe Gln Asp Gly
               260                 265                 270
   Cys Val Val Leu Ala Asp Ile Ile Leu His Cys Thr Gly Tyr Lys Tyr
           275                 280                 285
   His Phe Pro Phe Leu Glu Thr Asn Gly Ile Val Thr Val Asp Asn Asn
       290                 295                 300
   Arg Val Gly Pro Leu Tyr Lys His Val Phe Pro Pro Ala Leu Ala Pro
   305                 310                 315                 320
   Gly Leu Ser Phe Val Gly Leu Pro Phe Lys Val Val Pro Phe Pro Leu
                   325                 330                 335
   Phe Glu Leu Gln Ser Asn Trp Ile Ala Gly Val Leu Ser Asn Arg Ile
               340                 345                 350
   Ala Leu Pro Ser Lys Glu Glu Met Leu Ala Asp Val Lys Ala Phe Tyr
           355                 360                 365
   Glu Asn Leu Glu Ala Phe Gly Lys Pro Lys His Arg Thr His Glu Leu
       370                 375                 380
   Gly Asp Asp Met Pro Ala Tyr Leu Asp Trp Leu Ala Ala Val Cys Gly
   385                 390                 395                 400
   Cys Pro Ala Tyr Glu Glu Trp Arg Lys Glu Met Tyr Ile Ala Thr His
                   405                 410                 415
   Met Asn Lys Val Ala Asn Leu Arg Ser Tyr Arg Asp Asp Trp His Asp
               420                 425                 430
   Asn Glu Leu Ile Arg Gln Ala Tyr Glu Glu Phe Ser Lys Tyr Ala Thr
           435                 440                 445
   Asn Glu Gly Ser Gly Asn His Ser Lys Leu Ser Val
       450                 455                 460
<210> 13
   <211> 1600
   <212> DNA
   <213> Medicago truncatula
<400> 13
   atgaatgaac atgttcatac tgtaagcatt caattcgatt ccaagccaat gaaattcatc 60
   atgtccaccg caacaccact tctcacaccc cgccacgtgg cagtcatcgg agccggcgcc 120
   ggaggcttag tagcagcacg cgagctccga cgagaaggac atcaagtagt agtcttcgag 180
   cgaggagaag aattgggcgg ttcatgggtc tacacttcag aggtagaatc cgacccactc 240
   ggtttggacc cgaaccggaa gcttatccac tcgagcctat acaattcact ccgaaccaat 300
   ttgcctcggg agagtatggg tttccgagat taccctttta ggaggaaaga agagaagggg 360
   agagattcta gaaggttccc gagtcatgga gaggtattga tgtatttgaa ggattttgct 420
   gcggattttg agattagtga tttggtgagg ttgaagacag aggtggtgtt tgctggggtg 480
   ggtgaaggtg gaaaatggac ggtgagatct agatcagtgg agagagaatg tgtggatgag 540
   atttatgatg ctgttgttgt ttgcaatgga cattattttc aaccaagact tcccaatatt 600
   cctggcatta atgcatggcc agggaagcaa atgcatagcc ataattacag aacacccgag 660
   ccctttcaag atcaagttgt agttctaatt ggtggtgctg ccagtgcggt tgatatttct 720
   cgagacgtgg caaccgttgc taaagaagtt catattgcag ctaggtctgt tgaagaagat 780
   aagcttggaa agttacctgg ccatgataac atgtggcttc attctatgat tgacagtgtt 840
   catgaagatg gtgcagtggt ttttaaagat ggaaatgcag ttatcgctga cttcattgta 900
   cattgcacag ggtacaagta tgattttcct ttccttgaaa ccaacagcgt ggtgactgta 960
   gatgacaatc gtgttggacc actctacaag catgtttttc caccggcgtt agctccatgg 1020
   ctttcctttg ttgggttacc ttggaaggtt gctcccttcc ctttgtttga attgcagagt 1080
   aagtggatag ctggagtttt gtctaatcgc attgcccttc cttcagaaga ggagatgact 1140
   aaagatattg aagcttttta cttgtcactt gaagaatctg gcattcctaa gaggcacact 1200
   cataatatgg gcacgggcac ggccgatgtt cagtgggact acaataactg gcttgcagat 1260
   cagtgtggtg ttcctgctat ggaagaatgg agaaggcaaa tgtatatggc tacatcgaag 1320
   aacaggctct tgcgacctga gacttatcgt gatgagtggg acgatgatga cattgttcaa 1380
   ctagctgagc atgaatttgc taagtatcag atataatgtt gtattgtttt gagatttacc 1440
   aagtacaagt cattcatgcg ttatacgcct agttcagtgt tattcttaac gatcaaaaat 1500
   cagctttaaa gtgcaaataa gaatgtaaat tatatatgtt tggaatactt tcaataattc 1560
   attaatgaac atgtgataat gatgtgatct ctttttattt 1600
<210> 14
   <211> 471
   <212> PRT
   <213> Medicago truncatula
<400> 14
   Met Asn Glu His Val His Thr Val Ser Ile Gln Phe Asp Ser Lys Pro
   1 5 10 15
   Met Lys Phe Ile Met Ser Thr Ala Thr Pro Leu Leu Thr Pro Arg His
   20 25 30
   Val Ala Val Ile Gly Ala Gly Ala Gly Gly Leu Val Ala Ala Arg Glu
   35 40 45
   Leu Arg Arg Glu Gly His Gln Val Val Val Phe Glu Arg Gly Glu Glu
   50 55 60
   Leu Gly Gly Ser Trp Val Tyr Thr Ser Glu Val Glu Ser Asp Pro Leu
   65 70 75 80
   Gly Leu Asp Pro Asn Arg Lys Leu Ile His Ser Ser Leu Tyr Asn Ser
   85 90 95
   Leu Arg Thr Asn Leu Pro Arg Glu Ser Met Gly Phe Arg Asp Tyr Pro
   100 105 110
   Phe Arg Arg Lys Glu Glu Lys Gly Arg Asp Ser Arg Arg Phe Pro Ser
   115 120 125
   His Gly Glu Val Leu Met Tyr Leu Lys Asp Phe Ala Ala Asp Phe Glu
   130 135 140
   Ile Ser Asp Leu Val Arg Leu Lys Thr Glu Val Val Phe Ala Gly Val
   145 150 155 160
   Gly Glu Gly Gly Lys Trp Thr Val Arg Ser Arg Ser Val Glu Arg Glu
   165 170 175
   Cys Val Asp Glu Ile Tyr Asp Ala Val Val Val Cys Asn Gly His Tyr
   180 185 190
   Phe Gln Pro Arg Leu Pro Asn Ile Pro Gly Ile Asn Ala Trp Pro Gly
   195 200 205
   Lys Gln Met His Ser His Asn Tyr Arg Thr Pro Glu Pro Phe Gln Asp
   210 215 220
   Gln Val Val Val Leu Ile Gly Gly Ala Ala Ser Ala Val Asp Ile Ser
   225 230 235 240
   Arg Asp Val Ala Thr Val Ala Lys Glu Val His Ile Ala Ala Arg Ser
   245 250 255
   Val Glu Glu Asp Lys Leu Gly Lys Leu Pro Gly His Asp Asn Met Trp
               260                 265                 270
   Leu His Ser Met Ile Asp Ser Val His Glu Asp Gly Ala Val Val Phe
           275                 280                 285
   Lys Asp Gly Asn Ala Val Ile Ala Asp Phe Ile Val His Cys Thr Gly
       290                 295                 300
   Tyr Lys Tyr Asp Phe Pro Phe Leu Glu Thr Asn Ser Val Val Thr Val
   305                 310                 315                 320
   Asp Asp Asn Arg Val Gly Pro Leu Tyr Lys His Val Phe Pro Pro Ala
                   325                 330                 335
   Leu Ala Pro Trp Leu Ser Phe Val Gly Leu Pro Trp Lys Val Ala Pro
               340                 345                 350
   Phe Pro Leu Phe Glu Leu Gln Ser Lys Trp Ile Ala Gly Val Leu Ser
           355                 360                 365
   Asn Arg Ile Ala Leu Pro Ser Glu Glu Glu Met Thr Lys Asp Ile Glu
       370                 375                 380
   Ala Phe Tyr Leu Ser Leu Glu Glu Ser Gly Ile Pro Lys Arg His Thr
   385                 390                 395                 400
   His Asn Met Gly Thr Gly Thr Ala Asp Val Gln Trp Asp Tyr Asn Asn
                   405                 410                 415
   Trp Leu Ala Asp Gln Cys Gly Val Pro Ala Met Glu Glu Trp Arg Arg
               420                 425                 430
   Gln Met Tyr Met Ala Thr Ser Lys Asn Arg Leu Leu Arg Pro Glu Thr
           435                 440                 445
   Tyr Arg Asp Glu Trp Asp Asp Asp Asp Ile Val Gln Leu Ala Glu His
       450                 455                 460
   Glu Phe Ala Lys Tyr Gln Ile
   465                 470
<210> 15
   <211> 2747
   <212> DNA
   <213> Oryza sativa
<400> 15
   catgcctacg cagcctcatg tccagtcgag tgtaaccaca agcccacggg aatttgctgt 60
   ccaatgaaga ccccacaaaa cgacaaactc caataccaca cacctccgct tccctccaaa 120
   tcgcaacaga aatccgaagc gaaatcgcgc acgcaccgtc tcgcgatgcc gtccccgtcg 180
   ctccgcctcg ccgtcgtcgg cgcgggcgcc gccggcctgg tggcggcgcg ggagctccgc 240
   cgggagggcc actcccccgt ggtgttcgag cgcgccgcct ccgtgggcgg cacgtggctc 300
   tacgacgccg cccccgccac ctccgacccg ctcgccgccg gcgccgccca ctccagcctc 360
   tacgcctcgc tccgcaccaa cctgccgcgg gaggtgatgg gcttcctcga ctttcccttc 420
   gcctcctccg ccgcggaggc cggcggcggc ggcgacacgc gcaggttccc cggccacgac 480
   gaggtgctcc ggtatctgga ggagttcgcg cggcggttcg acctgtacgg cctcgtccgc 540
   ttcgggacgg aggtggttag ggttcggagg gatggcggcg gcggcggcgg gaggtgggcg 600
   gtgacgtcga ggaagatcgg ggagaagggg aggcgtgagg aggaggagga ggtgtatgat 660
   gccatcgtgg tttgcaatgg ccattacacg gagcctcgcg tcgcccacat acctggtaat 720
   ctcctcgtca ctagcaaagt tgcaatctaa tcaattttgc ttgaactact cctaatcatg 780
   gacagattca aggaattaaa taatttggta cttcgtactg ttgccaatgt atggttgcaa 840
   tgttgtgatc gtcgaatcca ggaaggttaa tttaactcca aattgaactc aaccgtttga 900
   agttttgaaa atagattggg attgatttca aatctgtatt tttttaacac tgttatgatg 960
   atcatcaaat ccaggaaggt taagtaaact caaaattaaa ctcaactgtt aggttgggat 1020
   tgacttcaaa tatgtattct ttaaacactg acaggggtgg aggcttggcc tggaaagcag 1080
   atgcatagcc acaattaccg cgttccagag cctttccacg atcaagtaac tgtctttctt 1140
   tacctgtgca atctttccta tcatgcattt gtgcttaaat gttatcttgg ttgatgcgtt 1200
   gcacttgtag gtagtgatca taatcggggc atcagcaagt gcagtagaca tctcaaggga 1260
   ccttgcaggt gttgcagaag aggttcatgt tgctgataga tcagcacctg cctgcacttg 1320
   caaaaggcag cctggatatg ataatatgtg gctccattcc atggtaaacg cccttttctc 1380
   gtggtgagtg atagcatatg gtagctttat ccgctgaaag ggctgccaca tttagcacaa 1440
   ctagaaaact aattttcaag ctgcagattg atcatgcaca agaagatggc tgcgtggtgt 1500
   ttcaggatgg cagctcaatc aaagccgatg tcatcatgca ctgtactggg tatgtaaacc 1560
   tgcactaccc tgcaacccat ttctcggctt cttgtgcgaa attgcatttt tgttactacc 1620
   tccgtttcag gttatgccta gattcattaa tatcaatatg aatatgagca atgctagaaa 1680
   gtcttataac ctaaaacgga ggaagtactt cagttgaaac taacaatgtg ttcctttcat 1740
   ctgcctgtcg actagctact tgtatgattt tccattcctt gaggatgata gcgccatcac 1800
   cgttgatgac aactgtgtcg atccactata caagcacgtt ttcccaccag aagtagcacc 1860
   tcacctgtcc ttcatcggat tgccatggaa ggtcattatg tgtgcaaaaa gtgatgccat 1920
   tcactttaga tgcacttgta attaagttgt cttgatcttg tgatgatcat aggatgtaaa 1980
   gcattcccct gccttgcttc ttgcaggtca ttccttttcc attgtttgaa ctccaaagca 2040
   aatgggttgc cggcgtgcta tcaggacgag tcaagcttcc ttcgagcgaa gaaatgatgg 2100
   aagatgtgaa agccttccac tcgaaaatgg aagcgcgtgg atggcctaag agatacgccc 2160
   acaacttttc agactgtcag gtagcctgga gatgctttga gtgtcagtta ccaaagttct 2220
   aatgttttga aacgaaatta ataaatatga ttgtcatcta cctgcaattt ttcagtagtt 2280
   tcgtttatgc tcccttgata agcttgtttt catttccagt ttgaatatga tgattggctt 2340
   gcggagcaat gtggccatcc accaattgaa caatggagga agctgatgta tgctgctaat 2400
   tcagagaaca aggctgctcg tccggagagt taccgcgatg agtgggacga tgatcatctt 2460
   gtggcagaag cagcagaaga tttcaagaaa tacttgtaaa atctcaagaa gatttcattc 2520
   aatgtacatg attgcaaatt tgcaatgcag aaaacatcag agaataattc tgtacaccca 2580
   aaatctcaat tcatgtctgg aatgggcaca aatgctcgtc atcagatagt tggtttactt 2640
   gtgtattatt tgatcatttg atgcctgtag attgtaataa taacctgaag caaaaacaag 2700
   agaataattc tgtgcatgag aaaggagaaa ccttgagtct ggaacgg 2747
<210> 16
   <211> 482
   <212> PRT
   <213> Oryza sativa
<400> 16
   Met Lys Thr Pro Gln Asn Asp Lys Leu Gln Tyr His Thr Pro Pro Leu
   1               5                   10                  15
   Pro Ser Lys Ser Gln Gln Lys Ser Glu Ala Lys Ser Arg Thr His Arg
               20                  25                  30
   Leu Ala Met Pro Ser Pro Ser Leu Arg Leu Ala Val Val Gly Ala Gly
           35                  40                  45
   Ala Ala Gly Leu Val Ala Ala Arg Glu Leu Arg Arg Glu Gly His Ser
       50                  55                  60
   Pro Val Val Phe Glu Arg Ala Ala Ser Val Gly Gly Thr Trp Leu Tyr
   65                  70                  75                  80
   Asp Ala Ala Pro Ala Thr Ser Asp Pro Leu Ala Ala Gly Ala Ala His
                   85                  90                  95
   Ser Ser Leu Tyr Ala Ser Leu Arg Thr Asn Leu Pro Arg Glu Val Met
               100                 105                 110
   Gly Phe Leu Asp Phe Pro Phe Ala Ser Ser Ala Ala Glu Ala Gly Gly
           115                 120                 125
   Gly Gly Asp Thr Arg Arg Phe Pro Gly His Asp Glu Val Leu Arg Tyr
       130                 135                 140
   Leu Glu Glu Phe Ala Arg Arg Phe Asp Leu Tyr Gly Leu Val Arg Phe
   145                 150                 155                 160
   Gly Thr Glu Val Val Arg Val Arg Arg Asp Gly Gly Gly Gly Gly Gly
                   165                 170                 175
   Arg Trp Ala Val Thr Ser Arg Lys Ile Gly Glu Lys Gly Arg Arg Glu
               180                 185                 190
   Glu Glu Glu Glu Val Tyr Asp Ala Ile Val Val Cys Asn Gly His Tyr
           195                 200                 205
   Thr Glu Pro Arg Val Ala His Ile Pro Gly Val Glu Ala Trp Pro Gly
       210                 215                 220
   Lys Gln Met His Ser His Asn Tyr Arg Val Pro Glu Pro Phe His Asp
   225                 230                 235                 240
   Gln Val Val Ile Ile Ile Gly Ala Ser Ala Ser Ala Val Asp Ile Ser
                   245                 250                 255
   Arg Asp Leu Ala Gly Val Ala Glu Glu Val His Val Ala Asp Arg Ser
               260                 265                 270
   Ala Pro Ala Cys Thr Cys Lys Arg Gln Pro Gly Tyr Asp Asn Met Trp
           275                 280                 285
   Leu His Ser Met Ile Asp His Ala Gln Glu Asp Gly Cys Val Val Phe
       290                 295                 300
   Gln Asp Gly Ser Ser Ile Lys Ala Asp Val Ile Met His Cys Thr Gly
   305                 310                 315                 320
   Tyr Leu Tyr Asp Phe Pro Phe Leu Glu Asp Asp Ser Ala Ile Thr Val
                   325                 330                 335
   Asp Asp Asn Cys Val Asp Pro Leu Tyr Lys His Val Phe Pro Pro Glu
               340                 345                 350
   Val Ala Pro His Leu Ser Phe Ile Gly Leu Pro Trp Lys Val Ile Pro
           355                 360                 365
   Phe Pro Leu Phe Glu Leu Gln Ser Lys Trp Val Ala Gly Val Leu Ser
   370 375 380
   Gly Arg Val Lys Leu Pro Ser Ser Glu Glu Met Met Glu Asp Val Lys
   385 390 395 400
   Ala Phe His Ser Lys Met Glu Ala Arg Gly Trp Pro Lys Arg Tyr Ala
   405 410 415
   His Asn Phe Ser Asp Cys Gln Phe Glu Tyr Asp Asp Trp Leu Ala Glu
   420 425 430
   Gln Cys Gly His Pro Pro Ile Glu Gln Trp Arg Lys Leu Met Tyr Ala
   435 440 445
   Ala Asn Ser Glu Asn Lys Ala Ala Arg Pro Glu Ser Tyr Arg Asp Glu
   450 455 460
   Trp Asp Asp Asp His Leu Val Ala Glu Ala Ala Glu Asp Phe Lys Lys
   465 470 475 480
   Tyr Leu
<210> 17
   <211> 1552
   <212> DNA
   <213> Vitis vinifera
<400> 17
   ctctactatc ttcccatggc gccctccatc tccctgttca aatcacgtga cgttgccgtc 60
   atcggggctg gcgctgccgg tttagtcgcc gcccgtgagc tccgccgtga aggccacaag 120
   gtcgttgtct tcgagcggga acgccaagtg ggtgggacct gggtctacac gcccacagtg 180
   gagacggatc cacttggctc cgacccgtct cgacacatag tccactccag cctctacgcc 240
   tccctccgca ccaacctccc tagagaggtc atgggttttc tggactaccc cttcgtatcc 300
   actggtgaac cacataggga ccccagaagg tttccgggtc accgagaggt ctcgctttat 360
   ctcaaggatt ttgcggttgg gtttggactc aatgaattaa tccgcttcga gacggaggta 420
   gtttatgctg gtttggtcga ggatgagaag tggagggtga agtctagaag cggaaacgat 480
   gcggcaattg atgtggagga gatttttgat gctgtggttg tttgcaatgg ccattacaca 540
   gagccccgtc ttgcagaaat tcctggcatt gatgcatggc caggaaagca tatgcatagt 600
   cacaattatc gtattcctga gccctttcga gatcaggttg tagttttgat agggggtgct 660
   gcaagtgctg tcgacatctc tatggacatt gctcaagttg ctaaagcagt tcatattgca 720
   tctagatcag ttgaggctgg aatcttgaaa aagttatctg gcaatgccat tgataacatg      780
   tggcttcatc ctatgataga aagtgtccag aaagatggta ctgtgatatt ttatgatggg      840
   agtgtggttc ttgctgatgt aattctgcac tgcacgggat acaagtatca tttccctttt      900
   cttgacacca gtggaattgt gactgtggat gacaatcgtg tgggacctct atacaagcat      960
   atttttccac cacatttggc tccagggctt tcctttgttg gtttgccatg gaaggtcctc     1020
   cctttcccca tgtttgaatt ccaaagcaaa tggatagcag gtgctctctc aggtcggatt     1080
   ggactcccat cgcaggagga gatgatggca gatgtttcag ccttttattt gtcactagaa     1140
   gcttctgaca caccaaagca ctacactcac aacttggctg attctcaggt aaatttgaac     1200
   tcttatataa gtgggttagg atactgtcat gttcattttt cttactggtt atctctcaaa     1260
   gtaatgttga aactgttctt ggatggcatt ttgcagtttg agtatgatga ttggcttgcc     1320
   ttggaatgcg ggattccagg cgttgaagaa tggagaaaga aaatgtatga agcaactgcc     1380
   aagaacaaga aggtccgacc agacaaatac cgcgacaaat gggaagatga agacttaatg     1440
   ttggaagctc agaaggactt cgctggatgc cgcctgaatg gggctggtga caattgaaac     1500
   caacctcccc tacaaaataa gcaatgaaaa aaaaaaaaaa gcccgataaa ga             1552
<210> 18
   <211> 493
   <212> PRT
   <213> Vitis vinifera
<400> 18
   Met Ala Pro Ser Ile Ser Leu Phe Lys Ser Arg Asp Val Ala Val Ile
   1               5                   10                  15
   Gly Ala Gly Ala Ala Gly Leu Val Ala Ala Arg Glu Leu Arg Arg Glu
               20                  25                  30
   Gly His Lys Val Val Val Phe Glu Arg Glu Arg Gln Val Gly Gly Thr
           35                  40                  45
   Trp Val Tyr Thr Pro Thr Val Glu Thr Asp Pro Leu Gly Ser Asp Pro
       50                  55                  60
   Ser Arg His Ile Val His Ser Ser Leu Tyr Ala Ser Leu Arg Thr Asn
   65                  70                  75                  80
   Leu Pro Arg Glu Val Met Gly Phe Leu Asp Tyr Pro Phe Val Ser Thr
                   85                  90                  95
   Gly Glu Pro His Arg Asp Pro Arg Arg Phe Pro Gly His Arg Glu Val
               100                 105                 110
   Ser Leu Tyr Leu Lys Asp Phe Ala Val Gly Phe Gly Leu Asn Glu Leu
           115                 120                 125
   Ile Arg Phe Glu Thr Glu Val Val Tyr Ala Gly Leu Val Glu Asp Glu
       130                 135                 140
   Lys Trp Arg Val Lys Ser Arg Ser Gly Asn Asp Ala Ala Ile Asp Val
   145                 150                 155                 160
   Glu Glu Ile Phe Asp Ala Val Val Val Cys Asn Gly His Tyr Thr Glu
                   165                 170                 175
   Pro Arg Leu Ala Glu Ile Pro Gly Ile Asp Ala Trp Pro Gly Lys His
               180                 185                 190
   Met His Ser His Asn Tyr Arg Ile Pro Glu Pro Phe Arg Asp Gln Val
           195                 200                 205
   Val Val Leu Ile Gly Gly Ala Ala Ser Ala Val Asp Ile Ser Met Asp
       210                 215                 220
   Ile Ala Gln Val Ala Lys Ala Val His Ile Ala Ser Arg Ser Val Glu
   225                 230                 235                 240
   Ala Gly Ile Leu Lys Lys Leu Ser Gly Asn Ala Ile Asp Asn Met Trp
                   245                 250                 255
   Leu His Pro Met Ile Glu Ser Val Gln Lys Asp Gly Thr Val Ile Phe
               260                 265                 270
   Tyr Asp Gly Ser Val Val Leu Ala Asp Val Ile Leu His Cys Thr Gly
           275                 280                 285
   Tyr Lys Tyr His Phe Pro Phe Leu Asp Thr Ser Gly Ile Val Thr Val
       290                 295                 300
   Asp Asp Asn Arg Val Gly Pro Leu Tyr Lys His Ile Phe Pro Pro His
   305                 310                 315                 320
   Leu Ala Pro Gly Leu Ser Phe Val Gly Leu Pro Trp Lys Val Leu Pro
                   325                 330                 335
   Phe Pro Met Phe Glu Phe Gln Ser Lys Trp Ile Ala Gly Ala Leu Ser
               340                 345                 350
   Gly Arg Ile Gly Leu Pro Ser Gln Glu Glu Met Met Ala Asp Val Ser
   355 360 365
   Ala Phe Tyr Leu Ser Leu Glu Ala Ser Asp Thr Pro Lys His Tyr Thr
   370 375 380
   His Asn Leu Ala Asp Ser Gln Val Asn Leu Asn Ser Tyr Ile Ser Gly
   385 390 395 400
   Leu Gly Tyr Cys His Val His Phe Ser Tyr Trp Leu Ser Leu Lys Val
   405 410 415
   Met Leu Lys Leu Phe Leu Asp Gly Ile Leu Gln Phe Glu Tyr Asp Asp
   420 425 430
   Trp Leu Ala Leu Glu Cys Gly Ile Pro Gly Val Glu Glu Trp Arg Lys
   435 440 445
   Lys Met Tyr Glu Ala Thr Ala Lys Asn Lys Lys Val Arg Pro Asp Lys
   450 455 460
   Tyr Arg Asp Lys Trp Glu Asp Glu Asp Leu Met Leu Glu Ala Gln Lys
   465 470 475 480
   Asp Phe Ala Gly Cys Arg Leu Asn Gly Ala Gly Asp Asn
   485 490
<210> 19
   <211> 1444
   <212> DNA
   <213> Vitis vinifera
<400> 19
   ctctactatc ttcccatggc gccctccatc tccctgttca aatcacgtga cgttgccgtc 60
   atcggggctg gcgctgccgg tttagtcgcc gcccgtgagc tccgccgtga aggccacaag 120
   gtcgttgtct tcgagcggga acgccaagtg ggtgggacct gggtctacac gcccacagtg 180
   gagacggatc cacttggctc cgacccgtct cgacacatag tccactccag cctctacgcc 240
   tccctccgca ccaacctccc tagagaggtc atgggttttc tggactaccc cttcgtatcc 300
   actggtgaac cacataggga ccccagaagg tttccgggtc accgagaggt ctcgctttat 360
   ctcaaggatt ttgcggttgg gtttggactc aatgaattaa tccgcttcga gacggaggta 420
   gtttatgctg gtttggtcga ggatgagaag tggagggtga agtctagaag cggaaacgat 480
   gcggcaattg atgtggagga gatttttgat gctgtggttg tttgcaatgg ccattacaca 540
   gagccccgtc ttgcagaaat tcctggcatt gatgcatggc caggaaagca tatgcatagt 600
   cacaattatc gtattcctga gccctttcga gatcaggttg tagttttgat agggggtgct 660
   gcaagtgctg tcgacatctc tatggacatt gctcaagttg ctaaagcagt tcatattgca 720
   tctagatcag ttgaggctgg aatcttgaaa aagttatctg gcaatgccat tgataacatg 780
   tggcttcatc ctatgataga aagtgtccag aaagatggta ctgtgatatt ttatgatggg 840
   agtgtggttc ttgctgatgt aattctgcac tgcacgggat acaagtatca tttccctttt 900
   cttgacacca gtggaattgt gactgtggat gacaatcgtg tgggacctct atacaagcat 960
   atttttccac cacatttggc tccagggctt tcctttgttg gtttgccatg gaaggtcctc 1020
   cctttcccca tgtttgaatt ccaaagcaaa tggatagcag gtgctctctc aggtcggatt 1080
   ggactcccat cgcaggagga gatgatggca gatgtttcag ccttttattt gtcactagaa 1140
   gcttctgaca caccaaagca ctacactcac aacttggctg attctcagtt tgagtatgat 1200
   gattggcttg ccttggaatg cgggattcca ggcgttgaag aatggagaaa gaaaatgtat 1260
   gaagcaactg ccaagaacaa gaaggtccga ccagacaaat accgcgacaa atgggaagat 1320
   gaagacttaa tgttggaagc tcagaaggac ttcgctggat gccgcctgaa tggggctggt 1380
   gacaattgaa accaacctcc cctacaaaat aagcaatgaa aaaaaaaaaa aagcccgata 1440
   aaga 1444
<210> 20
   <211> 457
   <212> PRT
   <213> Vitis vinifera
<400> 20
   Met Ala Pro Ser Ile Ser Leu Phe Lys Ser Arg Asp Val Ala Val Ile
   1               5                   10                  15
   Gly Ala Gly Ala Ala Gly Leu Val Ala Ala Arg Glu Leu Arg Arg Glu
               20                  25                  30
   Gly His Lys Val Val Val Phe Glu Arg Glu Arg Gln Val Gly Gly Thr
           35                  40                  45
   Trp Val Tyr Thr Pro Thr Val Glu Thr Asp Pro Leu Gly Ser Asp Pro
       50                  55                  60
   Ser Arg His Ile Val His Ser Ser Leu Tyr Ala Ser Leu Arg Thr Asn
   65                  70                  75                  80
   Leu Pro Arg Glu Val Met Gly Phe Leu Asp Tyr Pro Phe Val Ser Thr
                   85                  90                  95
   Gly Glu Pro His Arg Asp Pro Arg Arg Phe Pro Gly His Arg Glu Val
               100                 105                 110
   Ser Leu Tyr Leu Lys Asp Phe Ala Val Gly Phe Gly Leu Asn Glu Leu
           115                 120                 125
   Ile Arg Phe Glu Thr Glu Val Val Tyr Ala Gly Leu Val Glu Asp Glu
       130                 135                 140
   Lys Trp Arg Val Lys Ser Arg Ser Gly Asn Asp Ala Ala Ile Asp Val
   145                 150                 155                 160
   Glu Glu Ile Phe Asp Ala Val Val Val Cys Asn Gly His Tyr Thr Glu
                   165                 170                 175
   Pro Arg Leu Ala Glu Ile Pro Gly Ile Asp Ala Trp Pro Gly Lys His
               180                 185                 190
   Met His Ser His Asn Tyr Arg Ile Pro Glu Pro Phe Arg Asp Gln Val
           195                 200                 205
   Val Val Leu Ile Gly Gly Ala Ala Ser Ala Val Asp Ile Ser Met Asp
       210                 215                 220
   Ile Ala Gln Val Ala Lys Ala Val His Ile Ala Ser Arg Ser Val Glu
   225                 230                 235                 240
   Ala Gly Ile Leu Lys Lys Leu Ser Gly Asn Ala Ile Asp Asn Met Trp
                   245                 250                 255
   Leu His Pro Met Ile Glu Ser Val Gln Lys Asp Gly Thr Val Ile Phe
               260                 265                 270
   Tyr Asp Gly Ser Val Val Leu Ala Asp Val Ile Leu His Cys Thr Gly
           275                 280                 285
   Tyr Lys Tyr His Phe Pro Phe Leu Asp Thr Ser Gly Ile Val Thr Val
       290                 295                 300
   Asp Asp Asn Arg Val Gly Pro Leu Tyr Lys His Ile Phe Pro Pro His
   305                 310                 315                 320
   Leu Ala Pro Gly Leu Ser Phe Val Gly Leu Pro Trp Lys Val Leu Pro
                   325                 330                 335
   Phe Pro Met Phe Glu Phe Gln Ser Lys Trp Ile Ala Gly Ala Leu Ser
               340                 345                 350
   Gly Arg Ile Gly Leu Pro Ser Gln Glu Glu Met Met Ala Asp Val Ser
           355                 360                 365
   Ala Phe Tyr Leu Ser Leu Glu Ala Ser Asp Thr Pro Lys His Tyr Thr
       370                 375                 380
   His Asn Leu Ala Asp Ser Gln Phe Glu Tyr Asp Asp Trp Leu Ala Leu
   385                 390                 395                 400
   Glu Cys Gly Ile Pro Gly Val Glu Glu Trp Arg Lys Lys Met Tyr Glu
                   405                 410                 415
   Ala Thr Ala Lys Asn Lys Lys Val Arg Pro Asp Lys Tyr Arg Asp Lys
               420                 425                 430
   Trp Glu Asp Glu Asp Leu Met Leu Glu Ala Gln Lys Asp Phe Ala Gly
           435                 440                 445
   Cys Arg Leu Asn Gly Ala Gly Asp Asn
       450                 455
<210> 21
   <211> 1674
   <212> DNA
   <213> Vitis vinifera
<400> 21
   ctccactttc ttcccatggc gccctccatc tccctgttca aatcacgtga cgttgccgtc 60
   atcggggctg gcgctgccgg tttagttgcc gcccgtgagc tccgccgtga aggccacaag 120
   gtcgttgtct tcgagcggga acgccaagtg ggtgggacct gggtctacac gcccacagtg 180
   gagacggatc cacttggcgc cgacccgtct cgacacatag tccactccag cctctacgcc 240
   tccctccgca ccaacctccc cagagaggtc atgggttttc tggactaccc cttcgtatcc 300
   actggtgaac ctcataggga ccccagaagg tttccgggtc accgagaggt ctcgctttat 360
   ctcaaggatt ttgtggttgg gtttggactc aatgaattaa tccgcttcga gacggaggtg 420
   gtttatgctg gtttggttga ggatgagaag tggggagtga agtctagaag cggaaacgat 480
   gcggcaattg atgtggagga gatttttgat gctgtggttg tttgcaatgg ccattacaca 540
   gagccccgtc ttgcagaaat tcctggcatt gatgcatggc caggaaagca tatgcatagt 600
   cacaattatc gtactcctga gccctttcga gatcaggttg tagttttgat agggagtgct 660
   gcaagtgctg ttgacatctc tatggacatt gctcaagttg ctaaagcagt tcatattgca 720
   tctagatcag ttgaggctgg aatcttggaa aagttatctg gcaatgctgt tgataacatg 780
   tggcttcatc ctatgataga aagtgtccag aaagatggta ctgtgatatt ttatgatggg 840
   agtgtggttc ttgctgatgt aattctgcac tgcacgggat acaagtatca tttccctttt 900
   cttgacacca gtggaattgt gactgtggat gacaatcgtg tgggacctct atacaagcat 960
   atttttccac cacatttggc tccagggctt tcctttgttg gtctgctatg gaaggtcctc 1020
   cctttcccca tgtttgaatt ccaaagcaaa tggatagcag gtgctctctc aggtcggatt 1080
   ggactcccat cgcaggagga gatgatggca gatgtttcag ccttttattt gtcacgagaa 1140
   gcttctgaca caccaaagca ctacactcac aacttggctg attctcaggt aaatttgagc 1200
   tcttatataa gtgggttagg atactgtcat tttcattttt cttactggtt atctctcaaa 1260
   gtaatgttga aactgttctt ggatgctatt ttgcagtttg agtatgatga ttggcttgcc 1320
   ttggaatgcg ggattccagg cgttgaagaa tggagaaaga aaatgtatca agcaactgct 1380
   aagaataaga aggtccgacc agacaaatac cgcgacgaat gggaagatga agacttaacg 1440
   ttggaagctc agaaggactt cgccagatgc cgcccgaatg ggggtggcga caattgaaac 1500
   caacctcccc tacaaaataa gcaatgaaaa aaaaaaaaaa gcccgataaa gatggatctg 1560
   gatattgtct tggttgagtc attcattgtt cttctcttga gacttgaggt atattaattg 1620
   aataatcagc catagcgtag ataatttttt tttttatagc gattgttttg atcg 1674
<210> 22
   <211> 457
   <212> PRT
   <213> Vitis vinifera
<400> 22
   Met Ala Pro Ser Ile Ser Leu Phe Lys Ser Arg Asp Val Ala Val Ile
   1               5                   10                  15
   Gly Ala Gly Ala Ala Gly Leu Val Ala Ala Arg Glu Leu Arg Arg Glu
               20                  25                  30
   Gly His Lys Val Val Val Phe Glu Arg Glu Arg Gln Val Gly Gly Thr
           35                  40                  45
   Trp Val Tyr Thr Pro Thr Val Glu Thr Asp Pro Leu Gly Ala Asp Pro
       50                  55                  60
   Ser Arg His Ile Val His Ser Ser Leu Tyr Ala Ser Leu Arg Thr Asn
   65                  70                  75                  80
   Leu Pro Arg Glu Val Met Gly Phe Leu Asp Tyr Pro Phe Val Ser Thr
                   85                  90                  95
   Gly Glu Pro His Arg Asp Pro Arg Arg Phe Pro Gly His Arg Glu Val
               100                 105                 110
   Ser Leu Tyr Leu Lys Asp Phe Val Val Gly Phe Gly Leu Asn Glu Leu
           115                 120                 125
   Ile Arg Phe Glu Thr Glu Val Val Tyr Ala Gly Leu Val Glu Asp Glu
       130                 135                 140
   Lys Trp Gly Val Lys Ser Arg Ser Gly Asn Asp Ala Ala Ile Asp Val
   145                 150                 155                 160
   Glu Glu Ile Phe Asp Ala Val Val Val Cys Asn Gly His Tyr Thr Glu
                   165                 170                 175
   Pro Arg Leu Ala Glu Ile Pro Gly Ile Asp Ala Trp Pro Gly Lys His
               180                 185                 190
   Met His Ser His Asn Tyr Arg Thr Pro Glu Pro Phe Arg Asp Gln Val
           195                 200                 205
   Val Val Leu Ile Gly Ser Ala Ala Ser Ala Val Asp Ile Ser Met Asp
       210                 215                 220
   Ile Ala Gln Val Ala Lys Ala Val His Ile Ala Ser Arg Ser Val Glu
   225                 230                 235                 240
   Ala Gly Ile Leu Glu Lys Leu Ser Gly Asn Ala Val Asp Asn Met Trp
                   245                 250                 255
   Leu His Pro Met Ile Glu Ser Val Gln Lys Asp Gly Thr Val Ile Phe
               260                 265                 270
   Tyr Asp Gly Ser Val Val Leu Ala Asp Val Ile Leu His Cys Thr Gly
           275                 280                 285
   Tyr Lys Tyr His Phe Pro Phe Leu Asp Thr Ser Gly Ile Val Thr Val
       290                 295                 300
   Asp Asp Asn Arg Val Gly Pro Leu Tyr Lys His Ile Phe Pro Pro His
   305                 310                 315                 320
   Leu Ala Pro Gly Leu Ser Phe Val Gly Leu Leu Trp Lys Val Leu Pro
                   325                 330                 335
   Phe Pro Met Phe Glu Phe Gln Ser Lys Trp Ile Ala Gly Ala Leu Ser
               340                 345                 350
   Gly Arg Ile Gly Leu Pro Ser Gln Glu Glu Met Met Ala Asp Val Ser
           355                 360                 365
   Ala Phe Tyr Leu Ser Arg Glu Ala Ser Asp Thr Pro Lys His Tyr Thr
   370 375 380
   His Asn Leu Ala Asp Ser Gln Phe Glu Tyr Asp Asp Trp Leu Ala Leu
   385 390 395 400
   Glu Cys Gly Ile Pro Gly Val Glu Glu Trp Arg Lys Lys Met Tyr Gln
   405 410 415
   Ala Thr Ala Lys Asn Lys Lys Val Arg Pro Asp Lys Tyr Arg Asp Glu
   420 425 430
   Trp Glu Asp Glu Asp Leu Thr Leu Glu Ala Gln Lys Asp Phe Ala Arg
   435 440 445
   Cys Arg Pro Asn Gly Gly Gly Asp Asn
   450 455
<210> 23
   <211> 887
   <212> DNA
   <213> Gossypium hirsutum
<400> 23
   ctagatcagt ggcggatgaa acgtatatga aacagcctgg ttacgataat ttgtggttcc 60
   attccatgat agatcatgca catgaggatg gcatggtggt tttccgaaat gggaaaacag 120
   tgcttgctga tctcattatg cactgcactg ggtacaagta tcacttccct ttccttgaca 180
   caaaaggcat tgtgactgtg gacgataatc gtcttggacc actatacaag cacgtctttc 240
   ccccagcctt agccccatac ctttcattta ttgggatacc atggaagatt gttcctttcc 300
   ccttatttga gtttcaaagc aaatggatag ccggtatttt gtccggtcgt attacacttc 360
   catcacaaaa ggaaatgatg gaagatattc aagcatttta ctcggcactt gaagattcta 420
   gtataccaaa acggtatact cattgcattg gtcaatctca ggttgaatac aataattggc 480
   ttgctacaca atgtggttgc caaggtgttg aaaaatggag agaagcaatg tattctatgg 540
   cttcggagaa tcggcgtctt ctaccagaga tgtaccgtga tgaatgggat gatcaccacc 600
   tggtttcaga agcttatgag gatttcatta agtacccttc agcatcaaac ctttagagac 660
   aaaaaataaa aataaaaatc aatttacaat ggtgaaggat gtcattcacc ctgttgtata 720
   caacccgggt ctgtaccgtg gcagggtact attctaccac tagaccactg gtgcttgtgc 780
   gatgaaagtc tcgatataca taaatctagc acaagagtta ctaaacgaag agaattgaag 840
   gagaatcaac tatatgaatt ttattgaata aaaaaaaaaa aaaaaaa 887
<210> 24
   <211> 217
   <212> PRT
   <213> Gossypium hirsutum
<400> 24
   Arg Ser Val Ala Asp Glu Thr Tyr Met Lys Gln Pro Gly Tyr Asp Asn
   1               5                   10                  15
   Leu Trp Phe His Ser Met Ile Asp His Ala His Glu Asp Gly Met Val
               20                  25                  30
   Val Phe Arg Asn Gly Lys Thr Val Leu Ala Asp Leu Ile Met His Cys
           35                  40                  45
   Thr Gly Tyr Lys Tyr His Phe Pro Phe Leu Asp Thr Lys Gly Ile Val
       50                  55                  60
   Thr Val Asp Asp Asn Arg Leu Gly Pro Leu Tyr Lys His Val Phe Pro
   65                  70                  75                  80
   Pro Ala Leu Ala Pro Tyr Leu Ser Phe Ile Gly Ile Pro Trp Lys Ile
                   85                  90                  95
   Val Pro Phe Pro Leu Phe Glu Phe Gln Ser Lys Trp Ile Ala Gly Ile
               100                 105                 110
   Leu Ser Gly Arg Ile Thr Leu Pro Ser Gln Lys Glu Met Met Glu Asp
           115                 120                 125
   Ile Gln Ala Phe Tyr Ser Ala Leu Glu Asp Ser Ser Ile Pro Lys Arg
       130                 135                 140
   Tyr Thr His Cys Ile Gly Gln Ser Gln Val Glu Tyr Asn Asn Trp Leu
   145                 150                 155                 160
   Ala Thr Gln Cys Gly Cys Gln Gly Val Glu Lys Trp Arg Glu Ala Met
                   165                 170                 175
   Tyr Ser Met Ala Ser Glu Asn Arg Arg Leu Leu Pro Glu Met Tyr Arg
               180                 185                 190
   Asp Glu Trp Asp Asp His His Leu Val Ser Glu Ala Tyr Glu Asp Phe
           195                 200                 205
   Ile Lys Tyr Pro Ser Ala Ser Asn Leu
       210                 215
<210> 25
   <211> 1262
   <212> DNA
   <213> Zea mays
<400> 25
   atggaatgtt gttgctgggc tggctacagt acagtacagg tgcaggcttc attcctccag 60
   tggcggcgga caacgccgaa cagacagaag aatactgtag agaagatcgt cgcacccacc 120
   ggaggacgag acgggcccta ggcacaccac gcaatcagcc gccccgcgcc cccgcccgcg 180
   gttggcgatg ttgccgtgac gtcttccagg gaggagaagc caacatcaca aactccacga 240
   cgaataactg gcagtagaag ccgagaaggt aggcccgctc gttccaacat cacaaactcc 300
   acacggctct cctgtctccg ctgcccgctc cacctccctc catgccgtcg gcttccctcc 360
   gcctcgccgt cgtcggcgcg ggcgcggcgg gcctggttgc cgcccgcgag ctacgccgcg 420
   agggccatgc gcccgtcgtc ttcgagcgcg ccgccgccgt tgggggcact tggctctaca 480
   cgcctcccgc cacgtcctcc gacccgctcg gcgccgcggc gacgcattcc agcctctacg 540
   catcgctccg caccaacctg ccacgcgaga ccatgggctt cctcgacttc cccttcgccg 600
   ctggcgccgc gggctcccga gacccccgcc ggtttcccgg gcacgaggag gtgctccgct 660
   acctggaggc gttcgcgcgc cggttcgacc tgctccggct cgtccgcttc gagacggagg 720
   tgctcagtgt gaggagggaa gacggaggga ggtgggctgt gacgtcgagg aagctcgggg 780
   ataaggggag cggcgaggag gagttctatg atgccgtcgt ggtctgcaat ggtcactaca 840
   cggagccacg cctcgccgtc attcccgttt gagtatgatg attggctcgc tgagcaatgt 900
   ggccatccac cagtcgaaga atggaggaag cagatgtatg ctgtaacttc aatgaacaag 960
   gcagctcgtc ctgagagtta ccgtgatgaa tgggatgacg agcatctggt ggccgaagca 1020
   aatgaatact tcaagaaatt cttgtaaatt ctttcttact attctcatcc catattcttt 1080
   cggcataccc gaggctgatc tcaactgcaa tatgcaaata tgaataacca tttaagtgat 1140
   gtggattgga tacacttctg ggttagcatt tcatcgatca ttcgatcgat gtatatatat 1200
   gagactgttc tggtagtaaa caatcttgta gtaaactgtg gattggtcat caattaacaa 1260
   ca 1262
<210> 26
   <211> 176
   <212> PRT
   <213> Zea mays
<400> 26
   Met Pro Ser Ala Ser Leu Arg Leu Ala Val Val Gly Ala Gly Ala Ala
   1               5                   10                  15
   Gly Leu Val Ala Ala Arg Glu Leu Arg Arg Glu Gly His Ala Pro Val
               20                  25                  30
   Val Phe Glu Arg Ala Ala Ala Val Gly Gly Thr Trp Leu Tyr Thr Pro
           35                  40                  45
   Pro Ala Thr Ser Ser Asp Pro Leu Gly Ala Ala Ala Thr His Ser Ser
       50                  55                  60
   Leu Tyr Ala Ser Leu Arg Thr Asn Leu Pro Arg Glu Thr Met Gly Phe
   65                  70                  75                  80
   Leu Asp Phe Pro Phe Ala Ala Gly Ala Ala Gly Ser Arg Asp Pro Arg
                   85                  90                  95
   Arg Phe Pro Gly His Glu Glu Val Leu Arg Tyr Leu Glu Ala Phe Ala
               100                 105                 110
   Arg Arg Phe Asp Leu Leu Arg Leu Val Arg Phe Glu Thr Glu Val Leu
           115                 120                 125
   Ser Val Arg Arg Glu Asp Gly Gly Arg Trp Ala Val Thr Ser Arg Lys
       130                 135                 140
   Leu Gly Asp Lys Gly Ser Gly Glu Glu Glu Phe Tyr Asp Ala Val Val
   145                 150                 155                 160
   Val Cys Asn Gly His Tyr Thr Glu Pro Arg Leu Ala Val Ile Pro Val
                   165                 170                 175
<210> 27
   <211> 1359
   <212> DNA
   <213> Populus trichocarpa
<400> 27
   atgcaaacat caaatgctac ttcgctcacc tctcgccacg tagctgtcat cggtgccggc      60
   gccgccggtt tagtggcggc acgtgagctc cggcgtgaag gtcaccaagt ggttgtcttt     120
   gagaaagata gccaaattgg tgggacatgg gtgtacactc cacaggtcga aaccgaccct     180
   cttgggctag acccgacccg acacatcgtc cacaccagtt tatacaagtc cctccggacc     240
   aacttgccga gagagtcgat gggctttatg gattatccat tcgtgacccg agcgggtgaa     300
   gggagcgacc ctagaaggtt cccgggtcat gcagaagtgt tgaagtatct gcaagatttt     360
   gcaagggagt ttgggattga agaaatggtg aggtttgagt gtgaagtggt tagtgtggag     420
   atggttgata atgagaaatt gaaagtgaag tgtaaaagga tgagacctga tggtggtgat     480
   gatgatctgc tagatgaggt ttttgatgct gttgttgttt gtaatggaca tttcacatac      540
   cctcgtattg ctgaaatccc tggcatcaac ttgtggcccg gaatgcaaat acatagccat     600
   aactatcgta ctcctgaact cttcaaggat aaagttgtaa ttttaattgg cagttctgca     660
   agtgctattg atttatccct tgagattggt ggaattgcca aagaggtgca cattgcatct     720
   agatcagttg ccaatgatac atatgaaaag cgggctgaat gtgataatat atggctacat     780
   tctatgataa aaagcgcaca taaagatggt tctgtggctt tccgagatgg taacactatc     840
   gtcgctgata ttattctgca ttgcacaggg tacaagtatt acttcccatt cctcaaaacc     900
   aatggcattg tgactgtgga tgacaatcgt gttggaccac tctacaagca tgttttccca     960
   cccatttttg ccccgcagct ttcctttgtc ggactaccct acaggagttt acctttccca     1020
   atctttgaaa ttcaaagcaa gtggatttct ggtgttctat ctgatcgaat tgtgctccct    1080
   tcacaagagg acatgatgga agatgttaac accttctact cgacacttga agattctggt    1140
   gtgcctaagc atcacactca tagcatgggg gacacaatga ttgactacaa tgcttgggtt    1200
   gcttctctgt gtcaatgtcc ttgctttgaa gaatggagag tacaaatgtt ctatgaaacg    1260
   gccaagagat tgaacgccaa cccaaagaca tttcgcgatg aatgggaaga tgacaacctg    1320
   gtcttgcaag cctgtgaaga tttcagcaaa tacatctga                           1359
<210> 28
   <211> 452
   <212> PRT
   <213> Populus trichocarpa
<400> 28
   Met Gln Thr Ser Asn Ala Thr Ser Leu Thr Ser Arg His Val Ala Val
   1               5                   10                  15
   Ile Gly Ala Gly Ala Ala Gly Leu Val Ala Ala Arg Glu Leu Arg Arg
               20                  25                  30
   Glu Gly His Gln Val Val Val Phe Glu Lys Asp Ser Gln Ile Gly Gly
           35                  40                  45
   Thr Trp Val Tyr Thr Pro Gln Val Glu Thr Asp Pro Leu Gly Leu Asp
       50                  55                  60
   Pro Thr Arg His Ile Val His Thr Ser Leu Tyr Lys Ser Leu Arg Thr
   65                  70                  75                  80
   Asn Leu Pro Arg Glu Ser Met Gly Phe Met Asp Tyr Pro Phe Val Thr
                   85                  90                  95
   Arg Ala Gly Glu Gly Ser Asp Pro Arg Arg Phe Pro Gly His Ala Glu
               100                 105                 110
   Val Leu Lys Tyr Leu Gln Asp Phe Ala Arg Glu Phe Gly Ile Glu Glu
           115                 120                 125
   Met Val Arg Phe Glu Cys Glu Val Val Ser Val Glu Met Val Asp Asn
       130                 135                 140
   Glu Lys Leu Lys Val Lys Cys Lys Arg Met Arg Pro Asp Gly Gly Asp
   145                 150                 155                 160
   Asp Asp Leu Leu Asp Glu Val Phe Asp Ala Val Val Val Cys Asn Gly
                   165                 170                 175
   His Phe Thr Tyr Pro Arg Ile Ala Glu Ile Pro Gly Ile Asn Leu Trp
               180                 185                 190
   Pro Gly Met Gln Ile His Ser His Asn Tyr Arg Thr Pro Glu Leu Phe
           195                 200                 205
   Lys Asp Lys Val Val Ile Leu Ile Gly Ser Ser Ala Ser Ala Ile Asp
       210                 215                 220
   Leu Ser Leu Glu Ile Gly Gly Ile Ala Lys Glu Val His Ile Ala Ser
   225                 230                 235                 240
   Arg Ser Val Ala Asn Asp Thr Tyr Glu Lys Arg Ala Glu Cys Asp Asn
                   245                 250                 255
   Ile Trp Leu His Ser Met Ile Lys Ser Ala His Lys Asp Gly Ser Val
               260                 265                 270
   Ala Phe Arg Asp Gly Asn Thr Ile Val Ala Asp Ile Ile Leu His Cys
           275                 280                 285
   Thr Gly Tyr Lys Tyr Tyr Phe Pro Phe Leu Lys Thr Asn Gly Ile Val
       290                 295                 300
   Thr Val Asp Asp Asn Arg Val Gly Pro Leu Tyr Lys His Val Phe Pro
   305                 310                 315                 320
   Pro Ile Phe Ala Pro Gln Leu Ser Phe Val Gly Leu Pro Tyr Arg Ser
                   325                 330                 335
   Leu Pro Phe Pro Ile Phe Glu Ile Gln Ser Lys Trp Ile Ser Gly Val
               340                 345                 350
   Leu Ser Asp Arg Ile Val Leu Pro Ser Gln Glu Asp Met Met Glu Asp
           355                 360                 365
   Val Asn Thr Phe Tyr Ser Thr Leu Glu Asp Ser Gly Val Pro Lys His
       370                 375                 380
   His Thr His Ser Met Gly Asp Thr Met Ile Asp Tyr Asn Ala Trp Val
   385 390 395 400
   Ala Ser Leu Cys Gln Cys Pro Cys Phe Glu Glu Trp Arg Val Gln Met
   405 410 415
   Phe Tyr Glu Thr Ala Lys Arg Leu Asn Ala Asn Pro Lys Thr Phe Arg
   420 425 430
   Asp Glu Trp Glu Asp Asp Asn Leu Val Leu Gln Ala Cys Glu Asp Phe
   435 440 445
   Ser Lys Tyr Ile
   450
<210> 29
   <211> 1395
   <212> DNA
   <213> Populus trichocarpa
<400> 29
   atgcaagcat caccgaattt gctctcctcc caccacgtcg ccgtgatcgg ggcaggagcc      60
   gccggtctgg tggctgcacg tgagctccac cgagagggtc acaaagtggt ggtctttgag     120
   aaagatgacc aagttggtgg tctctggatg tacgatcccc gtgtagaacc cgaccctctc     180
   gggcttgacc taacccgacc tgttgttcac tcaagtctct acgagtctct caggaccaac     240
   ttgccaaggg agacgatggg ttttatggac tacccgtttg tgacccgaga gggtgaggga     300
   agagacccga gaaggtttcc gggtcataga gaagtgttga tgtatttgca ggattatgcc     360
   agggaatttg ggattgaaga gatggtaagg tttgggtgtg aggtggtgaa tgtagagatg     420
   attgatagtg ggaaatggaa agtgaagtca aaaaggaaga gacttgatga taatgataga     480
   ggtgatgatt ttgctgatca tgaggatttt gatgctgttg ttgtttgcgt tggacattac     540
   acccaacctc gtatcgctga aattcctggc atcaatttgt ggccggggaa gcagatacac     600
   agccacaact atcgtattcc tgagcctttc agggatcaaa tcataatttt gataggagct     660
   tctgcgagcg ctgctgatat atccgtggaa attgctggac ttgccaaaga ggttcacatt     720
   gctcgtagat cggctgtaga tgatgataca tacgaaaaaa agcctggata tgataacata     780
   tggcttcatt ccacgataga aagagcatgt gaagatggta ctgtcatttt ccgagatggc     840
   agtgttatcc tagctgacgt tattctgcac tgcaccgggt acaaatatgg cttccctttt     900
   ctgaaaactg atggcatcgt gactgtggat gacaatcgtg tggggccatt gtacaagcat     960
   gttttccctc caatcttggc cccgtggctt tcctttgtcg ggatacccta ttggactttc    1020
   cctttcccaa cgttcgaagt tcaaagcaag tggattgctg gtgttttatc aggtcgaatt    1080
   gctcttcctt cacaagagga catggtggaa gatgttaaga tctactactc tgaacttgaa    1140
   gcttctggtg tacctaagca tcacactcat aacttagctc attctacaaa tgactacaac    1200
   atgtggcttg cctcccagtg tcagtgttca tgctttgaag aatggagaat tgaaatgtcc    1260
   catgaaattc ttaagaactg gcgtgccagg ccaaatatgt atcgtgacga atgggacgac    1320
   gaccacctga tcttgcaagc ccatgaagac ttcaacagac gcatctcaaa caaagccagt    1380
   aatggtcata tctga                                                     1395
<210> 30
   <211> 464
   <212> PRT
   <213> Populus trichocarpa
<400> 30
   Met Gln Ala Ser Pro Asn Leu Leu Ser Ser His His Val Ala Val Ile
   1               5                   10                  15
   Gly Ala Gly Ala Ala Gly Leu Val Ala Ala Arg Glu Leu His Arg Glu
               20                  25                  30
   Gly His Lys Val Val Val Phe Glu Lys Asp Asp Gln Val Gly Gly Leu
           35                  40                  45
   Trp Met Tyr Asp Pro Arg Val Glu Pro Asp Pro Leu Gly Leu Asp Leu
       50                  55                  60
   Thr Arg Pro Val Val His Ser Ser Leu Tyr Glu Ser Leu Arg Thr Asn
   65                  70                  75                  80
   Leu Pro Arg Glu Thr Met Gly Phe Met Asp Tyr Pro Phe Val Thr Arg
                   85                  90                  95
   Glu Gly Glu Gly Arg Asp Pro Arg Arg Phe Pro Gly His Arg Glu Val
               100                 105                 110
   Leu Met Tyr Leu Gln Asp Tyr Ala Arg Glu Phe Gly Ile Glu Glu Met
           115                 120                 125
   Val Arg Phe Gly Cys Glu Val Val Asn Val Glu Met Ile Asp Ser Gly
       130                 135                 140
   Lys Trp Lys Val Lys Ser Lys Arg Lys Arg Leu Asp Asp Asn Asp Arg
   145                 150                 155                 160
   Gly Asp Asp Phe Ala Asp His Glu Asp Phe Asp Ala Val Val Val Cys
                   165                 170                 175
   Val Gly His Tyr Thr Gln Pro Arg Ile Ala Glu Ile Pro Gly Ile Asn
   180 185 190
   Leu Trp Pro Gly Lys Gln Ile His Ser His Asn Tyr Arg Ile Pro Glu
   195 200 205
   Pro Phe Arg Asp Gln Ile Ile Ile Leu Ile Gly Ala Ser Ala Ser Ala
   210 215 220
   Ala Asp Ile Ser Val Glu Ile Ala Gly Leu Ala Lys Glu Val His Ile
   225 230 235 240
   Ala Arg Arg Ser Ala Val Asp Asp Asp Thr Tyr Glu Lys Lys Pro Gly
   245 250 255
   Tyr Asp Asn Ile Trp Leu His Ser Thr Ile Glu Arg Ala Cys Glu Asp
   260 265 270
   Gly Thr Val Ile Phe Arg Asp Gly Ser Val Ile Leu Ala Asp Val Ile
   275 280 285
   Leu His Cys Thr Gly Tyr Lys Tyr Gly Phe Pro Phe Leu Lys Thr Asp
   290 295 300
   Gly Ile Val Thr Val Asp Asp Asn Arg Val Gly Pro Leu Tyr Lys His
   305 310 315 320
   Val Phe Pro Pro Ile Leu Ala Pro Trp Leu Ser Phe Val Gly Ile Pro
   325 330 335
   Tyr Trp Thr Phe Pro Phe Pro Thr Phe Glu Val Gln Ser Lys Trp Ile
   340 345 350
   Ala Gly Val Leu Ser Gly Arg Ile Ala Leu Pro Ser Gln Glu Asp Met
   355 360 365
   Val Glu Asp Val Lys Ile Tyr Tyr Ser Glu Leu Glu Ala Ser Gly Val
   370 375 380
   Pro Lys His His Thr His Asn Leu Ala His Ser Thr Asn Asp Tyr Asn
   385 390 395 400
   Met Trp Leu Ala Ser Gln Cys Gln Cys Ser Cys Phe Glu Glu Trp Arg
   405 410 415
   Ile Glu Met Ser His Glu Ile Leu Lys Asn Trp Arg Ala Arg Pro Asn
   420 425 430
   Met Tyr Arg Asp Glu Trp Asp Asp Asp His Leu Ile Leu Gln Ala His
   435 440 445
   Glu Asp Phe Asn Arg Arg Ile Ser Asn Lys Ala Ser Asn Gly His Ile
   450 455 460
<210> 31
   <211> 1680
   <212> DNA
   <213> Populus trichocarpa
<400> 31
   tggggaccct gctgacgcta gcatttctca agtgtccaaa aaaccgaact ctcttcgaca      60
   ggtcaccaca acaacaccaa tcaaacattt atcaatgcca ccacctcaac ttcctccacc     120
   aatctcccgc cacgtggcgg tgatcggtgc cggagccgcc ggcctcgtta gtgcccgtga     180
   gctccggaga gagggtcatg atgttgtagt ctttgaaaga gacaaccaag taggtggcac     240
   atgggtgtac aatccccgag tcgagcccga cccgttaagc ctcgacccga atcgacgcat     300
   aattcactcg agcctctata gctccctccg gaccaacctc ccaagagaag taatgggttt     360
   caaagattat ccctttatag caaaaaatga taaaaagaga gaccagagaa ggtttccggg     420
   ccatcgagag gtgttgttgt atttgcagga ttttgcaagt gagtttggga ttgaagaaat     480
   ggtgaggttt gatactgaag tggttcatgt ggggcctgtt gaggataata ttggaaagtg     540
   gattgtgagg tctaaaagga aaataagtga tgatgatagg gaggttagtt ttggatttga     600
   tgttgacgag gagatttatg atgctgttgt tatctgtaat ggacattaca ctgaacctcg     660
   tattgctcaa ataccaggga tcagttcatg gccaggaaaa cagatgcata gccacaatta     720
   tcgtactcct gagggctttc aagatcaagt ggcaattttg attggaagtt cagctagttc     780
   tgatgatata tccagagaaa ttgctggagt tgctaaagag gtccatgttg cctcaagatc     840
   agttgcggac gaaacatatc aagagcagcc tggatatgat aatatgtggc ttcattctat     900
   gatagaaagt gtgcatgatg atggttctgt gatcttcaga aatgggagag ttgtcgttgc     960
   tgacattatt ctacattgca ctgggtacaa gtatcacttc ccttttctag acaccaatgg    1020
   cattgtgacc atggatgaaa atcgtgtggc ccccctgtac aagcaagttt ttccaccagt    1080
   tctggcccca tggctttcat ttgttgggtt accgtggaag gttgtccctt ttcccttggt    1140
   tgaacttcaa accaagtgga ttgctggtgt tttatcaggt catattgcac ttccgtcacc    1200
   tgaggagatg atggaagatg ttaaagcctt ctatgagaca ctagaatctt ccaacaaacc    1260
   caaacactac actcataatt tgggtggttg tcagttcgag tacgacaact ggcttgcttc    1320
   tcagtgcggt tgcccaggga tcgaagaatg gagaaggcaa atgtatgatg cagctagcaa    1380
   gagtaagcgg ctccggccag agatataccg tgatgaatgg gatgatgatg acctggtctt    1440
   ggaagcctac ggggacttca caaagtacac ttgaaaaagt tgaagcaaca gctgcatctc    1500
   tcaatggagg tgttcgaagg ataggaaagg aagacgataa attattaggc tggcctaatt    1560
   gtcaacatct gaatttgtgg gatcaatcat catcgttgtt aatttggact tgtatttcca    1620
   tgattcgcag ttctttacgt gaataaagaa tcaatgaaga tatccatatc catatatgaa    1680
<210> 32
   <211> 459
   <212> PRT
   <213> Populus trichocarpa
<400> 32
   Met Pro Pro Pro Gln Leu Pro Pro Pro Ile Ser Arg His Val Ala Val
   1               5                   10                  15
   Ile Gly Ala Gly Ala Ala Gly Leu Val Ser Ala Arg Glu Leu Arg Arg
               20                  25                  30
   Glu Gly His Asp Val Val Val Phe Glu Arg Asp Asn Gln Val Gly Gly
           35                  40                  45
   Thr Trp Val Tyr Asn Pro Arg Val Glu Pro Asp Pro Leu Ser Leu Asp
       50                  55                  60
   Pro Asn Arg Arg Ile Ile His Ser Ser Leu Tyr Ser Ser Leu Arg Thr
   65                  70                  75                  80
   Asn Leu Pro Arg Glu Val Met Gly Phe Lys Asp Tyr Pro Phe Ile Ala
                   85                  90                  95
   Lys Asn Asp Lys Lys Arg Asp Gln Arg Arg Phe Pro Gly His Arg Glu
               100                 105                 110
   Val Leu Leu Tyr Leu Gln Asp Phe Ala Ser Glu Phe Gly Ile Glu Glu
           115                 120                 125
   Met Val Arg Phe Asp Thr Glu Val Val His Val Gly Pro Val Glu Asp
       130                 135                 140
   Asn Ile Gly Lys Trp Ile Val Arg Ser Lys Arg Lys Ile Ser Asp Asp
   145                 150                 155                 160
   Asp Arg Glu Val Ser Phe Gly Phe Asp Val Asp Glu Glu Ile Tyr Asp
                   165                 170                 175
   Ala Val Val Ile Cys Asn Gly His Tyr Thr Glu Pro Arg Ile Ala Gln
               180                 185                 190
   Ile Pro Gly Ile Ser Ser Trp Pro Gly Lys Gln Met His Ser His Asn
           195                 200                 205
   Tyr Arg Thr Pro Glu Gly Phe Gln Asp Gln Val Ala Ile Leu Ile Gly
       210                 215                 220
   Ser Ser Ala Ser Ser Asp Asp Ile Ser Arg Glu Ile Ala Gly Val Ala
   225                 230                 235                 240
   Lys Glu Val His Val Ala Ser Arg Ser Val Ala Asp Glu Thr Tyr Gln
                   245                 250                 255
   Glu Gln Pro Gly Tyr Asp Asn Met Trp Leu His Ser Met Ile Glu Ser
               260                 265                 270
   Val His Asp Asp Gly Ser Val Ile Phe Arg Asn Gly Arg Val Val Val
           275                 280                 285
   Ala Asp Ile Ile Leu His Cys Thr Gly Tyr Lys Tyr His Phe Pro Phe
       290                 295                 300
   Leu Asp Thr Asn Gly Ile Val Thr Met Asp Glu Asn Arg Val Ala Pro
   305                 310                 315                 320
   Leu Tyr Lys Gln Val Phe Pro Pro Val Leu Ala Pro Trp Leu Ser Phe
                   325                 330                 335
   Val Gly Leu Pro Trp Lys Val Val Pro Phe Pro Leu Val Glu Leu Gln
               340                 345                 350
   Thr Lys Trp Ile Ala Gly Val Leu Ser Gly His Ile Ala Leu Pro Ser
           355                 360                 365
   Pro Glu Glu Met Met Glu Asp Val Lys Ala Phe Tyr Glu Thr Leu Glu
       370                 375                 380
   Ser Ser Asn Lys Pro Lys His Tyr Thr His Asn Leu Gly Gly Cys Gln
   385                 390                 395                 400
   Phe Glu Tyr Asp Asn Trp Leu Ala Ser Gln Cys Gly Cys Pro Gly Ile
                   405                 410                 415
   Glu Glu Trp Arg Arg Gln Met Tyr Asp Ala Ala Ser Lys Ser Lys Arg
               420                 425                 430
   Leu Arg Pro Glu Ile Tyr Arg Asp Glu Trp Asp Asp Asp Asp Leu Val
           435                 440                 445
   Leu Glu Ala Tyr Gly Asp Phe Thr Lys Tyr Thr
           450                     455
<210> 33
   <211> 1347
   <212> DNA
   <213> Glycine max
<400> 33
   atgatgtcca gcgcagtcac actcctgacg ccgcgccacg tggcagtgat cggcgcgggc      60
   gccgccggcc tagtggcggc tcgggagctc cggcgagaag ggcatcgcgt ggtggttttc     120
   gagaaagggg aggaagtggg tggcatgtgg gtgtacagtc cggaggtgga ttcggatccg     180
   ctgggtttgg aggcgaagcg gagattagtc cactcgagcc tctacgattc gctccgaacg     240
   aatctgtctc gggagagcat gagtttccga gattaccctt tcaggaggag ggaggggaaa     300
   gggagggatt ctcgaaggtt cccgggtcac agagaggtgt tactgtactt gcaggatttc     360
   gctgctgaat ttgaaatcgg agaattggtg aggtttggaa cggaggtttt gtttgctgga     420
   ttggatcagt gtggaaagtg gaggctgact tcaacatcac cccatactca tcctgtggat     480
   gagatttacg acgcccttat catttgcaac ggccattacg ttcagcctcg tcttcctcat     540
   atccccggga ttaatgcatg gccagggaag cagatgcata gccataatta tagaacacct     600
   gagccctttc aagatcaagt tgtagttcta attggtagtt ctgctagtgc ggttgatatc     660
   tctcgagata tcgcaacagt tgctaaagaa gtccacattg cagctaggtc agttgaagaa     720
   gataagctag gaaaggtgcc tggccatgag aatatgtggc ttcattctat gattgacagc     780
   gttcatgaag atggtacagt ggtttttcaa gatggaaatg cagttggtgc tgacttcatc     840
   atacattgca cagggtacaa gtatgatttt cccttccttg aaaccaatgg ggaggtgact     900
   gtagatgaca accgtgtagg accactctac aaacatgttt tcccaccagc cttggctcca     960
   tggctttctt ttgttgggtt gccttggaag gttgctccct tctccttgtt cgaactgcag    1020
   agcaagtgga tagctggaat cttgtctaat cgcattgcac ttccttcgaa agaggagatg    1080
   gctaaagacg ttgatgcttt ttactcatca cttgaagcct ctggcactcc taagcgttac    1140
   actcataata tgggcattct tcagtgggac tacaataact ggattgcgga tcagtgtggg    1200
   gttccttcta ttgaagaatg gagaaggcaa atgtatatag ccacatctaa gaacagggtg    1260
   ctgcgacccg agtcttaccg tgacgagtgg gacgatgatg acttggttct gcaagctcaa    1320
   caggattttg ccaattatct cacttga                                        1347
<210> 34
   <211> 448
   <212> PRT
   <213> Glycine max
<400> 34
   Met Met Ser Ser Ala Val Thr Leu Leu Thr Pro Arg His Val Ala Val
   1               5                   10                  15
   Ile Gly Ala Gly Ala Ala Gly Leu Val Ala Ala Arg Glu Leu Arg Arg
               20                  25                  30
   Glu Gly His Arg Val Val Val Phe Glu Lys Gly Glu Glu Val Gly Gly
           35                  40                  45
   Met Trp Val Tyr Ser Pro Glu Val Asp Ser Asp Pro Leu Gly Leu Glu
       50                  55                  60
   Ala Lys Arg Arg Leu Val His Ser Ser Leu Tyr Asp Ser Leu Arg Thr
   65                  70                  75                  80
   Asn Leu Ser Arg Glu Ser Met Ser Phe Arg Asp Tyr Pro Phe Arg Arg
                   85                  90                  95
   Arg Glu Gly Lys Gly Arg Asp Ser Arg Arg Phe Pro Gly His Arg Glu
               100                 105                 110
   Val Leu Leu Tyr Leu Gln Asp Phe Ala Ala Glu Phe Glu Ile Gly Glu
           115                 120                 125
   Leu Val Arg Phe Gly Thr Glu Val Leu Phe Ala Gly Leu Asp Gln Cys
       130                 135                 140
   Gly Lys Trp Arg Leu Thr Ser Thr Ser Pro His Thr His Pro Val Asp
   145                 150                 155                 160
   Glu Ile Tyr Asp Ala Leu Ile Ile Cys Asn Gly His Tyr Val Gln Pro
                   165                 170                 175
   Arg Leu Pro His Ile Pro Gly Ile Asn Ala Trp Pro Gly Lys Gln Met
               180                 185                 190
   His Ser His Asn Tyr Arg Thr Pro Glu Pro Phe Gln Asp Gln Val Val
           195                 200                 205
   Val Leu Ile Gly Ser Ser Ala Ser Ala Val Asp Ile Ser Arg Asp Ile
       210                 215                 220
   Ala Thr Val Ala Lys Glu Val His Ile Ala Ala Arg Ser Val Glu Glu
   225                 230                 235                 240
   Asp Lys Leu Gly Lys Val Pro Gly His Glu Asn Met Trp Leu His Ser
   245 250 255
   Met Ile Asp Ser Val His Glu Asp Gly Thr Val Val Phe Gln Asp Gly
   260 265 270
   Asn Ala Val Gly Ala Asp Phe Ile Ile His Cys Thr Gly Tyr Lys Tyr
   275 280 285
   Asp Phe Pro Phe Leu Glu Thr Asn Gly Glu Val Thr Val Asp Asp Asn
   290 295 300
   Arg Val Gly Pro Leu Tyr Lys His Val Phe Pro Pro Ala Leu Ala Pro
   305 310 315 320
   Trp Leu Ser Phe Val Gly Leu Pro Trp Lys Val Ala Pro Phe Ser Leu
   325 330 335
   Phe Glu Leu Gln Ser Lys Trp Ile Ala Gly Ile Leu Ser Asn Arg Ile
   340 345 350
   Ala Leu Pro Ser Lys Glu Glu Met Ala Lys Asp Val Asp Ala Phe Tyr
   355 360 365
   Ser Ser Leu Glu Ala Ser Gly Thr Pro Lys Arg Tyr Thr His Asn Met
   370 375 380
   Gly Ile Leu Gln Trp Asp Tyr Asn Asn Trp Ile Ala Asp Gln Cys Gly
   385 390 395 400
   Val Pro Ser Ile Glu Glu Trp Arg Arg Gln Met Tyr Ile Ala Thr Ser
   405 410 415
   Lys Asn Arg Val Leu Arg Pro Glu Ser Tyr Arg Asp Glu Trp Asp Asp
   420 425 430
   Asp Asp Leu Val Leu Gln Ala Gln Gln Asp Phe Ala Asn Tyr Leu Thr
   435 440 445
<210> 35
   <211> 1703
   <212> DNA
   <213> Solanum lycopersicum
<400> 35
   ggtggcgtga cactcccgct gcctttagta gatccgcccc cgctgaatgt ggtgaggaat 60
   caaccaacaa atccacatgc aaagatactc aaatacaaaa ctcaaccaaa caaatccaca 120
   tgcaaactca aacacaacat aaaatattac tttagtcatg attcttaaaa atgtcatctt 180
   ttcaaaccaa ccttatactc gtacacttgt atgccatttg cccatgtctc aaaattcctc 240
   caaaaacgtc gccgttattg gtgcgggctc cgcgggcctc gttgcggccc gagaactcca 300
   acgagaaggt catagagtag ttgtattcga acgagaaaat caattaggag gcacatgggt 360
   ttacacgccc gatacagaat ccgacccggt tgggatcgac ccgaatcggg agattgttca 420
   ttcaagtctt tattcatctc tccgtgttaa tcttccccgg gaagtaatgg gttttgggga 480
   ttacccgttt gtggccaaga aaaagcccgg tagagacccg agaaggtatc cgagtcatgg 540
   ggaggtgttg gagtatttga atgattttgc tgttgatttt gggattattg gggttgtgag 600
   gtttgggatg gaagtggggt ttgtgggaaa gatggagaat ggaaaatgga aggttagttg 660
   tagaaagagg gaaaatgatg atttgtttgc taatgaggag tatgatgctg ttgtaatatg 720
   taatggacac tatactgaac caagaattgc tgatattcct ggaatcgaag tatggcctgg 780
   aaagcaaatt cacagccaca actaccgtgt tcctgaccct tttcgagacc aagttgttgt 840
   gctgataggt ggtgctgcaa gtgctactga tatctccagg gaaattgctg aagttgctaa 900
   agaggtccac atttcttcta ggtcagctac tagtggagtt ccgatgaagc tgcctggtta 960
   tgataatatt tggctccata atatgattga agctgttggc agtgatggtg gcgtgaattt 1020
   tcaagatggg tcgaaaatcc ttgctgacat catcctacac tgcacagggt acaaatatca 1080
   ttttcctttc ctcgaaacta acgggatagt gactgtggat gacaaccgtg ttggtccact 1140
   ttacaagcac gttttcccac cagcctttgc accaagcctt tcatttgttg ggctgccttg 1200
   gaaggttata ccattcttct tgtgtgaatt gcaaagcaag tggatcgctg gtgttttatc 1260
   tggtcgaatt tctctcccat caaaggaaga tatgaatgct gatattgaag ctttctactc 1320
   atccatggca gcctcttgca ttccaaaacg gtacactcac aatatggacg actctcagtt 1380
   tgactacgat gattggttgg ctgctcagtg tggatctaca ccctttgaag aatggagaaa 1440
   acaaatgtac ttaatctcaa gaaagaacaa aaggactctg cccgagacat atcgtgacga 1500
   gtgggacgat gatgacttga tcattcaagc tcatgaagac ttcgtaaaat atattcctga 1560
   actagctcaa gaacagaagc tctcaagatg attaattttg ttgttacatg aaaatatagc 1620
   tgaataaatc gagaagtact gtaaataaac aggaaattac tcaattaatt tcaattgcaa 1680
   ctcttgccac atgaaaaaaa aaa 1703
<210> 36
   <211> 477
   <212> PRT
   <213> Solanum lycopersicum
<400> 36
   Met Ile Leu Lys Asn Val Ile Phe Ser Asn Gln Pro Tyr Thr Arg Thr
   1               5                   10                  15
   Leu Val Cys His Leu Pro Met Ser Gln Asn Ser Ser Lys Asn Val Ala
               20                  25                  30
   Val Ile Gly Ala Gly Ser Ala Gly Leu Val Ala Ala Arg Glu Leu Gln
           35                  40                  45
   Arg Glu Gly His Arg Val Val Val Phe Glu Arg Glu Asn Gln Leu Gly
       50                  55                  60
   Gly Thr Trp Val Tyr Thr Pro Asp Thr Glu Ser Asp Pro Val Gly Ile
   65                  70                  75                  80
   Asp Pro Asn Arg Glu Ile Val His Ser Ser Leu Tyr Ser Ser Leu Arg
                   85                  90                  95
   Val Asn Leu Pro Arg Glu Val Met Gly Phe Gly Asp Tyr Pro Phe Val
               100                 105                 110
   Ala Lys Lys Lys Pro Gly Arg Asp Pro Arg Arg Tyr Pro Ser His Gly
           115                 120                 125
   Glu Val Leu Glu Tyr Leu Asn Asp Phe Ala Val Asp Phe Gly Ile Ile
       130                 135                 140
   Gly Val Val Arg Phe Gly Met Glu Val Gly Phe Val Gly Lys Met Glu
   145                 150                 155                 160
   Asn Gly Lys Trp Lys Val Ser Cys Arg Lys Arg Glu Asn Asp Asp Leu
                   165                 170                 175
   Phe Ala Asn Glu Glu Tyr Asp Ala Val Val Ile Cys Asn Gly His Tyr
               180                 185                 190
   Thr Glu Pro Arg Ile Ala Asp Ile Pro Gly Ile Glu Val Trp Pro Gly
           195                 200                 205
   Lys Gln Ile His Ser His Asn Tyr Arg Val Pro Asp Pro Phe Arg Asp
       210                 215                 220
   Gln Val Val Val Leu Ile Gly Gly Ala Ala Ser Ala Thr Asp Ile Ser
   225                 230                 235                 240
   Arg Glu Ile Ala Glu Val Ala Lys Glu Val His Ile Ser Ser Arg Ser
                   245                 250                 255
   Ala Thr Ser Gly Val Pro Met Lys Leu Pro Gly Tyr Asp Asn Ile Trp
               260                 265                 270
   Leu His Asn Met Ile Glu Ala Val Gly Ser Asp Gly Gly Val Asn Phe
   275 280 285
   Gln Asp Gly Ser Lys Ile Leu Ala Asp Ile Ile Leu His Cys Thr Gly
   290 295 300
   Tyr Lys Tyr His Phe Pro Phe Leu Glu Thr Asn Gly Ile Val Thr Val
   305 310 315 320
   Asp Asp Asn Arg Val Gly Pro Leu Tyr Lys His Val Phe Pro Pro Ala
   325 330 335
   Phe Ala Pro Ser Leu Ser Phe Val Gly Leu Pro Trp Lys Val Ile Pro
   340 345 350
   Phe Phe Leu Cys Glu Leu Gln Ser Lys Trp Ile Ala Gly Val Leu Ser
   355 360 365
   Gly Arg Ile Ser Leu Pro Ser Lys Glu Asp Met Asn Ala Asp Ile Glu
   370 375 380
   Ala Phe Tyr Ser Ser Met Ala Ala Ser Cys Ile Pro Lys Arg Tyr Thr
   385 390 395 400
   His Asn Met Asp Asp Ser Gln Phe Asp Tyr Asp Asp Trp Leu Ala Ala
   405 410 415
   Gln Cys Gly Ser Thr Pro Phe Glu Glu Trp Arg Lys Gln Met Tyr Leu
   420 425 430
   Ile Ser Arg Lys Asn Lys Arg Thr Leu Pro Glu Thr Tyr Arg Asp Glu
   435 440 445
   Trp Asp Asp Asp Asp Leu Ile Ile Gln Ala His Glu Asp Phe Val Lys
   450 455 460
   Tyr Ile Pro Glu Leu Ala Gln Glu Gln Lys Leu Ser Arg
   465 470 475
<210> 37
   <211> 4956
   <212> DNA
   <213> Solanum lycopersicum
<400> 37
   atgattctta aaaatgtcat cttttcaaac caaccttata ctcgtacact tgtatgccat 60
   ttgcccatgt ctcaaaattc ctccaaaaac gtcgccgtta ttggtgcggg ctccgcgggc 120
   ctcgttgcgg cccgagaact ccaacgagaa ggtcatagag tagttgtatt cgaacgagaa 180
   aatcaattag gaggcacatg ggtttacacg cccgatacag aatccgaccc ggttgggatc 240
   gacccgaatc gggagattgt tcattcaagt ctttattcat ctctccgtgt taatcttccc 300
   cgggaagtaa tgggttttgg ggattacccg tttgtggcca agaaaaagcc cggtagagac 360
   ccgagaaggt atccgagtca tggggaggtg ttggagtatt tgaatgattt tgctgttgat 420
   tttgggatta ttggggttgt gaggtttggg atggaagtgg ggtttgtggg aaagatggag 480
   aatggaaaat ggaaggttag ttgtagaaag agggaaaatg atgatttgtt tgctaatgag 540
   gagtatgatg ctgttgtaat atgtaatgga cactatactg aaccaagaat tgctgatatt 600
   cctggtaatt tattgaaaaa atttgatctt tatgttgaat aaagtaccat atctccttta 660
   gtcactatca gatttttcga aattatagat tctttggtga aataagttta atgctgatga 720
   acttttttac acttctgtgc ttttagtaat ccagttgtag tacctttttt ttagcttttg 780
   tgcaccaggt atttgataac tttgtcctag gacaaatgga aaaaatgatt ttaacttgac 840
   attttattga cctctagatc atgcccttgg gtgcatccaa ttgtactttg atctttagtt 900
   gttcatactt gaatagaata ttatctccag gagttatgtt tctctttgta gtaattgtag 960
   attcgtttag ttgagggaat aagtctaaag tgttgatgaa atttttgttt tagtgataac 1020
   gtttgatatg aagatttcaa acatctgtac attgtgctaa tagttacatc ctactaactg 1080
   aaatataagt tgtaccctgt gggcgagcaa agttgatctt tttatgacaa agatcaacct 1140
   tcttcttttc tctttctgtt gttaaggcag tgtccacgag aaagaatgaa tattgtacag 1200
   ttaagattag tcttttttca gtggaaagat tccagccttc tttttggctt agacataatt 1260
   ttgtttctct tatgtggcac acatatgtta agtaactttt ttcctgttat ggttttatac 1320
   gttgtgagca gttcaaaatt ttgattttta tttgaattcg cacatctttt ccattttctg 1380
   tcttgttcag aacaaccata taacaaagtg aagtattctg aaattcaata gtgtcttttt 1440
   taacttgaaa gtgtggctga aattttcttt tttccatttt aacatcattt tgtggcaata 1500
   ctatatgatt agctctgaga atatctgtga gttgccaaac tattgacata ctaagtcaat 1560
   atgcagtttt tgaagtttct aactggaggc ctctagcctt tcaggaatcg aagtatggcc 1620
   tggaaagcaa attcacagcc acaactaccg tgttcctgac ccttttcgag accaagtatg 1680
   tgtcaggttc attgtttctt taagatgttg atgtttctga actaatactg ttgtgactga 1740
   aatgcttgca gttacttcaa ttatgaattt gctctactta ttggaagcga tcttttaatt 1800
   acttactaaa agtgttctgt gatattctat tgcgtgaagt ttcaggccag atataataga 1860
   ttattgaatg gaacgttctt gaacaattct attattgaat tcttactttt gcatgaagca 1920
   ctggatgtat gagtgatgac gccaaaaaca aagaaaactt taaattttaa atctaccttt 1980
   tcttcataaa tgaatattaa tagttagtac aagtatagta gtcctccttc ctaggaagct 2040
   gtttagaaac gatggactat acagaaaatg aacattatgt atatatgctt atatgagcct 2100
   aggtatgact tgggaaaaat gctccttggt tatatcagct tctaatattt gacaaatata 2160
   gctcaagaaa tagtatcatc tttttgcctg atcccatggc ataactacca ccctttgtac 2220
   caagttcaga gtaaatgaca aatggttttt tgccaggttg ttgtgctgat aggtggtgct 2280
   gcaagtgcta ctgatatctc cagggaaatt gctgaagttg ctaaagaggt ccacatttct 2340
   tctaggtcag ctactagtgg agttccgatg aagctgcctg gttatgataa tatttggctc 2400
   cataatatgg taaagtggtt aataacttgt atattcatgt ggaggtttgt caacgcttga 2460
   tgctgaatct gtacttcaca tcccctggat actatttaat gaaatcctct tacgtcataa 2520
   aaagaaaaga aaaaaacaca tgatactgaa tcgtcagttg cgtaggaaaa aatttatact 2580
   agctaatgat gcagattgaa gctgttggca gtgatggtgg cgtgaatttt caagatgggt 2640
   cgaaaatcct tgctgacatc atcctacact gcacagggtg agtgattagt cctattgaaa 2700
   cttccctttt cgcctacggt taaagaaaat gaaagaaagc tgacattgat agcctattct 2760
   ttttcttttt gaggaaatgt gaagggtaat gcatgcctgt ttttctgagg gtcataatga 2820
   tatgaaattg cgaggtgaac tgaccttata aaatagcaaa agaatgatga ggtcaacaga 2880
   tagttaaagt aggaactggc atgataaggg tagataatgg atccaaaggc aagaaacagt 2940
   aaataaagta gttgatctgc tcttgagtag gcagtctcaa aatgaacctc ccatattata 3000
   tgtttaattt cataattgta agcgtaaaaa gatattctaa tctataggtt agaaacagta 3060
   aattaagtag ttaatctcct tgagtacgcc aatctaaaaa taaaccttgt agcctttaat 3120
   acctttaggg gctgtttggt tgatgggatg gcatagacaa ggatatccca ttggattatt 3180
   ttgtcttacc ttctacaagg gataaaatat cccatcattt atactaaagt ggtgggtcaa 3240
   aataatacct atcaccaatc acaagataaa ataaccacat gggatatatc gggattatta 3300
   tcgttatccc atctaccaaa tgacccctaa gtataagctt aaaggaatgt ctattaatat 3360
   tgtcttatcc cagaccccta aaagattgaa gaatgtctat taatattttc atagcctata 3420
   agaacatcag aaatccagta gttatctatt taagtattac agtttagtga ggcagatttg 3480
   gttagatatt gtgaaggtag ccaaagacta aggagtgtaa atttttctgt tccgggttac 3540
   agaaacgaag ggaagttatc ttcagctaaa atgaatacta atttatgtga gtttggtagt 3600
   acaggcctcc taattcatag taagatgtct ctaaccttta cgattgtaat aaaaaataat 3660
   tgctagtaat cttacaaaat aatcaaattg atgggagaat aagtatcatg aatgtttctt 3720
   atctttgttt cccaggtaca aatatcattt tcctttcctc gaaactaacg ggatagtgac 3780
   tgtggatgac aaccgtgttg gtccacttta caagcacgtt ttcccaccag cctttgcacc 3840
   aagcctttca tttgttgggc tgccttggaa ggtaaaattt agagggtttg tgctggggtt 3900
   tatagattta cattttaaat ggtggacttg aacgtaatat ttgtcctggt tggcaccagg 3960
   ttataccatt cttcttgtgt gaattgcaaa gcaagtggat cgctggtgtt ttatctggtc 4020
   gaatttctct cccatcaaag gaagatatga atgctgatat tgaagctttc tactcatcca 4080
   tggcagcctc ttgcattcca aaacggtaca ctcacaatat ggacgactct caggtataac 4140
   atctgccaaa aaaccttgtc gaatggtttt aggttttttg ttttgttcta tgggtaaagt 4200
   gtggtagaaa cagctgaaca cttcatgcct acccgtaaca tacagaagtt cttggttaca 4260
   ccatcttagt taagttagaa aagaaaagaa agaaacagag aaaagaatcg aaactgaaga 4320
   attagagtgt agtaagcttc tgtcattaat tcagtgcacg atcctgattt agttggggtt 4380
   ccaatgtggg cttcaaacac cgggtgggaa acccaaaaag aaaagaaaag aaagaaagcg 4440
   agtgtagagc ttctgtcatt gattaacata gactgctaat atgaacacat ccaagttggg 4500
   ggttctttag cacggtgaca ttatagtcgt cctttatgag atatgaatct ctgagcgtgt 4560
   tgagcatgtt tactttctgt atatgctggc taaacaagat tgttgcacag caacaccaaa 4620
   ccaaatggct ttgccccttt tatacaagag tgtagctctg gtgttcatta tgttatgtat 4680
   tatagattac caaaacagta ttacatatag ttcgtgcctc gtaaataatc cctctcttta 4740
   cagtttgact acgatgattg gttggctgct cagtgtggat ctacaccctt tgaagaatgg 4800
   agaaaacaaa tgtacttaat ctcaagaaag aacaaaagga ctctgcccga gacatatcgt 4860
   gacgagtggg acgatgatga cttgatcatt caagctcatg aagacttcgt aaaatatatt 4920
   cctgaactag ctcaagaaca gaagctctca agatga 4956
<210> 38
   <211> 213
   <212> PRT
   <213> Solanum lycopersicum
<400> 38
   Met Ile Leu Lys Asn Val Ile Phe Ser Asn Gln Pro Tyr Thr Arg Thr
   1 5 10 15
   Leu Val Cys His Leu Pro Met Ser Gln Asn Ser Ser Lys Asn Val Ala
   20 25 30
   Val Ile Gly Ala Gly Ser Ala Gly Leu Val Ala Ala Arg Glu Leu Gln
   35 40 45
   Arg Glu Gly His Arg Val Val Val Phe Glu Arg Glu Asn Gln Leu Gly
   50 55 60
   Gly Thr Trp Val Tyr Thr Pro Asp Thr Glu Ser Asp Pro Val Gly Ile
   65 70 75 80
   Asp Pro Asn Arg Glu Ile Val His Ser Ser Leu Tyr Ser Ser Leu Arg
                   85                  90                  95
   Val Asn Leu Pro Arg Glu Val Met Gly Phe Gly Asp Tyr Pro Phe Val
               100                 105                 110
   Ala Lys Lys Lys Pro Gly Arg Asp Pro Arg Arg Tyr Pro Ser His Gly
           115                 120                 125
   Glu Val Leu Glu Tyr Leu Asn Asp Phe Ala Val Asp Phe Gly Ile Ile
       130                 135                 140
   Gly Val Val Arg Phe Gly Met Glu Val Gly Phe Val Gly Lys Met Glu
   145                 150                 155                 160
   Asn Gly Lys Trp Lys Val Ser Cys Arg Lys Arg Glu Asn Asp Asp Leu
                   165                 170                 175
   Phe Ala Asn Glu Glu Tyr Asp Ala Val Val Ile Cys Asn Gly His Tyr
               180                 185                 190
   Thr Glu Pro Arg Ile Ala Asp Ile Pro Gly Asn Leu Leu Lys Lys Phe
           195                 200                 205
   Asp Leu Tyr Val Glu
       210
<210> 39
   <211> 2044
   <212> DNA
   <213> Homo sapiens
<400> 39
   caggacgtag acacacagaa gaaaagaaga caaagaacgg gttaccatgg ggaagaaagt      60
   ggccatcatt ggagctggtg tgagtggctt ggcctccatc aggagctgtc tggaagaggg     120
   gctggagccc acctgctttg agaagagcaa tgacattggg ggcctgtgga aattttcaga     180
   ccatgcagag gagggcaggg ctagcattta caaatcagtc ttttccaact cttccaaaga     240
   gatgatgtgt ttcccagact tcccatttcc cgatgacttc cccaacttta tgcacaacag     300
   caagatccag gaatatatca ttgcatttgc caaagaaaag aacctcctga agtacataca     360
   atttaagaca tttgtatcca gtgtaaataa acatcctgat tttgcaacta ctggccagtg     420
   ggatgttacc actgaaaggg atggtaaaaa agaatcggct gtctttgatg ctgtaatggt     480
   ttgttccgga catcatgtgt atcccaacct accaaaagag tcctttccag gactaaacca     540
   ctttaaaggc aaatgcttcc acagcaggga ctataaagaa ccaggtgtat tcaatggaaa     600
   gcgtgtcctg gtggttggcc tggggaattc gggctgtgat attgccacag aactcagccg 660
   cacagcagaa caggtcatga tcagttccag aagtggctcc tgggtgatga gccgggtctg 720
   ggacaatggt tatccttggg acatgctgct cgtcactcga tttggaacct tcctcaagaa 780
   caatttaccg acagccatct ctgactggtt gtacatgaag cagatgaatg caagattcaa 840
   gcatgaaaac tatggcttga tgcctttaaa tggagtcctg aggaaagagc ctgtatttaa 900
   cgatgagctc ccagcaagca ttctgtgtgg cattgtgtcc gtaaagccta acgtgaagga 960
   attcacagag acctcggcca tttttgagga tgggaccata tttgagggca ttgactgtgt 1020
   aatctttgca acagggtata gttttgccta ccccttcctt gatgagtcta tcatcaaaag 1080
   cagaaacaat gagatcattt tatttaaagg agtatttcct cctctacttg agaagtcaac 1140
   catagcagtg attggctttg tccagtccct tggggctgcc attcccacag ttgacctcca 1200
   gtcccgctgg gcagcacaag taataaaggg aacttgtact ttgccttcta tggaagacat 1260
   gatgaatgat attaatgaga aaatggagaa aaagcgcaaa tggtttggca aaagcgagac 1320
   catacagaca gattacattg tttatatgga tgaactctcc tccttcattg gggcaaagcc 1380
   caacatccca tggctgtttc tcacagatcc caaattggcc atggaagttt attttggccc 1440
   ttgtagtccc taccagttta ggctggtggg cccagggcag tggccaggag ccagaaatgc 1500
   catactgacc cagtgggacc ggtcgttgaa acccatgcag acacgagtgg tcgggagact 1560
   tcagaagcct tgcttctttt tccattggct gaagctcttt gcaattccta ttctgttaat 1620
   cgctgttttc cttgtgttga cctaatcatc attttctcta ggatttctga aagttactga 1680
   caatacccag acaggggctt tgctatttaa aaattaaaat tttcacacca cctgcttttc 1740
   tattcagcat cttttgcagt actctgtaga cattagtcag taatacagtg ttatttctag 1800
   gctctgaaat agccacttta agaatcatgt catgatctta agagagcact aatcatttct 1860
   gtttgagttc cactaacact tcaaaatcag aactatgttc tttatatcta acttaaatca 1920
   tttcctgaaa cattttgaca tgattccttt ttccttttaa acaatgtatg aaagatgtat 1980
   tttaaatcta aataaagagc aaattaagca gaataaaaaa aaaaaaaaaa aaaaaaaaaa 2040
   aaaa 2044
<210> 40
   <211> 532
   <212> PRT
   <213> Homo sapiens
<400> 40
   Met Gly Lys Lys Val Ala Ile Ile Gly Ala Gly Val Ser Gly Leu Ala
   1               5                   10                  15
   Ser Ile Arg Ser Cys Leu Glu Glu Gly Leu Glu Pro Thr Cys Phe Glu
               20                  25                  30
   Lys Ser Asn Asp Ile Gly Gly Leu Trp Lys Phe Ser Asp His Ala Glu
           35                  40                  45
   Glu Gly Arg Ala Ser Ile Tyr Lys Ser Val Phe Ser Asn Ser Ser Lys
       50                  55                  60
   Glu Met Met Cys Phe Pro Asp Phe Pro Phe Pro Asp Asp Phe Pro Asn
   65                  70                  75                  80
   Phe Met His Asn Ser Lys Ile Gln Glu Tyr Ile Ile Ala Phe Ala Lys
                   85                  90                  95
   Glu Lys Asn Leu Leu Lys Tyr Ile Gln Phe Lys Thr Phe Val Ser Ser
               100                 105                 110
   Val Asn Lys His Pro Asp Phe Ala Thr Thr Gly Gln Trp Asp Val Thr
           115                 120                 125
   Thr Glu Arg Asp Gly Lys Lys Glu Ser Ala Val Phe Asp Ala Val Met
       130                 135                 140
   Val Cys Ser Gly His His Val Tyr Pro Asn Leu Pro Lys Glu Ser Phe
   145                 150                 155                 160
   Pro Gly Leu Asn His Phe Lys Gly Lys Cys Phe His Ser Arg Asp Tyr
                   165                 170                 175
   Lys Glu Pro Gly Val Phe Asn Gly Lys Arg Val Leu Val Val Gly Leu
               180                 185                 190
   Gly Asn Ser Gly Cys Asp Ile Ala Thr Glu Leu Ser Arg Thr Ala Glu
           195                 200                 205
   Gln Val Met Ile Ser Ser Arg Ser Gly Ser Trp Val Met Ser Arg Val
       210                 215                 220
   Trp Asp Asn Gly Tyr Pro Trp Asp Met Leu Leu Val Thr Arg Phe Gly
   225                 230                 235                 240
   Thr Phe Leu Lys Asn Asn Leu Pro Thr Ala Ile Ser Asp Trp Leu Tyr
                   245                 250                 255
   Met Lys Gln Met Asn Ala Arg Phe Lys His Glu Asn Tyr Gly Leu Met
               260                 265                 270
   Pro Leu Asn Gly Val Leu Arg Lys Glu Pro Val Phe Asn Asp Glu Leu
           275                 280                 285
   Pro Ala Ser Ile Leu Cys Gly Ile Val Ser Val Lys Pro Asn Val Lys
       290                 295                 300
   Glu Phe Thr Glu Thr Ser Ala Ile Phe Glu Asp Gly Thr Ile Phe Glu
   305                 310                 315                 320
   Gly Ile Asp Cys Val Ile Phe Ala Thr Gly Tyr Ser Phe Ala Tyr Pro
                   325                 330                 335
   Phe Leu Asp Glu Ser Ile Ile Lys Ser Arg Asn Asn Glu Ile Ile Leu
               340                 345                 350
   Phe Lys Gly Val Phe Pro Pro Leu Leu Glu Lys Ser Thr Ile Ala Val
           355                 360                 365
   Ile Gly Phe Val Gln Ser Leu Gly Ala Ala Ile Pro Thr Val Asp Leu
       370                 375                 380
   Gln Ser Arg Trp Ala Ala Gln Val Ile Lys Gly Thr Cys Thr Leu Pro
   385                 390                 395                 400
   Ser Met Glu Asp Met Met Asn Asp Ile Asn Glu Lys Met Glu Lys Lys
                   405                 410                 415
   Arg Lys Trp Phe Gly Lys Ser Glu Thr Ile Gln Thr Asp Tyr Ile Val
               420                 425                 430
   Tyr Met Asp Glu Leu Ser Ser Phe Ile Gly Ala Lys Pro Asn Ile Pro
           435                 440                 445
   Trp Leu Phe Leu Thr Asp Pro Lys Leu Ala Met Glu Val Tyr Phe Gly
       450                 455                 460
   Pro Cys Ser Pro Tyr Gln Phe Arg Leu Val Gly Pro Gly Gln Trp Pro
   465                 470                 475                 480
   Gly Ala Arg Asn Ala Ile Leu Thr Gln Trp Asp Arg Ser Leu Lys Pro
                   485                 490                 495
   Met Gln Thr Arg Val Val Gly Arg Leu Gln Lys Pro Cys Phe Phe Phe
               500                 505                 510
   His Trp Leu Lys Leu Phe Ala Ile Pro Ile Leu Leu Ile Ala Val Phe
           515                 520                 525
   Leu Val Leu Thr
   530
<210> 41
   <211> 2190
   <212> DNA
   <213> Oryctolagus cuniculus
<400> 41
   tcacagcatc cggcggctcg cagcggcggg acatcgcgga cgttcgcgca ggcggactag      60
   tgacttccac gcaagacagg cgacgctgcc gggagaccat ggccgggaaa agagtggcgg     120
   tgattggggc gggagcgagc gggctggctt gcatcaagtg ctgcctggaa gagggcttgg     180
   agcccgtctg cttcgaaagg accgacgaca tcggaggtct gtggaggttc caggaaagtc     240
   ccgacgaagg aagggccagt atctacaaat ccgtgatcat caacacctcc aaggagatga     300
   tgtgcttcag tgactacccc atcccggatc attttcctaa cttcatgcac aactcccagg     360
   tcctggagta cttcaggatg tacgccaaag aatttggtct tctgaagtat attcagttta     420
   agaccactgt gtgcagtgtg aagaagcggc ctgatttctc cacgtcgggc caatgggagg     480
   tgctgactga gtgcgaaggg aaaaaggaga gtgctgtctt cgatggggtc ctggtttgca     540
   ccggccatca caccagtgct cacctgccac tggaaagctt ccctgggatt gagaagttca     600
   aagggcagta cttgcacagt cgagactata agaacccaga gaaattcact ggaaagagag     660
   tcattgtcat tggcattggg aattctggag gggacctggc tgtggagatc agccacacag      720
   ccaagcaggt cttcctcagc accaggagag gggcttggat catgaatcgt gtcggcgacc      780
   atggatatcc tattgatata ctgctgtctt ctcgatttag tcaatttttg aagaagatta     840
   ctggtgaaac aatagcaaat tcatttttgg aaagaaagat gaaccaaagg tttgaccatg     900
   caatgtttgg tctgaagcct aaacacagag ctttgagtca acacccaaca gtaaatgatg     960
   acctgccaaa tcgtatcatt tctggctctg tcaagatcaa aggaaatgtg aaggaattca    1020
   cagaaacagc tgccatattt gaagatggct ccagggagga tgacattgat gctgttattt    1080
   ttgccacagg ctatagcttt tcctttcctt ttcttgaaga ctctgtcaaa gtggtgaaaa    1140
   acaaggtatc tctgtataaa aaggtcttcc cccctaacct ggaaaagcca actcttgcaa    1200
   tcataggctt gatccagccc ctgggagcca ttatgcccat ttcagagctc caagcacgat    1260
   gggccaccct agtgtttaaa gggctaaaga ctttaccctc acaaagtgaa atgatgacag    1320
   aaatatctca ggttcaagag aaaatggcaa aaaggtatgt ggagagccaa cgccatacca    1380
   ttcagggaga ctacatagag accatggaag aaattgctga tttggtgggg gtcaggccaa    1440
   atttgctgtc tctggctttc accgacccca ggctggcatt acaattactt ttgggaccct    1500
   gtactccagt ccattatcgt ctccagggcc gtggaaagtg ggatggggct cggaaaacca    1560
   tccttaccgt agaagatcgg atcaggaagc ctctgatgac aagagtcacg gaaagcagta      1620
   actctgtgac ctcgatgatg acaatgggca agtttatgct agctattgct ttcttagcca      1680
   tagctgtggt ttatttttag ctgtcccttt gtcattgcct ctgctttcat tgggaagctt      1740
   aacttagaga gagatacctt cagaatttta caagatcaaa tgaccctcct ctttcaaatt      1800
   gccccatttc tctttcaaaa gcattaattc tctcttcatt ttcctacagt gagatccaag      1860
   cttttcattt gcactaagca tctcctcacc tctcatgagc cttcactttc tctctccaga      1920
   gcagctcggg tactcttagt catctttgta tgtccctagc agagtagttg acatttggct      1980
   ggtgtttaac caatgtttgg tgttgtggct caaagtctgt ttttgtatgg gaaatgactg      2040
   actgtataac tctgcttggg atggaatttg gttttccatt atttttgtct ttaacattat      2100
   aacaaatgta tgtttcctga gaaataagat taataatgac cttcgtaatt gtagacaaat      2160
   aaatacttaa gttactttgt tctacatgcc                                       2190
<210> 42
   <211> 533
   <212> PRT
   <213> Oryctolagus cuniculus
<400> 42
   Met Ala Gly Lys Arg Val Ala Val Ile Gly Ala Gly Ala Ser Gly Leu
   1               5                   10                  15
   Ala Cys Ile Lys Cys Cys Leu Glu Glu Gly Leu Glu Pro Val Cys Phe
               20                  25                  30
   Glu Arg Thr Asp Asp Ile Gly Gly Leu Trp Arg Phe Gln Glu Ser Pro
           35                  40                  45
   Asp Glu Gly Arg Ala Ser Ile Tyr Lys Ser Val Ile Ile Asn Thr Ser
       50                  55                  60
   Lys Glu Met Met Cys Phe Ser Asp Tyr Pro Ile Pro Asp His Phe Pro
   65                  70                  75                  80
   Asn Phe Met His Asn Ser Gln Val Leu Glu Tyr Phe Arg Met Tyr Ala
                   85                  90                  95
   Lys Glu Phe Gly Leu Leu Lys Tyr Ile Gln Phe Lys Thr Thr Val Cys
               100                 105                 110
   Ser Val Lys Lys Arg Pro Asp Phe Ser Thr Ser Gly Gln Trp Glu Val
           115                 120                 125
   Leu Thr Glu Cys Glu Gly Lys Lys Glu Ser Ala Val Phe Asp Gly Val
       130                 135                 140
   Leu Val Cys Thr Gly His His Thr Ser Ala His Leu Pro Leu Glu Ser
   145                 150                 155                 160
   Phe Pro Gly Ile Glu Lys Phe Lys Gly Gln Tyr Leu His Ser Arg Asp
                   165                 170                 175
   Tyr Lys Asn Pro Glu Lys Phe Thr Gly Lys Arg Val Ile Val Ile Gly
               180                 185                 190
   Ile Gly Asn Ser Gly Gly Asp Leu Ala Val Glu Ile Ser His Thr Ala
           195                 200                 205
   Lys Gln Val Phe Leu Ser Thr Arg Arg Gly Ala Trp Ile Met Asn Arg
       210                 215                 220
   Val Gly Asp His Gly Tyr Pro Ile Asp Ile Leu Leu Ser Ser Arg Phe
   225                 230                 235                 240
   Ser Gln Phe Leu Lys Lys Ile Thr Gly Glu Thr Ile Ala Asn Ser Phe
                   245                 250                 255
   Leu Glu Arg Lys Met Asn Gln Arg Phe Asp His Ala Met Phe Gly Leu
               260                 265                 270
   Lys Pro Lys His Arg Ala Leu Ser Gln His Pro Thr Val Asn Asp Asp
           275                 280                 285
   Leu Pro Asn Arg Ile Ile Ser Gly Ser Val Lys Ile Lys Gly Asn Val
       290                 295                 300
   Lys Glu Phe Thr Glu Thr Ala Ala Ile Phe Glu Asp Gly Ser Arg Glu
   305                 310                 315                 320
   Asp Asp Ile Asp Ala Val Ile Phe Ala Thr Gly Tyr Ser Phe Ser Phe
                   325                 330                 335
   Pro Phe Leu Glu Asp Ser Val Lys Val Val Lys Asn Lys Val Ser Leu
               340                 345                 350
   Tyr Lys Lys Val Phe Pro Pro Asn Leu Glu Lys Pro Thr Leu Ala Ile
           355                 360                 365
   Ile Gly Leu Ile Gln Pro Leu Gly Ala Ile Met Pro Ile Ser Glu Leu
       370                 375                 380
   Gln Ala Arg Trp Ala Thr Leu Val Phe Lys Gly Leu Lys Thr Leu Pro
   385                 390                 395                 400
   Ser Gln Ser Glu Met Met Thr Glu Ile Ser Gln Val Gln Glu Lys Met
                   405                 410                 415
   Ala Lys Arg Tyr Val Glu Ser Gln Arg His Thr Ile Gln Gly Asp Tyr
               420                 425                 430
   Ile Glu Thr Met Glu Glu Ile Ala Asp Leu Val Gly Val Arg Pro Asn
           435                 440                 445
   Leu Leu Ser Leu Ala Phe Thr Asp Pro Arg Leu Ala Leu Gln Leu Leu
       450                 455                 460
   Leu Gly Pro Cys Thr Pro Val His Tyr Arg Leu Gln Gly Arg Gly Lys
   465                 470                 475                 480
   Trp Asp Gly Ala Arg Lys Thr Ile Leu Thr Val Glu Asp Arg Ile Arg
                   485                 490                 495
   Lys Pro Leu Met Thr Arg Val Thr Glu Ser Ser Asn Ser Val Thr Ser
               500                 505                 510
   Met Met Thr Met Gly Lys Phe Met Leu Ala Ile Ala Phe Leu Ala Ile
           515                 520                 525
   Ala Val Val Tyr Phe
       530
<210> 43
   <211> 445
   <212> PRT
   <213> Artificial Sequence
<220>
   <223> consensus sequence
<220>
   <221> misc_feature
   <222> (4)..(4)
   <223> Xaa can be any naturally occurring amino acid
<220>
   <221> misc_feature
   <222> (6)..(6)
   <223> Xaa can be any naturally occurring amino acid
<220>
   <221> misc_feature
   <222> (8)..(8)
   <223> Xaa can be any naturally occurring amino acid
<220>
   <221> misc_feature
   <222> (12)..(12)
   <223> Xaa can be any naturally occurring amino acid
<220>
   <221> misc_feature
   <222> (75)..(75)
   <223> Xaa can be any naturally occurring amino acid
<220>
   <221> misc_feature
   <222> (108)..(108)
   <223> Xaa can be any naturally occurring amino acid
<220>
   <221> misc_feature
   <222> (114)..(114)
   <223> Xaa can be any naturally occurring amino acid
<220>
   <221> misc_feature
   <222> (138)..(138)
   <223> Xaa can be any naturally occurring amino acid
<220>
   <221> misc_feature
   <222> (141)..(141)
   <223> Xaa can be any naturally occurring amino acid
<220>
   <221> misc_feature
   <222> (156)..(156)
   <223> Xaa can be any naturally occurring amino acid
<220>
   <221> misc_feature
   <222> (158)..(158)
   <223> Xaa can be any naturally occurring amino acid
<220>
   <221> misc_feature
   <222> (211)..(211)
   <223> Xaa can be any naturally occurring amino acid
<220>
   <221> misc_feature
   <222> (221)..(221)
   <223> Xaa can be any naturally occurring amino acid
<220>
   <221> misc_feature
   <222> (245)..(245)
   <223> Xaa can be any naturally occurring amino acid
<220>
   <221> misc_feature
   <222> (247)..(247)
   <223> Xaa can be any naturally occurring amino acid
<220>
   <221> misc_feature
   <222> (330)..(330)
   <223> Xaa can be any naturally occurring amino acid
<220>
   <221> misc_feature
   <222> (355)..(355)
   <223> Xaa can be any naturally occurring amino acid
<220>
   <221> misc_feature
   <222> (387)..(387)
   <223> Xaa can be any naturally occurring amino acid
<220>
   <221> misc_feature
   <222> (394)..(394)
   <223> Xaa can be any naturally occurring amino acid
<220>
   <221> misc_feature
   <222> (417)..(417)
   <223> Xaa can be any naturally occurring amino acid
<220>
   <221> misc_feature
   <222> (444)..(444)
   <223> Xaa can be any naturally occurring amino acid
<220>
   <221> misc_feature
   <222> (445)..(445)
   <223> Xaa can be any naturally occurring amino acid
<400> 43
   Met Ala Pro Xaa Ile Xaa Leu Xaa Thr Ser Arg Xaa Val Ala Val Ile
   1               5                   10                  15
   Gly Ala Gly Ala Ala Gly Leu Val Ala Ala Arg Glu Leu Arg Arg Glu
               20                  25                  30
   Gly His Lys Val Val Val Phe Glu Arg Glu Asn Gln Val Gly Gly Thr
           35                  40                  45
   Trp Val Tyr Thr Pro Glu Val Glu Ser Asp Pro Leu Gly Leu Asp Pro
       50                  55                  60
   Asn Arg Thr Ile Val His Ser Ser Leu Tyr Xaa Ser Leu Arg Thr Asn
   65                  70                  75                  80
   Leu Pro Arg Glu Val Met Gly Phe Arg Asp Tyr Pro Phe Val Pro Arg
                   85                  90                  95
   Glu Gly Glu Gly Arg Asp Pro Arg Arg Phe Pro Xaa His Arg Glu Val
               100                 105                 110
   Leu Xaa Tyr Leu Glu Asp Phe Ala Arg Glu Phe Gly Ile Glu Glu Leu
           115                 120                 125
   Val Arg Phe Gly Thr Glu Val Val Phe Xaa Gly Leu Xaa Asp Gly Lys
       130                 135                 140
   Trp Arg Val Lys Ser Arg Ser Glu Asp Gly Asp Xaa Val Xaa Glu Ile
   145                 150                 155                 160
   Phe Asp Ala Val Val Val Cys Asn Gly His Tyr Thr Glu Pro Arg Val
                   165                 170                 175
   Ala Glu Ile Pro Gly Ile Asp Ala Trp Pro Gly Lys Gln Met His Ser
               180                 185                 190
   His Asn Tyr Arg Thr Pro Glu Pro Phe Arg Asp Gln Val Val Val Leu
           195                 200                 205
   Ile Gly Xaa Ser Ala Ser Ala Val Asp Ile Ser Arg Xaa Ile Ala Gly
       210                 215                 220
   Val Ala Lys Glu Val His Ile Ala Ser Arg Ser Val Glu Ala Glu Thr
   225                 230                 235                 240
   Leu Glu Lys Leu Xaa Gly Xaa Asp Asn Met Trp Leu His Ser Met Ile
                   245                 250                 255
   Glu Ser Val His Lys Asp Gly Thr Val Val Phe Gln Asp Gly Ser Val
               260                 265                 270
   Val Leu Ala Asp Val Ile Leu His Cys Thr Gly Tyr Lys Tyr His Phe
           275                 280                 285
   Pro Phe Leu Glu Thr Asn Gly Ile Val Thr Val Asp Asp Asn Arg Val
       290                 295                 300
   Gly Pro Leu Tyr Lys His Val Phe Pro Pro Ala Leu Ala Pro Gly Leu
   305                 310                 315                 320
   Ser Phe Val Gly Leu Pro Trp Lys Val Xaa Pro Phe Pro Leu Phe Glu
                   325                 330                 335
   Leu Gln Ser Lys Trp Ile Ala Gly Val Leu Ser Gly Arg Ile Ala Leu
               340                 345                 350
   Pro Ser Xaa Glu Glu Met Met Ala Asp Val Lys Ala Phe Tyr Ser Ser
           355                 360                 365
   Leu Glu Ala Ser Gly Lys Pro Lys His Tyr Thr His Asn Leu Gly Asp
   370 375 380
   Ser Gln Xaa Tyr Asp Asn Trp Leu Ala Xaa Gln Cys Gly Cys Pro Pro
   385 390 395 400
   Val Glu Glu Trp Arg Lys Gln Met Tyr Ile Ala Thr Ser Lys Asn Lys
   405 410 415
   Xaa Ala Arg Pro Glu Thr Tyr Arg Asp Glu Trp Asp Asp Asp Asp Leu
   420 425 430
   Ile Leu Glx Ala Tyr Glu Asp Phe Ala Lys Tyr Xaa Xaa
   435 440 445
<210> 44
   <211> 1397
   <212> DNA
   <213> Artificial Sequence
<220>
   <223> 5'UTR in combination with the DNA sequence of AtFMO GS
<400> 44